# EUROPEAN PATENT SPECIFICATION

(11) **EP 2 729 563 B1**
(45) Date of publication and mention of the grant of the patent: **12.09.2018**
(21) Application number: 12732669.2
(22) Date of filing: 06.07.2012
(51) Int. Cl.: C12N 7/00, C12N 15/70

(54) **GENETICALLY MODIFIED PHAGE AND USE THEREOF**
GENETISCH MODIFIZIERTE PHAGEN UND VERWENDUNG DAFÜR
PHAGE MODIFIÉ GÉNÉTIQUEMENT ET UTILISATION ASSOCIÉE

(30) Priority: 07.07.2011 EP 11173033; 07.07.2011 US 201113178402
(43) Date of publication of application: 14.05.2014
(73) Proprietor: Delphi Genetics, 6041 Gosselies (BE)
(72) Inventor: SZPIRER, Cédric, B-6224 Fleurus (BE)
(74) Representative: Icosa
(86) International application number: PCT/EP2012/063244
(87) International publication number: WO 2013/004817

(56) References cited:
- WO-A1-2006/091483
- US-A- 4 952 496
- US-A1- 2002 090 678
- US-A1- 2003 003 472
- ALAN C CHRISTENSEN: "Bacteriophage Lambda-Based Expression Vectors", MOLECULAR BIOTECHNOLOGY, vol. 17, no. 3, 1 January 2001 (2001-01-01), pages 219-224, XP055012303, ISSN: 1073-6085, DOI: 10.1385/MB:17:3:219
- LIN C S ET AL: "Characterization of bacteriophage lambda Q- mutant for stable and efficient production of recombinant protein in Escherichia coli system", BIOTECHNOLOGY AND BIOENGINEERING, vol. 57, no. 5, 5 March 1998 (1998-03-05), pages 529-535, XP002523486, ISSN: 0006-3592, DOI: 10.1002/(SICI)1097-0290(19980305)57:5<529: :AID-BIT4>3.0.CO;2-I
- NANDAN PADUKONE ET AL: ".lambda. Vectors for stable cloned gene expression", BIOTECHNOLOGY PROGRESS, vol. 6, no. 4, 1 July 1990 (1990-07-01), pages 277-282, XP055012468, ISSN: 8756-7938, DOI: 10.1021/bp00004a008
- DATABASE EMBL [Online] 17 July 2009 (2009-07-17), Jeong H. et al.: "Complete nucleotide sequence of prophage DE3", XP002663847, Database accession no. EU078592 cited in the application
- FUDU MIAO ET AL: "Characterization of gene expression in recombinant Escherichia coli cells infected with phage .lambda.", BIOTECHNOLOGY PROGRESS., vol. 9, no. 2, 1 March 1993 (1993-03-01), pages 153-159, XP55223075, US ISSN: 8756-7938, DOI: 10.1021/bp00020a006
- STERNBERG ET AL: "A characterization of bacteriophage P1 DNA fragments cloned in a lambda Vector", VIROLOGY, ELSEVIER, AMSTERDAM, NL, vol. 96, no. 1, 15 July 1979 (1979-07-15), pages 129-142, XP023048740, ISSN: 0042-6822, DOI: 10.1016/0042-6822(79)90179-X [retrieved on 1979-07-15]
- DORGAI L ET AL: "Recognition of core binding sites by bacteriophage integrases", JOURNAL OF MOLECULAR BIOLOGY, ACADEMIC PRESS, UNITED KINGDOM, vol. 277, no. 5, 17 April 1998 (1998-04-17), pages 1059-1070, XP004454000, ISSN: 0022-2836, DOI: 10.1006/JMBI.1998.1642

## Description

### FIELD OF INVENTION

The present invention relates to the field of production of biomolecules of interest in biologic systems. Specifically, the present invention relates to a genetically modified phage integrated in the genome of a bacterial host cell, and use thereof in order to avoid phage contamination of the culture broth and/or bacterial lysis.

### BACKGROUND OF INVENTION

Bacterial cells are systems of choice for the production of biomolecules, especially of target proteins. Among other advantages, bacterial systems are easy to use and allow the rapid production of large quantities of proteins in a limited volume of culture.

One of the most widely and routinely used bacterial system is the bacteriophage T7 expression system. This bacterial system was described in US 4,952,496. In this system, the gene encoding the target protein is placed under the control of a T7 promoter, and is transformed in a bacterial host, usually *E. coli,* which comprises an integrated lambda DE3 lysogen phage. The lambda DE3 lysogen phage carries the gene encoding the T7 RNA polymerase under the control of a *lacUV5* promoter. When cultured on an IPTG-containing medium, the expression of the T7 RNA polymerase is induced, and allows the expression of the target protein. US 2003/003472 discloses, an *E. coli* strain: 5HT71, produced by lysogenisation of HB101 with λDE3.

Due to the integration of the gene of the T7 RNA polymerase (*T7 gene* 1) within the sequence of the *Int* gene, Lambda DE3 phage should be defective in its ability to enter into the lytic phase. However, bacterial lysis is observed during some protein productions and in the absence of any other phage, suggesting that the DE3 phage may recover its lytic properties. The bacterial lysis and even more, the presence of infectious phages in the culture broth is highly problematic because (i) it compromises the use of produced target proteins for some applications, such as, for example, pharmaceutical applications, (ii) the decontamination process in order to remove any trace of phages requires the shutdown of the production lines and the complete renewal of the batches of culture and (iii) it reduces dramatically the yield of recombinant protein.

Modified λ phages were described in the art. sternberg et al., 1979 (Virology, 96:129-142) relates to hybrid P1-λ vectors, wherein the *int* gene is deleted, and comprising a 6% deletion that removes DNA between lambda *P* and *Q* genes. Padukone et al., 1990 (Biotechnology pogress, 6:277-282) describes bacteriophage λ derived vectors for stable cloned gene expression, wherein said vectors comprise an inactivation of the *S* gene. Miao et al., 1993 (Biotechnology progress, 9:153-159) describes the infection of *E. coli* cells with a lambda phage (λCE6), wherein the *int* gene is deleted, and wherein the *S* gene contains the Sam7 lysis mutation. Lin et al., 1998 (Biotechnology and bioengineering, 57:529-535) relates to a bacteriophage λ comprising a mutation in the *Q* gene and to the use there for producing recombinant proteins in *E. coli* system. Dorgai et al, 1998 (J.Mol. Biol. 277:1059-1070) studies lambda and HK022 phages integrase recombination specificity in *att* nucleic acid sequences and *Int* protein sequences. In particular, Dorgai et al, 1998 describes a RW721 lysogenic bacteria carrying a λ Sam7 prophage and deleted for *att, Int* and *imm.* Christensen et al, 2001 (Mol. Biotechnology, 17:219-224) describes bacteriophage λ expression vectors comprising an expression system derived from bacteriophage T7 with mutation in the *Q* gene. US 2002/090678 relates to a method for producing biomolecules, wherein bacterial cells of the producer strain is infected with bacteriophage λ comprising mutation in one of the late phase *N, Q* and/or *R* genes. WO 2006/091483 relates to expression vector systems for use in expressing exogenous gene under the control of T7 polymerase promoter. Said expression vector comprises inactivated *Q, S, E,* or *R* genes. However, the use of these modified λ phages may still be associated with lysis of the host cells and subsequent contamination of the medium.

Alternative methods to the use of a phage have been described:
WO03/050240 describes an expression system for producing a target protein in a host cell comprising a gene encoding T7 RNA polymerase integrated using homologous recombination. However, the system of WO03/050240 is difficult to implement, due to the size of the T7 RNA polymerase gene and due to the fact that homologous recombination is not easy to do in all *E. coli* strains (as mentioned by Phue et al., Biotechnology and Bioengineering, 101, 831-836, 2008). Consequently, the number of transformed cells carrying the T7 RNA polymerase integration remains very low or these cells are not obtained. Moreover, using homologous recombination, it is necessary to use a selective marker to select bacteria containing integration of the T7 RNA polymerase gene. This marker will not be usable for another selection step and could be undesired for the final use of the strain. For example, selective markers often used are antibiotic resistance genes but it is recommended to avoid these genes in biopharmaceutical productions. An additional step is thus required to remove the antibiotic resistance gene from the strain and it is not always possible to do it.

WO2008/139153 describes another expression system, wherein the host cell is transformed with a plasmid comprising an expression cassette for T7 RNA polymerase. However, due to the use of a plasmid, hosts cells have to be maintained in selective conditions to make sure that they still comprise the plasmid. In addition, plasmids are frequently subjected to recombination, which impaired the expression system.

Therefore, there is a need for a novel method for producing a biomolecule of interest, wherein, when a phage is used, the culture is not contaminated by infectious phages or unintentionally lysed during growth or protein production.

The present invention hereby provides a genetically modified phage that does not recover its lytic properties during culture, thereby allowing the production of a biomolecule of interest without phage contamination.

### SUMMARY

One object of the invention is a bacterial host cell comprising a genetically modified phage wherein
- an expression system is inserted, said expression system comprising a nucleic acid sequence encoding a protein, said protein further inducing the expression of a biomolecule of interest, and
- the *int, S, R, Rz, Xis* genes are deleted,
wherein said genetically modified phage is integrated in the genome of said bacterial host cell, and
wherein the phage is the lambda phage, the 434phage, the phi80 phage, the phi81 phage, the HK97 phage, or the P21 phage.

In one embodiment, the *Q* gene is further inactivated.

In one embodiment, the promoter comprised in the expression system is inducible.

In another embodiment of the invention, the expression system is the T7 expression system.

In one embodiment, said phage is one of the phages P11 or P13, wherein
- P11 comprises the sequence NC_001416 wherein the coding sequences of genes *S, R, Rz, Xis* and *Int* are deleted, and
- P13 comprises the sequence NC_001416 wherein the coding sequences of genes *S, R, Rz, Q, Xis* and *Int* are deleted.

In one embodiment of the invention, the host cell is an enterobacteria, preferably *E. Coli,* more preferably BL21.

In another embodiment of the invention, the host cell as described here above further comprises the inactivation of at least one of the genes *tonA, galK, araB, araA, lon, ompT, rcsA, hsdR, mrr, endA* and *recA.*

In another embodiment of the invention, the host cell as described here above comprises the insertion of the ccdb gene.

Another object of the invention is a kit comprising the host cell as described here above and a plasmid comprising the ccdA gene.

Another object of the invention is a process for preparing a host cell as described here above, comprising infecting a host cell with a genetically modified phage as described here above, and using a helper phage for complementation.

Another object of the invention is a process for producing a biomolecule of interest, comprising
- cultivating a host cell comprising as described hereinabove, wherein said host cell comprises the nucleic acid sequence of the biomolecule of interest,
- expressing said biomolecule of interest.

### DETAILED DESCRIPTION

The present invention relates to a bacterial host cell comprising a genetically modified phage, wherein the ability of the phage to regain its lytic properties is limited.

The scope of the present invention is determined by the claims. The following embodiments are embodiments falling within the restrictions of the claims.

The Inventors focused on the genetic modification of a phage. Surprisingly, the Inventors showed that it was not possible to delete all viral sequences of the integrated phage, because the viability of the infected bacteria was severely compromised in that case. In particular, the Inventors showed that the deletion of a DNA fragment comprising the coding sequences of the *ral* gene and the *N* gene leads to the death of the host cell (See EXAMPLES). This result was surprising because the *ral* and *N* genes are not known to be involved in the lysogenic state; the N gene is only described as essential for lytic growth. On the contrary, in lysogenic state, a repressor of the phage, named CI or C2, blocks the expression of N and ral.

The present invention thus relates to a bacterial host cell comprising a genetically modified phage wherein
- an expression system is inserted, and
- the *Int, Xis, R, S,* and *Rz* genes are deleted and optionally wherein the *Q* gene is inactivated.

According to the disclosure, the genetically modified phage comprises an expression system and the *S* gene is inactivated.

According to the disclosure, the genetically modified phage comprises an expression system and the *R* gene is inactivated.

According to the disclosure, the genetically modified phage comprises an expression system and the *Q* gene is inactivated.

According to the disclosure, the genetically modified phage comprises an expression system and the *Int* gene is inactivated.

According to the disclosure, the genetically modified phage comprises an expression system and the *Xis* gene is inactivated.

According to the disclosure, the genetically modified phage comprises an expression system and the *S* and *Int* genes are inactivated.

According to the disclosure, the genetically modified phage comprises an expression system and the *S* and *Xis* genes are inactivated.

According to the disclosure, the genetically modified phage comprises an expression system and the *S* and *R* genes are inactivated.

According to the disclosure, the genetically modified phage comprises an expression system and the *S* and *Rz* genes are inactivated.

According to the disclosure, the genetically modified phage comprises an expression system and the *S* and *Q* genes are inactivated.

According to the disclosure, the genetically modified phage comprises an expression system and the *R* and *Rz* genes are inactivated.

According to the disclosure, the genetically modified phage comprises an expression system and the *R* and *Int* genes are inactivated.

According to the disclosure, the genetically modified phage comprises an expression system and the *R* and *Xis* genes are inactivated.

According to the disclosure, the genetically modified phage comprises an expression system and the *R* and *Q* genes are inactivated.

According to the disclosure, the genetically modified phage comprises an expression system and the *Q* and *Rz* genes are inactivated.

According to the disclosure, the genetically modified phage comprises an expression system and the *Q* and *Int* genes are inactivated.

According to the disclosure, the genetically modified phage comprises an expression system and the *Q* and *Xis* genes are inactivated.

According to the disclosure, the genetically modified phage comprises an expression system and the *S*, *Int* and *Xis* genes are inactivated.

According to the disclosure, the genetically modified phage comprises an expression system and the *S*, *Int* and *Rz* genes are inactivated.

According to the disclosure, the genetically modified phage comprises an expression system and the *S, Int* and *R* genes are inactivated.

According to the disclosure, the genetically modified phage comprises an expression system and the *S, Xis* and *Rz* genes are inactivated.

According to the disclosure, the genetically modified phage comprises an expression system and the *S, Xis* and *R* genes are inactivated.

According to the disclosure, the genetically modified phage comprises an expression system and the *S, R* and *Rz* genes are inactivated.

According to the disclosure, the genetically modified phage comprises an expression system and the *S, R* and *Q* genes are inactivated.

According to the disclosure, the genetically modified phage comprises an expression system and the *S, Q* and *Rz* genes are inactivated.

According to the disclosure, the genetically modified phage comprises an expression system and the *S, Q* and *Int* genes are inactivated.

According to the disclosure, the genetically modified phage comprises an expression system and the *S, Q* and *Xis* genes are inactivated.

According to the disclosure, the genetically modified phage comprises an expression system and the *R, Int* and *Xis* genes are inactivated.

According to the disclosure, the genetically modified phage comprises an expression system and the *R, Int* and *Rz* genes are inactivated.

According to the disclosure, the genetically modified phage comprises an expression system and the *R, Xis* and *Rz* genes are inactivated.

According to the disclosure, the genetically modified phage comprises an expression system and the *R, Q* and *Rz* genes are inactivated.

According to the disclosure, the genetically modified phage comprises an expression system and the *R, Xis* and *Q* genes are inactivated.

According to the disclosure, the genetically modified phage comprises an expression system and the *R, Int* and *Q* genes are inactivated.

According to the disclosure, the genetically modified phage comprises an expression system and the *Q, Int* and *Rz* genes are inactivated.

According to the disclosure, the genetically modified phage comprises an expression system and the *Q, Int* and *Xis* genes are inactivated.

According to the disclosure, the genetically modified phage comprises an expression system and the *Q, Xis* and *Rz* genes are inactivated.

According to the disclosure, the genetically modified phage comprises an expression system and the S, *Int, Xis* and *Rz* genes are inactivated.

According to the disclosure, the genetically modified phage comprises an expression system and the *S, Int, Xis* and *R* genes are inactivated.

According to the disclosure, the genetically modified phage comprises an expression system and the *S, Int, R* and *Rz* genes are inactivated.

According to the disclosure, the genetically modified phage comprises an expression system and the *S, R, Xis* and *Rz* genes are inactivated.

According to the disclosure, the genetically modified phage comprises an expression system and the *R, Rz, Xis* and *Int* genes are inactivated.

According to the disclosure, the genetically modified phage comprises an expression system and the *Q, R, Rz* and *S* genes are inactivated.

According to the disclosure, the genetically modified phage comprises an expression system and the *Q, R, Rz* and *Int* genes are inactivated.

According to the disclosure, the genetically modified phage comprises an expression system and the *Q, R, S* and *Int* genes are inactivated.

According to the disclosure, the genetically modified phage comprises an expression system and the *Q, Rz, S* and *Int* genes are inactivated.

According to the disclosure, the genetically modified phage comprises an expression system and the *Q, R, Rz* and *Xis* genes are inactivated.

According to the disclosure, the genetically modified phage comprises an expression system and the *Q, R, S* and *Xis* genes are inactivated.

According to the disclosure, the genetically modified phage comprises an expression system and the *Q, Rz, S* and *Xis* genes are inactivated.

According to the disclosure, the genetically modified phage comprises an expression system and the *Q, R, Int* and *Xis* genes are inactivated.

According to the disclosure, the genetically modified phage comprises an expression system and the *Q, Rz, Int* and *Xis* genes are inactivated.

According to the disclosure, the genetically modified phage comprises an expression system and the *Q, S, Int* and *Xis* genes are inactivated.

According to the disclosure, the genetically modified phage comprises an expression system and the *S, R, Rz, Xis* and *Int* genes are inactivated.

According to the disclosure, the genetically modified phage comprises an expression system and the *S, R, Q, Rz* and *Xis* genes are inactivated.

According to the disclosure, the genetically modified phage comprises an expression system and the S, *R, Q, Rz* and *Int* genes are inactivated.

According to the disclosure, the genetically modified phage comprises an expression system and the *S, R, Q, Xis* and *Int* genes are inactivated.

According to the disclosure, the genetically modified phage comprises an expression system and the S, *Q, Rz, Xis* and *Int* genes are inactivated.

According to the disclosure, the genetically modified phage comprises an expression system and the *R, Q, Rz, Xis* and *Int* genes are inactivated.

In one embodiment, the genetically modified phage comprises an expression system and the *S, R, Q, Rz, Xis* and *Int* genes are inactivated.

Examples of phages which can be used in the invention include, but are not limited to, the lambda (λ) phage, lambda-like and lambdoid phages. Lambda phage, also known as coliphage lambda, is a virus that infects *Escherichia coli.* Lambda is a temperate bacteriophage. Lambda-like phages form a family of bacteriophages and archaeal viruses which are characterized by long, non-contractile tails. Lambdoid phages are natural relatives of lambda phage. Most of them grow on *E. coli,* but a few come from other host cells, such as, for example, *Salmonella typhimurium.* These phages may have the same gene order as lambda.

Examples of lambda-like and lambdoid phages which could be used in the present invention include, but are not limited to, coliphage 434, phi80, phi81, HK97, P21 and P22.

In an embodiment, the phage is lambda (Enterobacteria phage lambda, accession number NC_001416). The organization of the genome of the lambda phage is shown in table 1 below.

**Table 1**

| **Start** | **End** | **Name** | **Description** |
|---|---|---|---|
| 191 | 736 | nu1 | DNA packaging protein |
| 711 | 2636 | A | DNA packaging protein |
| 2633 | 2839 | W | head-tail joining protein |
| 2836 | 4437 | B | capsid component |
| 4418 | 5737 | C | capsid component |
| 5132 | 5737 | nu3 | capsid assembly protein |
| 5747 | 6079 | D | head-DNA stabilization protein |
| 6135 | 7160 | E | capsid component |
| 7202 | 7600 | Fi | DNA packaging protein |
| 7612 | 7965 | Fii | head-tail joining protein |
| 7977 | 8555 | Z | tail component |
| 8552 | 8947 | U | tail component |
| 8955 | 9695 | V | tail component |
| 9711 | 10133 | G | tail component |
| 10115 | 10549 | T | tail component |
| 10542 | 13103 | H | tail component |
| 13100 | 13429 | M | tail component |
| 13429 | 14127 | L | tail component |
| 14276 | 14875 | K | tail component |
| 14773 | 15444 | I | tail component |
| 15505 | 18903 | J | tail:host specificity protein |
| 18965 | 19585 | lom | outer host membrane |
| 19650 | 20855 | orf-401 | Tail fiber protein |
| 20767 | 20147 | orf206b | hypothetical protein |
| 21029 | 21973 | orf-314 | Tail fiber |
| 21973 | 22557 | orf-194 | Putative fiber assembly protein |
| 23918 | 22686 | ea47 | ea47 |
| 25399 | 24509 | ea31 | ea31 |
| 26973 | 25396 | ea59 | ea59 |
| 28882 | 27812 | int | integration protein |
| 29078 | 28860 | xis | Excisionase |
| 29285 | 29118 | hypothetical | hypothetical protein |
| 29655 | 29374 | ea8.5 | ea8.5 |
| 30395 | 39847 | ea22 | ea22 |
| 31024 | 30839 | orf61 | hypothetical protein |
| 31196 | 31005 | orf63 | hypothetical protein |
| 31351 | 31169 | orf60a | hypothetical protein |
| 32028 | 31348 | exo | exonuclease |
| 32810 | 32025 | bet | bet |
| 33232 | 32816 | gam | host-nuclease inhibitor protein Gam |
| 33330 | 33187 | kil | host-killing protein |
| 33463 | 33299 | cIII | antitermination protein |
| 35582 | 33494 | ea10 | Putative single-stranded DNA binding protein |
| 35582 | 33930 | ral | restriction alleviation protein |
| 34357 | 34271 | orf28 | hypothetical protein |
| 34482 | 35036 | lambdap48 | Superinfection exclusion protein B |
| 35582 | 34560 | N | early gene regulator |
| 36259 | 35825 | rexb | exclusion protein |
| 37114 | 36275 | rexa | exclusion protein |
| 37940 | 37227 | cI | repressor |
| 38023 | 38135 | cro | antirepressor |
| 38360 | 38653 | cII | transcriptional activator |
| 38686 | 39585 | O | DNA replication protein |
| 39582 | 40283 | P | DNA replication protein |
| 40280 | 40570 | ren | ren exclusion protein |
| 40644 | 41084 | NinB | NinB |
| 41081 | 41953 | NinC | NinC protein |
| 41950 | 42123 | NinD | NinD protein |
| 42090 | 42272 | NinE | NinE protein |
| 42269 | 42439 | NinF | NinF protein |
| 42429 | 43043 | NinG | NinG protein |
| 43040 | 43246 | NinH | NinH protein |
| 43224 | 43889 | NinI | NinI protein |
| 43886 | 44509 | Q | late gene regulator |
| 44621 | 44815 | orf-64 | hypothetical protein |
| 45186 | 45509 | S | Cell lysis protein |
| 45493 | 45969 | R | endolysin |
| 45966 | 46427 | Rz | cell lysis protein |
| 46186 | 46368 | Rz1 | Rz1 protein |
| 46752 | 46459 | bor | Bor protein precursor |
| 47575 | 47042 | lambdap78 | putative enveloppe protein |
| 47738 | 47944 | lambdap79 | hypothetical protein |

In the present invention, the position of the residues within the sequence of the lambda phage relates to NC_001416.

In another embodiment, the phage is lambda DE3 (accession number EU078592). The Lambda DE3 phage is a modified lambda phage D69, comprising the gene encoding the T7 RNA polymerase under the control of a lacUV5 promoter. The list of the genes carried by the sequence of Lambda DE3 and their position are shown in the Table 2 below.

**Table 2**

| Start | End | Name | Description |
|---|---|---|---|
| 341 | 1423 | lacI | lactose operon repressor |
| 1546 | 1995 | lacZ | N-terminal fragment of beta-galactosidase |
| 2026 | 4677 | 1 | T7 DNA-directed RNA polymerase |
| 5804 | 5586 | xis | excisionase |
| 6011 | 5844 | hypothetical | Hypothetical protein |
| 6381 | 6100 | ea8.5 | ea8.5 |
| 7121 | 6573 | ea22 | ea22 |
| 7750 | 7565 | hypothetical | Hypothetical protein |
| 7922 | 7731 | hypothetical | Hypothetical protein |
| 8077 | 7895 | hypothetical | Hypothetical protein |
| 8754 | 8074 | exo | exonuclease |
| 9536 | 8751 | bet | Bet |
| 9958 | 9542 | gam | host-nuclease inhibitor protein Gam |
| 10056 | 9913 | kil | host-killing protein |
| 10189 | 10025 | cIII | antitermination protein |
| 10630 | 10262 | ea10 | putative single-stranded DNA binding protein |
| 11013 | 10813 | ral | restriction alleviation protein |
| 11083 | 10997 | hypothetical | Hypothetical protein |
| 11391 | 11092 | N | probable regulatory protein N (early gene regulator) |
| 12356 | 11706 | C2 | repressor protein C2 |
| 12437 | 12622 | cro | regulatory protein cro (Antirepressor) |
| 12738 | 13037 | cII | antitermination protein |
| 13070 | 13969 | O | DNA replication protein |
| 13966 | 14667 | P | DNA replication protein |
| 14664 | 15467 | ren | Ren exclusion protein |
| 15464 | 16087 | Q | late gene regulator |
| 16199 | 16393 | hypothetical | Hypothetical protein |
| 16764 | 17087 | S | cell lysis protein |
| 17071 | 17547 | R | cell lysis protein |
| 17544 | 18005 | Rz | cell lysis protein |
| 18330 | 18037 | Bor | Bor protein precursor |
| 19153 | 18620 | putative | putative envelope protein |
| 19316 | 19522 | hypothetical | Hypothetical protein |
| 20270 | 20815 | nu1 | DNA packaging protein |
| 20790 | 22715 | A | DNA packaging protein |
| 22712 | 22918 | W | head-tail joining protein |
| 22915 | 24516 | B | capsid component |
| 24497 | 25816 | C | capsid component |
| 25826 | 26158 | D | head-DNA stabilization protein |
| 26214 | 27239 | E | capsid component |
| 27281 | 27679 | Fi | DNA packaging protein |
| 27691 | 28044 | Fii | head-tail joining protein |
| 28056 | 28634 | Z | tail component |
| 28631 | 29026 | U | tail component |
| 29034 | 29774 | V | tail component |
| 29790 | 30212 | G | tail component |
| 30194 | 30628 | T | tail component |
| 30621 | 33182 | H | tail component |
| 33179 | 33508 | M | tail component |
| 33508 | 34206 | L | tail component |
| 34356 | 34955 | K | tail component |
| 34853 | 35524 | I | tail component |
| 35585 | 38983 | J | tail:host specificity protein |
| 39045 | 39665 | lom | outer host membrane |
| 39730 | 40935 | tail | tail fiber protein |
| 41109 | 41372 | tail | tail fiber |
| 42175 | 41237 | ea59 | ea59 |

In the present invention, the position of the residues within the sequence of the lambda DE3 phage relates to EU078592.

As used herein, an "expression system" refers to a linear or a circular DNA molecule composed of a fragment encoding a nucleic acid sequence operably linked to an additional fragment for the transcription of the system.

The additional fragment includes a promoter and a stop codon sequence. The expression system may further contain one or more origins of replication, one or more selection markers and a sequence encoding a ribosome binding site.

"Operably linked" means that fragments are arranged to be functioning as they are supposed to be, for example once transcription starts at the promoter, it goes through coded fragment to stop codon.

"Promoter" in the meaning of the present invention is an expression control element that permits binding of RNA polymerase and the initiation of transcription.

In one embodiment of the invention, the nucleic acid sequence is under the control of a "strong" promoter. A strong promoter is characterized by a high binding affinity of the promoter sequence to an RNA polymerase, usually the naturally occurring corresponding RNA polymerase, on the one hand and the rate of formation of mRNA by that RNA polymerase on the other hand.

In a preferred embodiment, the nucleic acid sequence is under the control of an "inducible promoter". An "inducible promoter" is a promoter that may be regulated by external factors, e.g., the presence of an inductor (also termed "inducer") molecule or the absence of a repressor molecule, or physical factors like increased or decreased temperature, osmolarity, or pH value. Different promoters and the respective induction principles were reviewed by Makrides et al. (Microbiological Reviews, 1996, (60)3: 512-538). Examples of inducible promoters which may be used in the present invention include, but are not limited to, the tac or the trc promoter, the lac or the lacUV5 promoter (all inducible by lactose or its analog IPTG (isopropylthiol-β-D-galactoside)), the tightly regulatable araBAD promoter (PBAD; Guzman et al., 1995, inducible by arabinose), the trp promoter (inducible by β-indole acrylic acid addition or tryptophan starvation, repressible by tryptophan addition), the lambda promoter pL (λ) (induction by an increase of temperature), the phoA promoter (inducible by phosphate starvation), the PprpB (induction with propionate) or other promoters suitable for recombinant protein expression, which all use *E. coli* RNA polymerase.

Among inducible promoters are those that show a "leaky" expression behavior. Such promoters (so-called "leaky promoters") are, in principle, inducible, but show nevertheless also basal expression without being externally induced. Inducible promoters that show leaky expression under non-induced conditions may behave similarly to constitutive promoters *(i.e.,* they are steadily and continuously active or they may be activated or enhanced as a result of certain cultivation conditions). Leaky promoters may be particularly useful for continuously operated cultivation processes. Examples of leaky promoters are the T7 promoter and the trp promoter.

In one embodiment of the invention, the promoter may also be constitutive, *i.e.,* a promoter which controls expression without the need for induction on the one hand, or the possibility of repression on the other hand. Hence, there is continuous and steady expression at a certain level. As an example, the strong constitutive HCD promoter (Poo et al., Biotechnology Letters, 2002, 24:1185-1189; Jeong et al., Protein expression and purification, 2004, 36:150-156) may be applied for constitutive expression.

In one embodiment, the expression system comprises a nucleic acid sequence encoding a protein that induces the expression of the biomolecule of interest. Advantageously, the expression of the biomolecule of interest is induced in particular conditions, such as, for example, under selection.

Examples of such nucleic acid sequences include, but are not limited to, the gene encoding the T7 RNA polymerase, *T7 gene* 1. In that case, the expression of the T7 RNA polymerase induces the expression of the biomolecule of interest placed under the control of a T7 promoter.

Preferably, the expression system is the T7 expression system. The T7 expression system was described in US4,952,496. The T7 expression system comprises a DNA fragment from the T7 phage, containing the entire coding sequence for the T7 RNA polymerase (*i.e.,* the *T7 gene* 1). Any natural active promoter of the *T7 gene 1* was removed and an inducible lacUV5 promoter was inserted ahead of the coding sequence. The lacUV5 promoter is induced by addition of IPTG to the culture medium.

According to another embodiment, the expression system comprises the nucleic acid sequence of the biomolecule of interest.

With regard to the biomolecule of interest, there are no limitations. It may, in principal, be any amino acid sequences, nucleic acid sequences, such as, for example, DNA or RNA. Examples of amino acid sequences include polypeptide, protein or peptide that is to be produced on a manufacturing scale, *e.g.,* an industrial biomolecule or a therapeutic biomolecule.

Examples for biomolecules that can be produced by the method of the invention are, without limitation, enzymes, regulatory proteins, receptors, peptides, e.g., peptide hormones, cytokines, membrane or transport proteins.

The biomolecules of interest may also be antigens as used for vaccination, vaccines, antigen-binding proteins, immune stimulatory proteins, allergens, full-length antibodies or antibody fragments or derivatives. Antibody derivatives may be selected from the group of single chain antibodies, (scFv), Fab fragments, Fv fragments, single domain antibodies (VH or VL fragment), domain antibodies like camelid single domain antibodies (VHH, nanobodies) or other antibody formats as described for instance in Andersen and Reilly (Current Opinion in Biotechnology, 2004, 15:456-462) or Holliger and Hudson (Nature Biotechnology, 2005 (23)9: 1126-1136).

The biomolecules of interest in the present invention can also be exemplified by protein (viral antigen), *e.g.,* coat protein, core protein, protease, reverse transcriptase, integrase, and so forth, encoded in the genome of a pathogenic virus, *e.g.,* hepatitis B virus, hepatitis C virus, I-HV, influenza, and so forth; growth factors such as platelet-derived growth factor (PDGF), stem cell growth factor (SCF), hepatocyte growth factor (HGF), transforming growth factor (TGF), nerve growth factor (NGF), epidermal growth factor (EGF), fibroblast growth factor (FGF), insulin-like growth factor (IGF), and so forth; cytokines such as tumor necrosis factor, interferon, interleukin, and so forth; hematopoietic factors such as erythropoietin, granulocyte colony-stimulating factor, granulocyte-macrophage colony-stimulating factor, macrophage colony-stimulating factor, thrombopoietin, and so forth; peptide hormones such as luteinizing hormone-releasing hormone (LB-RH), thyrotropin-releasing hormone (TRH), insulin, somatostatin, growth hormone, prolactin, adrenocorticotropic hormone (ACTH), melanocyte-stimulating hormone (MSH), thyroidstimulating hormone (TSH), luteinizing hormone (LU), follicle-stimulating hormone (FSH), vasopressin, oxytoxin, calcitonin, parathyroid hormone (PTH), glucagon, gastrin, secretin, pancreozymin, cholecystokinin, angiotensin, human placenta lacto8en, human chorionic gonadotropin (HCG), cerulein, motilin, and so forth; analgesic peptides such as enkephalin, endorphin, dynorphin, kyotorphin, and so forth; enzymes such as superoxide dismutase (SOD), urokinase, tissue plasminogen activator (TPA), asparaginase, kallikrein, and so forth; peptide neurotransmitters such as bombesin, neutrotensin, bradykinin, substance P, Alzheimer's amyloid peptide (AD), SOD1, presenillin 1 and 2, renin, Dsynuclein, amyloid A, amyloid P, activin, anti-HER-2, bombesin, enkephalinase, protease inhibitors, therapeutic enzymes, D 1- antitrypsin, mammalian trypsin inhibitor, mammalian pancreatic trypsin inhibitor, calcitonin, cardiac hypertrophy factor, cardiotrophins (such as cardiotrophin-1), CD proteins (such as CD-3, CD-4, CD-8 and CD-19), CFTR, CTNF, DNase, human chorionic gonadotropin, mouse gonadotropin-associated peptide, cytokines, transthyretin, amylin, lipoproteins, lymphokines, lysozyme, a growth hormone (including human growth hormone), bovine growth hormone, growth hormone releasing factor, parathyroid hormone, thyroid stimulating hormone, growth factors, brain-derived neurotrophic growth factor, epidermal growth factor (EGF), fibroblast growth factor (such as D FGF and D FGF), insulin-like growth factor-I and -II, des(1-3)-IGF-I (brain IGF-I), insulin-like growth factor binding proteins, nerve growth factor (such as NGF-D), platelet derived growth factor (PDGF), vascular endothelial growth factor (VEGF), receptors for growth hormones or growth factors, transforming growth factor (TGF) (such as TGF-D, TGF-D 1, TGF-D2, TGF-D3, TGF-D4 or TGF-D5), neurotrophic factors (such as neurotrophin-3, -4 ,-5, or -6), gelsolin, glucagon, kallikreins, mullerian-inhibiting substance, neurotrophic factors, p53, protein A or D, prorelaxin, relaxin A-chain, relaxin B-chain, rheumatoid factors, rhodopsin, a serum albumin (such as human serum albumin), inhibin, insulin, insulin chains, insulin A-chain, insulin D -chain, insulin receptor, proinsulin, luteinizing hormone, integrin, interleukins (ILs) (such as IL-1 to IL-10, IL-12, IL-13), erythropoietin, thrombopoietin, fibrillin, follicle stimulating hormone, clotting factors (such as factor VIIIC, factor IX, tissue factor, and von Willebrands factor), anticlotting factors (such as Protein C, atrial naturietic factor, lung surfactant), a plasminogen activator (such as human tissue plasminogen activator or urokinase), thrombin, tumor necrosis factor- D or D, D-ketoacid dehydrogenase, addressins, bone morphogenetic proteins (BMPs), collagen, colony stimulating factors (CSFs) (such as M-CSF, GM-CSF and G-CSF), decay accelerating factor, homing receptors, interferons (such as interferon-alpha, -gamma and -beta), keratin, osteoinductive factors, PRNP, regulatory proteins, superoxide dismutase, surface membrane proteins, transport proteins, T-cell receptors, antigens such as gpl 20(HIb) immuno toxins, atrial natriuretic peptide, seminal vesicle exocrine protein, D 2- microglobulin, PrP, precalcitonin, ataxin 1, ataxin 2, ataxin 3, ataxin 6, ataxin 7, huntingtin, androgen receptor, CREB-binding protein, gpl 20, p300, CREB, API, ras, NFAT, jun, fos, dentaorubral pallidoluysian atrophy-associated protein, a microbial protein (e.g., maltose binding protein, ABC transporter, glutathione S transferase, thioredoxin, D-lactamase), green fluorescent protein, red fluorescent protein, an enzyme such as superoxide dismu-tase, asparaginase, arginase, arginine deaminase, adenosine deaminase, ribonuclease, catalase, uricase, bilirubin oxidase, trypsin, papain, alkaline phosphatase, beta-glucoronidase, purine nucleoside phosphorylase or batroxobin, an opioid, *e.g.,* endorphins, enkephalins or non-natural opioids, a hormone or neuropeptide, *e.g.,* calcitonin, glucagon, gastrins, adrenocorticotropic hormone (ACTH), cholecystokinins, lutenizing hormone, gonadotropin-releassing hormone, chorionic gonadotropin, corticotrophin-releasing factor, vasopressin, oxytocin, antidiuretic hormones, thyroid-stimulating hormone, thyrotropin-releasing hormone, relaxin, prolactin, peptide YY, neuropeptide Y, pancreastic polypeptide, leptin, CART (cocaine and amphetamine regulated transcript), a CART related peptide, perilipin, melano- cortins (melanocyte-stimulating hormones) such as MC-4, melanin-concentrating hormones, natriuretic peptides, adrenomedullin, endothelin, secretin, amylin, vasoactive intestinal peptide (VIP), pituary adenylate cyclase activating polypeptide (PACAP), bombesin, bombesin- like peptides, thymosin, heparin-binding protein, soluble CD4, hypothalamic releasing facto- rand melanotonins or functional analogs thereof. In another embodiment of the invention the target protein may be a processing enzyme such as proteases (*e.g*., enterokinase, caspases trypsine like serine proteases), lipase, phospatase, glycosyl hydrolases (*e.g*., mannosidases, xylosidases, fucosidases), kinase, mono or dioxidase, peroxidase, transglutaminase, carboxypeptidase, amidase, esterase, and phosphatase.

Preferred sources for such mammalian polypeptides include human, bovine, equine, porcine, lupine and rodent sources, with human proteins being particularly preferred.

The biomolecule of interest of the present invention also encompasses variants of the aforementioned protein. These variants encompass, for example, protein that has the same activity as the aforementioned protein and that comprises an amino acid sequence with, in the amino acid sequence of the aforementioned protein, one or more deleted, substituted, inserted and/or added amino acids. Such protein can be exemplified by protein that has the same activity as the aforementioned protein and that comprises an amino acid sequence with, in the amino acid sequence of the aforementioned protein, one or more deleted, substituted, inserted and/or added amino acids. Two or more different types of modifications selected from deletion, substitution, insertion, and addition may be carried out concurrently.

The biomolecule of interest of the present invention also encompasses "partial peptides" of the aforementioned protein. A partial peptide of the protein can be exemplified by a partial peptide comprising an amino acid sequence in which a portion of the amino acid sequence of the aforementioned protein runs uninterrupted, wherein the partial peptide preferably has the same activity as said protein. Such a partial peptide can be exemplified by a polypeptide that has an amino acid sequence comprising at least 20 and preferably at least 50 of the amino acid residues in the amino acid sequence of the aforementioned protein. This polypeptide preferably contains the amino acid sequence that corresponds to the region that is involved with the activity of the aforementioned protein. In addition, the partial peptide used in the present invention may also be a partial peptide as yielded by a modification of this polypeptide wherein 1 or a plurality of amino acid residues (for example, approximately 1 to 20, more preferably approximately 1 to 10, and even more preferably approximately 1 to 5) is deleted from, substituted in, inserted into, and/or added to its amino acid sequence. The partial peptide used in the present invention can also be used as an antigen for antibody production.

In one embodiment of the invention, the biomolecule of interest is selected from the group comprising Human growth hormone, human insulin, follicle-stimulating hormone, Factor VIII, Erythropoeietin, Granulocyte colony-stimulating factor, Alpha-glactosidase A, Alpha-L-iduronidase, N-actetylgalactosamine-4-sulfatase, Dornase alfa, Tisssue plasminogen activator, Glucocerebrosidase, Interferon, Insulin-like growth factor 1, bovine somatotropin, Porcine somatotropin, bovine chymosin, and envelop protein of the hepaptitis B virus.

The biomolecule of interest also encompasses modified polypeptides or proteins that have underwent posttranslational and post-export modifications in the periplasm such as cyclization, glycosylation, phophorylation, methylation, oxidation, dehydratation, proteolytic cleavage.

In one embodiment, the biomolecule of interest is an enzyme for metabolizing a biomolecule in the extracellular medium (herein referred as "extracellular biomolecule"). In one embodiment, the extracellular biomolecule comprises a polysaccharide or a lipid. In one embodiment of the invention, the polysaccharide comprises alginate, pectin, cellulose, cellobiose, laminarin, or a mixture thereof. In one embodiment of the invention, the lipid comprises a fatty acid, a glycolipid, a betaine lipid, a glycerolipid, a phospholipid, a glycerolphospholipid, a sphingolipid, a sterol lipid, a prenol lipid, a saccharolipid, a polyketide, or a mixture thereof. In one embodiment of the invention, the biomolecule of interest is an enzyme converting the polysaccharide to a monosaccharide, an oligosaccharide, or both.

In one embodiment of the invention, the biomolecule of interest is an enzyme converting the lipid to a fatty acid, a monosaccharide, or both. In one embodiment of the invention, the monosaccharide or oligosaccharide is oligoalginate, mannuronate, guluronate, mannitol, a-keto acid, 4-deoxy-L-erythro-hexoselulose uronate (DEHU), 2-keto-3-deoxy D-gluconate (KDG), glucose, glucuronate, galacturonate, galactose, xylose, arabinose, or mannose. In one embodiment of the invention, the fatty acid is 14:0, trans-14, 16:0, 16:ln-7, trans-16, 16:2n-6, 18:0, 18:ln-9, 18:2n-6, 18:3n-6, 18:3n-3, 18:4n-3, 20:0, 20:2n-6, 20:3n-6, 20:4n-3,20:4n-6, or 20:5n-3.

In one embodiment of the invention, the biomolecule of interest is an enzyme converting the extracellular biomolecule to a commodity chemical. In one embodiment of the invention, the commodity chemical is ethanol, butanol, or biodiesel. In one embodiment of the invention, the biodiesel is a fatty acid, a fatty acid ester, or a terpenoid.

As used herein, the term "inactivated" refers to the interruption or to the suppression of the expression of a gene at transcriptional or translational levels. Preferably, the term "inactivated" refers to a gene whose transcription is suppressed.

According to the invention, the inactivation of a gene may be due to the mutation of the gene or to the insertion of the expression system within the coding sequence of the gene. In the meaning of the present invention, the term "mutation" refers to a stable change in the genetic sequence. Examples of mutation which could lead to the inactivation of a gene in the present invention include, but are not limited to, point mutations, insertions, deletions and amplification or gene duplication.

Preferably, the mutation is a deletion. The term "deletion" as used herein means the loss or absence of a gene, preferably the total loss or absence of a gene. More preferably, the deletion starts at or before the start codon of the deleted gene, and ends at or after the stop codon of the deleted gene.

In one embodiment of the invention, the *S* gene is inactivated. The *S* gene encodes both a holin (S105) and an anti-holin (S107) protein. The holin protein triggers the formation of holes in the membrane. The holin is required for release of the endolysin encoded by the *R* gene. At the opposite, the antiholin protein inhibits the S105 hole formation. According to an embodiment, the *S* gene has the sequence SEQ ID NO: 1. In another embodiment, the sequence of the *S* gene presents a sequence identity of at least 70%, at least 75%, at least 80%, at least 85%, at least 90%, at least 91%, at least 92%, at least 93%, at least 94%, at least 95%, 5 at least 96%, at least 97%, at least 98%, and even more preferably of at least 99% with SEQ ID NO: 1.

The *S* gene may contain conservative sequence modifications that refer to amino acid modifications that do not significantly affect or alter the function of the *S* protein. Such conservative modifications include amino acid substitutions, additions and deletions. Modifications can be introduced into the *S* gene sequence by standard techniques known in the art, such as site-directed mutagenesis and PCR-mediated mutagenesis. Conservative amino acid substitutions are typically those in which an amino acid residue is replaced with an amino acid residue having a side chain with similar physicochemical properties. The modified sequence of the *S* protein may comprise one, two, three, four or more amino acid insertions, deletions or substitutions. Where substitutions are made, preferred substitutions will be conservative modifications. Families of amino acid residues having similar side chains have been defined in the art. These families include amino acids with basic side chains (e.g., lysine, arginine, histidine), acidic side chains (*e.g.,* aspartic acid, glutamic acid), uncharged polar side chains (*e.g.* glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (*e.g.* threonine, valine, isoleucine) and aromatic side chains (*e.g.,* tyrosine, phenylalanine, tryptophan, histidine). Thus, one or more amino acid residues within the sequence of the *S* protein can be replaced with other amino acid residues from the same side chain family and the modified *S* protein can be tested for retained function *(i.e.,* the properties set forth herein) by comparison with the *S* protein encoded by the sequence SEQ ID NO: 1.

The term "identity" or "identical", when used in a relationship between the sequences of two or more polypeptides, refers to the degree of sequence relatedness between polypeptides, as determined by the number of matches between strings of two or more amino acid residues. "Identity" measures the percent of identical matches between the smaller of two or more sequences with gap alignments (if any) addressed by a particular mathematical model or computer program (*i.e.,* "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics 5 and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48,1073 (1988). Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. \2, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

In another embodiment, the *R* gene is inactivated. The *R* protein is an endolysin: this transglycosylase degrades the murein of the cell wall of the host cell. According to an embodiment, the *R* gene has the sequence SEQ ID NO: 2. In another embodiment, the sequence of the *R* gene presents a sequence identity of at least 70%, at least 75%, at least 80%, at least 85%, at least 90%, at least 91%, at least 92%, at least 93%, at least 94%, at least 95%, 5 at least 96%, at least 97%, at least 98%, and even more preferably of at least 99% with SEQ ID NO: 2.

The *R* gene may contain conservative sequence modifications as described here above that refer to amino acid modifications that do not significantly affect or alter the function of the *R* protein. The modified sequence of the *R* protein may comprise one, two, three, four or more amino acid insertions, deletions or substitutions. Thus, one or more amino acid residues within the sequence of the *R* protein can be replaced with other amino acid residues from the same side chain family and the modified *R* protein can be tested for retained function (*i.e,* the properties set forth herein) by comparison with the *R* protein encoded by the sequence SEQ ID NO: 2.

In another embodiment, the *Rz* gene is inactivated. The *Rz* protein belongs to the spanin family. This protein may be involved in disrupting the outer membrane of the host cell during the lytic phase. According to an embodiment, the *Rz* gene has the sequence SEQ ID NO: 3. In another embodiment, the sequence of the *Rz* gene presents a sequence identity of at least 70%, at least 75%, at least 80%, at least 85%, at least 90%, at least 91%, at least 92%, at least 93%, at least 94%, at least 95%, 5 at least 96%, at least 97%, at least 98%, and even more preferably of at least 99% with SEQ ID NO: 3.

The *Rz* gene may contain conservative sequence modifications as described here above that refer to amino acid modifications that do not significantly affect or alter the function of the *Rz* protein. The modified sequence of the *Rz* protein may comprise one, two, three, four or more amino acid insertions, deletions or substitutions. Thus, one or more amino acid residues within the sequence of the *Rz* protein can be replaced with other amino acid residues from the same side chain family and the modified *Rz* protein can be tested for retained function *(i.e.,* the properties set forth herein) by comparison with the *Rz* protein encoded by the sequence SEQ ID NO: 3.

In another embodiment, the *Q* gene is inactivated. The *Q* protein is a late gene regulator: the *Q* protein is an antiterminator of RNA synthesis from the promoter used for transcription of the entire "late" region of lambda DNA. According to an embodiment, the *Q* gene has the sequence SEQ ID NO: 35. In another embodiment, the sequence of the *Q* gene presents a sequence identity of at least 70%, at least 75%, at least 80%, at least 85%, at least 90%, at least 91%, at least 92%, at least 93%, at least 94%, at least 95%, 5 at least 96%, at least 97%, at least 98%, and even more preferably of at least 99% with SEQ ID NO: 35.

The *Q* gene may contain conservative sequence modifications as described here above that refer to amino acid modifications that do not significantly affect or alter the function of the *Q* protein. The modified sequence of the *Q* protein may comprise one, two, three, four or more amino acid insertions, deletions or substitutions. Thus, one or more amino acid residues within the sequence of the *Q* protein can be replaced with other amino acid residues from the same side chain family and the modified *Q* protein can be tested for retained function *(i.e.,* the properties set forth herein) by comparison with the *Q* protein encoded by the sequence SEQ ID NO: 35.

In another embodiment, a nucleic acid fragment comprising the coding sequence of the S gene is deleted. According to the invention, said nucleic acid fragment does not comprise the coding sequences of the *ral* gene (SEQ ID NO: 4) and/or of the *N* gene (SEQ ID NO: 5). Therefore, according to an embodiment, when the phage is lambda, the region between residues 33930 and 35582 is not deleted.

According to another embodiment, when the phage is lambda(DE3), the region between residues 10813 and 11391 is not deleted.

Preferably, said nucleic acid fragment does not comprise the coding sequence of N and C2 (SEQ ID NO: 6), as well as the regulatory sequences of the promoter of *C2.* To make sure that the regulatory sequences of C2 are not deleted, the fragment to be deleted may begin at the start codon ATG of the following gene: the Cro gene.

More preferably, said nucleic acid fragment does not comprise the coding sequence of *ral, N* and C2 (SEQ ID NO: 6), as well as the regulatory sequences of the promoter of *C2.*

The C2 gene encodes a repressor protein, which is important for maintaining the lysogenic state. This gene is also called cI in the sequence of several other lambdoid phages. In the lambda phage, the coding sequence of the *ral, N* and *cI* genes is from residues 33930 to 38040 or from residues 33930 to 38022. In the lambda DE3 phage, the coding sequence of the *ral, N,* C2 genes is from residues 10813 to 12436.

In one embodiment, the length of said deleted nucleic acid fragment is from 30 kb to 300 b, preferably from 5 kb to 500 b. In another embodiment, the length of said deleted nucleic acid fragment is 30 kb, 25 kb, 20 kb, 15 kb, 10 kb, 9 kb, 8 kb, 7 kb, 6 kb, 5 kb, 4 kb, 3 kb, 2kb, 1 kb, 750 b, 500b.

According to one embodiment where the phage is lambda, the deleted nucleic acid fragment starts at a position ranging from the position 35582 to the position 45186, preferably from 38041 to 45186 or from 38023 to 45186 and ends at a position ranging from the position 45510 to the position 48502

According to another embodiment where the phage is lambda DE3, the deleted nucleic acid fragment starts at a position ranging from the position 11392 to the position 16764, preferably from 12437 to the position 16764 and ends at a position ranging from 17088 and 42925.

In another embodiment, the nucleic acid fragment to be deleted comprises at least the coding sequence of the *S* and *R* genes. According to one embodiment, when the phage is lambda, the nucleic acid fragment starts at a position ranging from the position 35582 to the position 45186, preferably from 38041 to 45186 or from 38023 to 45186, and ends at a position ranging from the position 45970 to the position 48502. In another embodiment, when the phage is lambda DE3, the nucleic acid fragment starts at a position ranging from the position 11392 to the position 16764, preferably from 12437 to the position 16764, and ends at a position ranging from 17548 and 42925.

In another embodiment, the nucleic acid fragment to be deleted comprises at least the coding sequence of the *S, R* and *Rz* genes. According to one embodiment, when the phage is lambda, the nucleic acid fragment starts at a position ranging from the position 35582 to the position 45186, preferably from 38041 to 45186 or from 38023 to 45186. According to this embodiment, the fragment may end at a position ranging from the position 46428 to the position 46458. Still according to this embodiment, the nucleic acid fragment may end at a position ranging from 46428 to 48502.

According to another embodiment, when the phage is lambda DE3, the nucleic acid fragment starts at a position ranging from the position 11392 to the position 16764, preferably from 12437 to the position 16764. According to this embodiment, the fragment may end at a position ranging from position 18006 and 18036. Still according to this embodiment, the nucleic acid fragment may end at a position ranging from position 18006 to 24496. Still according to this embodiment, the nucleic acid fragment may end at a position ranging from position 18006 to 42925.

According to another embodiment, the nucleic acid fragment to be deleted comprises at least the coding sequence of the *R* gene.

According to one embodiment, when the phage is lambda, the nucleic acid fragment starts at a position ranging from the position 35582 to the position 45493, preferably ranging from the position 38041 or 38022 to the position 45493. According to this embodiment, the nucleic acid fragment to be deleted may end at a position ranging from 45970 to the position 48502.

According to another embodiment, when the phage is lambda DE3, the nucleic acid fragment starts at a position ranging from the position 11392 to the position 17071, preferably ranging from the position 12437 to the position 17071. According to this embodiment, the nucleic acid fragment to be deleted may end at a position ranging from 17548 to the position 24496. Still according to this embodiment, the nucleic acid fragment to be deleted may end at a position ranging from 17548 to the position 42925.

According to another embodiment, the nucleic acid fragment to be deleted comprises at least the coding sequence of the *R* and *Rz* genes.

According to one embodiment, when the phage is lambda, the nucleic acid fragment starts at a position ranging from the position 35582 to the position 45493, preferably ranging from the position 38041 or 38022 to the position 45493. According to this embodiment, the nucleic acid fragment to be deleted may end at a position ranging from 46428 to the position 46458. Still according to this embodiment, the nucleic acid fragment to be deleted may end at a position ranging from 46428 to the position 48502.

According to another embodiment, when the phage is lambda DE3, the nucleic acid fragment starts at a position ranging from the position 11392 to the position 17071, preferably ranging from the position 12437 to the position 17071. According to this embodiment, the nucleic acid fragment to be deleted may end at a position ranging from 18006 to the position 18330. Still according to this embodiment, the nucleic acid fragment to be deleted may end at a position ranging from 18006 to the position 24496. Still according to this embodiment, the nucleic acid fragment to be deleted may end at a position ranging from 18006 to the position 42925.

According to another embodiment, the nucleic acid fragment to be deleted comprises at least the coding sequence of the *Q* gene.

According to one embodiment where the phage is lambda, the deleted nucleic acid fragment starts at a position ranging from the position 35582 to the position 43886, preferably from 38041 to 43886 or from 38023 to 43886 and ends at a position ranging from the position 44510 to the position 48502.

According to another embodiment where the phage is lambda DE3, the deleted nucleic acid fragment starts at a position ranging from the position 11392 to the position 15464, preferably from 12437 to the position 15464 and ends at a position ranging from 16088 and 42925.

According to another embodiment, the nucleic acid fragment to be deleted comprises at least the coding sequence of the *Q* and *S* genes.

According to one embodiment where the phage is lambda, the deleted nucleic acid fragment starts at a position ranging from the position 35582 to the position 43886, preferably from 38041 to 43886 or from 38023 to 43886 and ends at a position ranging from the position 45510 to the position 48502.

According to another embodiment where the phage is lambda DE3, the deleted nucleic acid fragment starts at a position ranging from the position 11392 to the position 15464, preferably from 12437 to the position 15464 and ends at a position ranging from 17088 and 42925.

According to another embodiment, the nucleic acid fragment to be deleted comprises at least the coding sequence of the *Q, S* and *R* genes.

According to one embodiment where the phage is lambda, the deleted nucleic acid fragment starts at a position ranging from the position 35582 to the position 43886, preferably from 38041 to 43886 or from 38023 to 43886 and ends at a position ranging from the position 45970 to the position 48502.

According to another embodiment where the phage is lambda DE3, the deleted nucleic acid fragment starts at a position ranging from the position 11392 to the position 15464, preferably from 12437 to the position 15464 and ends at a position ranging from 17548 and 42925.

According to another embodiment, the nucleic acid fragment to be deleted comprises at least the coding sequence of the *Q, S*, *R* and *Rz* genes.

According to one embodiment where the phage is lambda, the deleted nucleic acid fragment starts at a position ranging from the position 35582 to the position 43886, preferably from 38041 to 43886 or from 38023 to 43886 and ends at a position ranging from the position 46428 to the position 48502.

According to another embodiment where the phage is lambda DE3, the deleted nucleic acid fragment starts at a position ranging from the position 11392 to the position 15464, preferably from 12437 to the position 15464 and ends at a position ranging from 18006 and 42925.

In one embodiment, the *Int* gene is inactivated. The Int protein manages the insertion and the excision of phage genome into the host's genome. According to an embodiment, the *Int* gene has the sequence SEQ ID NO: 7. In another embodiment, the sequence of the *Int* gene presents a sequence identity of at least 70%, at least 75%, at least 80%, at least 85%, at least 90%, at least 91%, at least 92%, at least 93%, at least 94%, at least 95%, 5 at least 96%, at least 97%, at least 98%, and even more preferably of at least 99% with SEQ ID NO: 7.

The *Int* gene may contain conservative sequence modifications as described here above that refer to amino acid modifications that do not significantly affect or alter the function of the Int protein. The modified sequence of the Int protein may comprise one, two, three, four or more amino acid insertions, deletions or substitutions. Thus, one or more amino acid residues within the sequence of the Int protein can be replaced with other amino acid residues from the same side chain family and the modified Int protein can be tested for retained function *(i.e.,* the properties set forth herein) by comparison with the Int protein encoded by the sequence SEQ ID NO: 7.

In one embodiment, the *Xis* gene is inactivated. The Xis protein is an excisionase, which is involved in the process of excision of the lambda phage DNA out of the bacterial host chromosome. According to an embodiment, the *Xis* gene has the sequence SEQ ID NO: 8. In another embodiment, the sequence of the *Xis* gene presents a sequence identity of at least 70%, at least 75%, at least 80%, at least 85%, at least 90%, at least 91%, at least 92%, at least 93%, at least 94%, at least 95%, 5 at least 96%, at least 97%, at least 98%, and even more preferably of at least 99% with SEQ ID NO: 8.

The *Xis* gene may contain conservative sequence modifications as described here above that refer to amino acid modifications that do not significantly affect or alter the function of the Xis protein. The modified sequence of the Xis protein may comprise one, two, three, four or more amino acid insertions, deletions or substitutions. Thus, one or more amino acid residues within the sequence of the Xis protein can be replaced with other amino acid residues from the same side chain family and the modified Xis protein can be tested for retained function (*i.e.,* the properties set forth herein) by comparison with the Xis protein encoded by the sequence SEQ ID NO: 8.

In one embodiment, a nucleic acid fragment comprising the coding sequence of the *Int* gene is deleted. According to an embodiment, the phage is lambda and the nucleic acid fragment to be deleted comprises the residues from position 27812 to 28882. According to another embodiment, the phage is lambda and the nucleic acid fragment to be deleted starts at a position ranging from position 1 to position 27812; and ends at a position ranging from 28882 to 33929.

In one embodiment, a nucleic acid fragment comprising the coding sequence of the *Xis* gene is deleted.

According to one embodiment, the phage is lambda and the deleted nucleic acid fragment to be deleted comprises the residues from position 28860 to 29078. According to another embodiment, the phage is lambda and the fragment starts at a position ranging from position 1 to position 28860, and ends at a position ranging from position 29078 to position 33929.

According to another embodiment, the phage is DE3 and the deleted nucleic acid comprises the residues from position 5586 to 5804. According to another embodiment, the fragment to be deleted starts at a position ranging from position 1 to position 5586, and ends at a position ranging from position 5804 to position 10812.

In one embodiment, a nucleic acid fragment comprising the coding sequences of the *Xis* and *Int* genes is deleted. According to an embodiment, the phage is lambda and the nucleic acid fragment to be deleted comprises the residues from position 27812 to 29078. According to another embodiment, the phage is lambda and the nucleic acid fragment to be deleted starts at a position ranging from position 1 to position 27812; and ends at a position ranging from 29078 to 33929.

In one embodiment of the invention, the genetically modified phage of the invention is further inactivated for the *kil* gene. According to an embodiment, the *kil* gene has the sequence SEQ ID NO: 36. In another embodiment, the *kil* gene presents a sequence identity of at least 70, 75, 80, 85, 90, 91, 92, 93, 94, 95, 96, 97, 98, more preferably of at least 99% with SEQ ID NO: 36. Preferably, the coding sequence of the *kil* gene is deleted. According to one embodiment, the phage is lambda and the deleted nucleic acid fragment comprises the residues from position 33187 to 33331.

According to another embodiment, the phage is DE3 and the deleted nucleic acid comprises the residues from position 9913 to 10057.

According to an embodiment, the *attP* sequence is not deleted from the sequence of the phage of the invention. The *attP* sequence is SEQ ID NO: 9. The *attP* sequence is located from position 27586 and 27817 in the sequence of the Lambda Phage, and from position 42788 to 42925 and from 1 to 94 in the genome of the Lambda DE3 phage (the genome of the phage is circular, the *attP* sequence is thus continue).

In one embodiment of the invention, the genetically modified phage comprises the *attP* sequence and the sequence of the C2 gene. In another embodiment, the genetically modified phage consists of the *attP* sequence and the sequence of the C2 gene.

According to a preferred embodiment, one genetically modified phage of the invention P11 has the sequence SEQ ID NO: 10 and is (DE3) ΔS-C, Δxis-ea10 (DE3 refers to lambda phage DE3 wherein the T7 RNA polymerase gene has been integrated within the sequence of *int* gene). Said modified phage P11 corresponds to the sequence NC_001416 wherein the coding sequences of genes *S, R, Rz, Xis* and *Int* are deleted.

According to another embodiment, one genetically modified phage of the invention P12 corresponds to the sequence NC_001416 wherein the coding sequences of genes *Int* and *S* are deleted (one example of P12 is the sequence DE3 ΔS, Δxis-ea10).

According to another embodiment, one genetically modified phage of the invention P13 corresponds to the sequence NC_001416 wherein the coding sequences of genes *S, R, Rz, Q, Xis* and *Int* are deleted (one example of P13 is the sequence DE3 ΔS-C, Δxis-ea10, ΔQ).

According to another embodiment, one genetically modified phage of the invention P14 corresponds to the sequence NC_0014l6 wherein the coding sequences of genes *R* and *Int* are deleted (one example of P14 is the sequence DE3 ΔR).

According to another embodiment, one genetically modified phage of the invention P15 corresponds to the sequence NC_0014l6 wherein the coding sequences of genes *Q* and *Int* are deleted (one example of P15 is the sequence DE3 ΔQ).

According to another embodiment, one genetically modified phage of the invention P16 corresponds to the sequence NC_0014l6 wherein the coding sequences of genes *S* and *Xis* are deleted (one example of P16 is the sequence NC_001416 ΔS, Δxis-ea10).

According to another embodiment, one genetically modified phage of the invention P17 corresponds to the sequence NC_0014l6 wherein the coding sequences of genes *R* and *Xis* are deleted (one example of P17 is the sequence NC_001416 ΔR, Δxis-ea10).

According to another embodiment, one genetically modified phage of the invention P18 corresponds to the sequence NC_0014l6 wherein the coding sequences of genes *Q* and *Xis* are deleted (one example of P18 is the sequence NC_001416 Δxis-ea10, ΔQ).

According to another embodiment, one genetically modified phage of the invention p19 corresponds to the sequence NC_001416 wherein the coding sequences of genes *R, Rz* and *Int* are deleted (one example of P19 is the sequence DE3 ΔR, ΔRz).

According to another embodiment, one genetically modified phage of the invention P20 corresponds to the sequence NC_0014l6 wherein the coding sequences of genes *S, Rz* and *Int* are deleted (one example of P20 is the sequence DE3 ΔS, ΔRz).

According to another embodiment, one genetically modified phage of the invention P21 corresponds to the sequence NC_0014l6 wherein the coding sequences of genes *Rz, Q* and *Int* are deleted (one example of P21 is the sequence DE3 ΔRz, ΔQ).

According to another embodiment, one genetically modified phage of the invention P22 corresponds to the sequence NC_001416 wherein the coding sequences of genes *S, Q* and *Int* are deleted (one example of P22 is the sequence DE3 ΔS, ΔQ).

According to another embodiment, one genetically modified phage of the invention P23 corresponds to the sequence NC_0014l6 wherein the coding sequences of genes *R, Q,* and *Int* are deleted (one example of P23 is the sequence DE3 ΔR, ΔQ).

According to another embodiment, one genetically modified phage of the invention P24 corresponds to the sequence NC_001416 wherein the coding sequences of genes *S, R* and *Int* are deleted (one example of P24 is the sequence DE3 ΔS, ΔR).

According to another embodiment, one genetically modified phage of the invention P25 corresponds to the sequence NC_001416 wherein the coding sequences of genes *R, Rz* and *Xis* are deleted (one example of P25 is the sequence NC_001416 ΔR, Δxis-ea10, ΔRz).

According to another embodiment, one genetically modified phage of the invention P26 corresponds to the sequence NC_0014l6 wherein the coding sequences of genes *S, Rz* and *Xis* are deleted (one example of P26 is the sequence NC_001416 ΔS, Δxis-ea10, ΔRz).

According to another embodiment, one genetically modified phage of the invention P27 corresponds to the sequence NC_0014l6 wherein the coding sequences of genes *Q, Rz* and *Xis* are deleted (one example of P27 is the sequence NC_0014l6 ΔRz, Δxis-ea10, ΔQ).

According to another embodiment, one genetically modified phage of the invention P28 corresponds to the sequence NC_001416 wherein the coding sequences of genes *S, Q* and *Xis* are deleted (one example of P28 is the sequence NC_001416 ΔS, Δxis-ea10, ΔQ).

According to another embodiment, one genetically modified phage of the invention P29 corresponds to the sequence NC_001416 wherein the coding sequences of genes *R, Q* and *Xis* are deleted (one example of P29 is the sequence NC_001416 ΔR, Δxis-ea10, ΔQ).

According to another embodiment, one genetically modified phage of the invention P30 corresponds to the sequence NC_001416 wherein the coding sequences of genes *R, S* and *Xis* are deleted (one example of P30 is the sequence NC_001416 ΔS, Δxis-ea10, ΔR).

According to another embodiment, one genetically modified phage of the invention P31 corresponds to the sequence NC_001416 wherein the coding sequences of genes *R, Xis* and *Int* are deleted (one example of P31 is the sequence DE3 ΔR, Δxis-ea10).

According to another embodiment, one genetically modified phage of the invention P32 corresponds to the sequence NC_0014l6 wherein the coding sequences of genes *S, Xis* and *Int* are deleted (one example of P32 is the sequence DE3 ΔS, Δxis-ea10).

According to another embodiment, one genetically modified phage of the invention P33 corresponds to the sequence NC_0014l6 wherein the coding sequences of genes *Q, Xis* and *Int* are deleted (one example of P33 is the sequence DE3 Δxis-ea10, ΔQ).

According to another embodiment, one genetically modified phage of the invention P34 corresponds to the sequence NC_001416 wherein the coding sequences of genes *S, R, Xis* and *Int* are deleted (one example of P34 is the sequence DE3 ΔS, Δxis-ea10, ΔR).

According to another embodiment, one genetically modified phage of the invention P35 corresponds to the sequence NC_0014l6 wherein the coding sequences of genes *R, Q, Xis* and *Int* are deleted (one example of P35 is the sequence DE3 ΔR, Δxis-ea10, ΔQ).

According to another embodiment, one genetically modified phage of the invention P36 corresponds to the sequence NC_001416 wherein the coding sequences of genes *S, Q, Xis* and *Int* are deleted (one example of P36 is the sequence DE3 ΔS, Δxis-ea10, ΔQ).

According to another embodiment, one genetically modified phage of the invention P37 corresponds to the sequence NC_0014l6 wherein the coding sequences of genes *R, Rz, Xis* and *Int* are deleted (one example of P37 is the sequence DE3 ΔR, Δxis-ea10, ΔRz).

According to another embodiment, one genetically modified phage of the invention P38 corresponds to the sequence NC_0014l6 wherein the coding sequences of genes *S, Rz, Xis* and *Int* are deleted (one example of P38 is the sequence DE3 ΔS, Δxis-ea10, ΔRz).

According to another embodiment, one genetically modified phage of the invention P39 corresponds to the sequence NC_001416 wherein the coding sequences of genes *Rz, Q, Xis* and *Int* are deleted (one example of P39 is the sequence DE3 ΔRz, Δxis-ea10, ΔQ).

According to another embodiment, one genetically modified phage of the invention P40 corresponds to the sequence NC_001416 wherein the coding sequences of genes *S, R, Q* and *Int* are deleted (one example of P40 is the sequence DE3 ΔS, ΔR, ΔQ).

According to another embodiment, one genetically modified phage of the invention P41 corresponds to the sequence NC_001416 wherein the coding sequences of genes *S, R, Rz* and *Int* are deleted (one example of P41 is the sequence DE3 ΔS-C).

According to another embodiment, one genetically modified phage of the invention P42 corresponds to the sequence NC_0014l6 wherein the coding sequences of genes *R, Rz, Q* and *Int* are deleted (one example of P42 is the sequence DE3 ΔR, ΔRz, ΔQ).

According to another embodiment, one genetically modified phage of the invention P43 corresponds to the sequence NC_001416 wherein the coding sequences of genes *S*, *Rz, Q* and *Int* are deleted (one example of P43 is the sequence DE3 ΔS, ΔRz, ΔQ).

According to another embodiment, one genetically modified phage of the invention P44 corresponds to the sequence NC_001416 wherein the coding sequences of genes *S, R, Q* and *Xis* are deleted (one example of P44 is the sequence NC_001416 ΔS, ΔR, Δxis-ea10, ΔQ).

According to another embodiment, one genetically modified phage of the invention P45 corresponds to the sequence NC_001416 wherein the coding sequences of genes *S, R, Rz* and *Xis* are deleted (one example of P45 is the sequence NC_001416 ΔS-C, Δxis-ea10).

According to another embodiment, one genetically modified phage of the invention P46 corresponds to the sequence NC_0014l6 wherein the coding sequences of genes *R, Rz, Q* and *Xis* are deleted (one example of P46 is the sequence NC_0014l6 ΔR, ΔRz, Δxis-ea10, ΔQ).

According to another embodiment, one genetically modified phage of the invention P47 corresponds to the sequence NC_001416 wherein the coding sequences of genes *S, Rz, Q* and *Xis* are deleted (one example of P47 is the sequence NC_0014l6 ΔS, ΔRz, Δxis-ea10, ΔQ).

According to another embodiment, one genetically modified phage of the invention P48 corresponds to the sequence NC_001416 wherein the coding sequences of genes *S, R, Q, Xis* and *Int* are deleted (one example of P48 is the sequence DE3 ΔS, ΔR, Δxis-ea10, ΔQ).

According to another embodiment, one genetically modified phage of the invention P49 corresponds to the sequence NC_0014l6 wherein the coding sequences of genes *S, Rz, Q, Xis* and *Int* are deleted (one example of P49 is the sequence DE3 ΔS, ΔRz, Δxis-ea10, ΔQ).

According to another embodiment, one genetically modified phage of the invention P50 corresponds to the sequence NC_0014l6 wherein the coding sequences of genes *R, Rz, Q, Xis* and *Int* are deleted (one example of P50 is the sequence DE3 ΔR, ΔRz, Δxis-ea10, ΔQ).

According to another embodiment, one genetically modified phage of the invention P51 corresponds to the sequence NC_001416 wherein the coding sequences of genes *S, R, Q, Rz* and *Int* are deleted (one example of P51 is the sequence DE3 ΔS-C, ΔQ).

According to another embodiment, one genetically modified phage of the invention P52 corresponds to the sequence NC_001416 wherein the coding sequences of genes *S, R, Rz, Q* and *Xis* are deleted (one example of P52 is the sequence NC_001416 ΔS-C, Δxis-ea10, ΔQ).

According to another embodiment, one genetically modified phage of the invention P53 corresponds to the sequence NC_001416 wherein the coding sequences of genes *Q, Xis* and *Int* are deleted (one example of P53 is the sequence DE3 Δxis-ea10, ΔQ).

The present invention also relates to a process for producing the modified phage of the invention, wherein said process comprises at least two steps of deletion of genes.

In an embodiment, the process of the invention is carried out with a phage integrated in the genome of a host cell.

In an embodiment, the host cell is a microorganism, preferably a prokaryote, more preferably a bacterium, more preferably a gram negative bacterium. Advantageously, the host cell is a bacterium from the *Enterobacteriacea* family according to the current applicable taxonomy. If the taxonomy should change, the skilled artisan would know how to adapt the changes in the taxonomy to deduce the strains that could be used in the present invention. Examples of bacteria from the *Enterobacteriacea* family include, but are not limited to, bacteria belonging to the genera *Escherichia, Enterobacter, Erwinia, Klebsiella, Pantoea, Photorhabdus, Providencia, Salmonella, Serratia, Shigella, Morganella* and *Yersinia.* According to a preferred embodiment, the host cell belongs to the *Escherichia* genus, and more preferably the host cell is *Escherichia coli* (*E. coli*).

Methods for deleting genes from an integrated phage are well known to the skilled artisan. Examples of such methods include, but are not limited to homologous recombination (also called recombineering) using the lambda Red-encoded genes: *exo, bet* and *gam.* It is also possible to use the corresponding *recE* and *recT* genes from the prophage Rac. The genes *exo* and *recE* encode a 5'-3' exonuclease that produces 3' overhangs. The *bet* and *recT* genes encode a pairing or also called annealing protein that binds the 3' overhangs and mediates its annealing and homologous recombination between two complementary DNA sequences. The *gam* gene encodes an inhibitor of the *E. coli* RecBCD exonuclease and thus protects linear DNA fragments of interest. The method of recombineering was well described by several researchers including Datsenko and Wanner (PNAS 97-12, 6640-6645, 2000) and Stewart et al (WO0104288). The principle of the method is to generate (by PCR amplification for example) a DNA fragment containing the fragment to integrate and two recombination arms. These arms are homologous to the regions adjacent to the gene to be inactivated. They will be used to target the insertion of the fragment of interest. It is possible to create this kind of DNA fragment by PCR using primers containing homologous arms from 20 to 60 nucleotides.

The present invention also relates to a host cell comprising the genetically modified phage of the invention. In a preferred embodiment, the host cell of the invention comprises the genetically modified phage integrated in its genome.

In one embodiment, the host cell is a microorganism, preferably a prokaryote, more preferably a bacterium, more preferably a gram negative bacterium. Advantageously, the host cell is a bacterium from the *Enterobacteriacea* family according to the current applicable taxonomy. If the taxonomy should change, the skilled artisan would know how to adapt the changes in the taxonomy to deduce the strains that could be used in the present invention. Examples of bacteria from the *Enterobacteriacea* family include, but are not limited to, bacteria belonging to the genera *Escherichia, Enterobacter, Erwinia, Klebsiella, Pantoea, Photorhabdus, Providencia, Salmonella, Serratia, Shigella, Morganella* and *Yersinia.* According to a preferred embodiment, the host cell belongs to the *Escherichia* genus, and more preferably the host cell is *Escherichia coli* (*E. coli*). Examples of strains of *E. coli* which could be used in the present invention include, but are not limited to, strains derived from *E. Coli* K-12, *E. coli* B or *E. coli* W, such as, for example, MG1655, W3110, DG1, DG2, Top10, DH10B, DH5alpha, HMS174, BL21, BL21(DE3), HMS174(DE3), BL21(DE3) pLysS, BL21(DE3) pLysE.

In one embodiment, genes of the host cells may be inactivated.

In one embodiment, the gene *tonA* (also known as *fhuA,* SEQ ID NO: 11) is inactivated. The TonA/FhuA protein is a receptor for the phages T1, T5 and Phi80.

In one embodiment, the gene *galK* (SEQ ID NO: 12) is inactivated. The deletion of this gene allows the use of the *galK* positive/negative selection for deletion of genes by a method based on homologous recombination.

In one embodiment, the gene *araB* (SEQ ID NO: 13) is inactivated. In another embodiment, the gene *araA* (SEQ ID NO: 14) is inactivated. The inactivation of *araB* and/or *araA* is recommended for the use of the promoter PBAD (inducible by arabinose) within the host cell.

In one embodiment, the gene *lon* (SEQ ID NO: 15) and/or the gene *ompT* (SEQ ID NO: 16) are inactivated. The Lon protein is an ATP dependent protease. The OmpT protein is an outer membrane protease. Preferably, the genes *lon* and *ompT* are inactivated.

In one embodiment, the gene *rcsA* (SEQ ID NO: 17) is inactivated. The protein RcsA is a positive regulator of the synthesis of the capsule, which is degraded by the Lon protease. In one embodiment, the gene *hsdR* (SEQ ID NO: 18) and/or the gene *mrr* (SEQ ID NO: 19) are inactivated. The HsdR and Mrr proteins are restriction enzymes with different specificity. Preferably, the genes *hsdR* and *mrr* are both inactivated.

In one embodiment, the gene *endA* (SEQ ID NO: 20) and/or the gene *recA* (SEQ ID NO: 21) are inactivated. EndA is a DNA specific endonuclease. RecA is a recombination protein with protease and nuclease activity. Preferably, the genes *endA* and *recA* are both inactivated.

In one embodiment of the invention, at least one of the genes *tonA, galK, araB, araA, lon, ompT, rcsA, hsdR, mrr, endA* and *recA* are inactivated. Preferably, the inactivated genes are deleted.

In a preferred embodiment, the genes *tonA, galK, araB, lon, ompT, rcsA, hsdR, mrr, endA* and *recA* are inactivated. Preferably, the genes *tonA, galK, araB, lon, ompT, rcsA, hsdR, mrr, endA* and *recA* are deleted.

In one embodiment of the invention, the host cell of the invention is transformed with a nucleic acid sequence encoding a toxic molecule, as described in US2004/0115811. The presence of the nucleic acid sequence encoding the toxic molecule will allow the selection of recombinant clones having integrated a gene of interest and a nucleotide sequence encoding a functional antidote protein to a toxic molecule, wherein said recombinant clones are the ones which survive following their integration into a host cell comprising in its genome a nucleotide sequence encoding said toxic molecule.

According to a preferred embodiment of the present invention, the antidote protein and the toxic molecule are respectively, an anti-poison protein and a poison protein. Said anti-poison or poison proteins could be wild type proteins or modified proteins which are naturally or artificially poisonous and affect one or more vital functions of a cell (preferably, a prokaryote cell) and may lead to the killing of the cell.

The antidote protein and the toxic molecule are preferably selected from the group consisting of CcdA/CcdB proteins, Kis/Kid proteins, Phd/Doc proteins, RelB/relE proteins, PasB (or PasC)/PasA proteins, mazF/mazE proteins as described in US2004/0115811, or any other couple of anti-poison/poison molecules which are or are not of plasmid origin. The toxic molecule can also be a toxin protein being naturally or artificially toxic and affecting one or more vital functions of a (prokaryote) cell. The protein encoded by the gene sacB (from Bacillus amylolique-faciens), the protein GpE, the protein GATA-1 and the protein Crp are other examples of such toxic molecules. The gene *sacB* encodes the levan sucrase which catalyses the hydrolysis of sucrose into products which are toxic for *E. Coli* (Pierce et al. Proc. Natl. Acad. Sci., Vol. 89, N[deg.]6 (1992) p. 2056-2060). The protein GpE encodes the E genes from the bacteriophage [phi]X174 which includes six unique restriction sites and encodes gpE and which causes lysis of *E. Coli* cell (Heinrich et al., Gene, Vol. 42(3) (1986) p. 345-349). The protein GATA-1 has been described by Trudel et al. (Biotechniques 1996, Vol. 20(4), p. 684-693). The protein Crp has been described by Schlieper et al. (Anal. Biochem. 1998, Vol. 257(2), p. 203-209).

The antidote proteins to said toxic molecule are any protein able to reduce or suppress the effect of the corresponding toxic molecule on a cell (preferably a prokaryotic cell), when said toxic molecule is produced by said cell.

According to a preferred embodiment, the host cell of the invention comprises a nucleic acid sequence encoding the protein CcdB. The *ccdB* gene has the sequence SEQ ID NO: 22.

The present invention also relates to a kit comprising a host cell as hereinabove described, wherein the gene encoding a poison protein is inserted, and a plasmid carrying the nucleic acid sequence of the gene encoding the anti-poison protein. The expression of the anti-poison protein in the host cell is required for maintaining the viability of the host cell. In a preferred embodiment, the poison protein is encoded by the *ccdB* gene, and the anti-poison protein is encoded by the *ccdA* gene (SEQ ID NO: 23).

According to an embodiment of the invention, the plasmid of the kit may further contain, or may be modified to further contain, the nucleic acid sequence of the biomolecule of interest.

The present invention also refers to a process for preparing a host cell as hereinabove described.

In one embodiment, the process of the invention comprises a step of infection of the host cell by a genetically modified phage according to the invention. In one embodiment of the invention, said infection step includes the use of a helper phage. In the meaning of the present invention, the term "helper phage" refers to a phage used to complement a deletion or an inactivation of another phage. The helper phage will provide the missing functions to another phage to be able to infect bacteria or to prepare phage stock. Usually, the helper phage cannot form a lysogen by itself because it is cI minus (it has no repressor and is thus virulent).

The process for infecting a host cell with a phage using a helper phage is well known in the art. The first step is the preparation of the lysates and the second one is the lysogenization. Briefly, the bacterial lysates of the helper phage are prepared using standard methods as described in "Molecular cloning: a laboratory manual", Sambrook et al (2001, ISBN 978-087969577-4) or in "Large- and Small-Scale Preparation of Bacteriophage lambda lysate and DNA", Su et al, BioTechniques 25:44-46 (July 1998). Preparation of the phage of interest is done using the same principle, after phage induction (most often using UV irradiation or any situation where a lysogen undergoes DNA damage or the SOS response of the host or Cro production) in order to launch the lytic cycle and using a helper phage to provide the missing functions. An alternative to the helper phage is the use of a plasmid encoding the missing functions.

Next, the phage lysates are mixed with the targeted bacteria and plated on LB plates in order to get lysogens (as described in lambda DE3 lysogenization kit from Novagen, User Protocol TB031 or an alternative method is described in Studier and Moffat, Journal of Molecular Biology, 1986, 189:113-130). A selection phage can be used to select specifically bacteria containing the phage of interest. This selection phage is a virulent phage having the same immunity as the phage of interest. Consequently, the selection phage is unable to form plaques or to kill bacteria lysogens for the phage of interest because this phage produces the cI repressor (also called C2 in DE3 lambda phage).

The present invention thus also relates to a kit comprising the modified phage of the invention, as hereinabove described, and a helper phage. Examples of helper phages include, but are not limited to, helper phage B10 or any other lambdoid phage with a different immunity than the phage of interest (example: phage with immunity 434, 80, ...). Some helper phages and their manipulations were well described in the literature including in Haldimann and Wanner (Journal of Bacteriology, 183, 6384-6393, 2001).

In one embodiment, the process for preparing a host cell of the invention further comprises a step of deletion, wherein nucleic acid sequences of the host cell are deleted. Methods for deleting the sequence of a gene are well known by the skilled artisan. The more efficient method is the homologous recombination method mediated by the lambda Red-encoded genes or the *recE* and *recT* genes from the prophage Rac. As described above, this method was well described by several researchers including Datsenko and Wanner (PNAS 97-12, 6640-6645, 2000) and Stewart et al (WO0104288). PCR products are generated using primers with 20- to 60-nt extensions that are homologous to regions adjacent to the gene to be inactivated. Since only a small amount of bacteria will effectively recombine the fragment of interest, it is necessary to have a strong selection marker to select it. Antibiotic markers can be used to select the recombinants: the modified primers are used to amplify an antibiotic resistance gene. After transformation and activation of the recombination genes, recombinant bacteria are selected on medium containing the appropriate antibiotic. In this case, the targeted gene is replaced by an antibiotic resistance gene. In order to use the same strategy for the next deletion, it is necessary to remove this antibiotic resistance gene during a second step. As described in Datsenko and Wanner, it is possible to use antibiotic resistance gene that are flanked by FRT (FLP recognition target) sites. The resistance genes are then eliminated by using a helper plasmid encoding the FLP recombinase. The antibiotic resistance gene is removed by this site-specific recombinase but this method leaves traces: one site-specific recombination site is still present after removal of the antibiotic resistance gene.

To avoid the presence of this site, more preferably, the method of the invention uses *galK* as a marker gene. The principle of the galK selection is described in Warming et al. (Nucleic acid research, 2005, 33(4)). This method uses galK as a positive selection marker (growth on minmal medium containing galactose) during the first recombination (insertion). The removal of this marker is performed during a second homologous recombination step. During this step, galK is used as a negative selection marker on minimal medium containing 2-deoxy-galactose (DOG). The galK gene product, galactokinase, catalyzes the first step in the galactose degradation pathway. Galactokinase also efficiently catalyzes the phosphorylation of the DOG galactose analog. The product of this reaction cannot be further metabolized, leading to the accumulation of a toxic molecule (2-deoxy-galactose-1-phosphate). The advantage of this method is to avoid the presence of specific recombination site after deletion of the targeted gene and removal of the selective marker.

The present invention also relates to a process for producing a biomolecule of interest, comprising
- cultivating a host cell comprising the genetically modified phage according to the invention and the nucleic acid sequence of the biomolecule of interest,
- recovering the biomolecule of interest.

In one embodiment of the invention, the nucleic acid sequence of the biomolecule of interest is comprised within the expression system of the genetically modified phage. According to this embodiment, the production of the biomolecule of interest is direct, *i.e.,* results from the expression of the gene of the expression system, for example by culture in a medium wherein the promoter comprised in the expression system is induced.

In another embodiment of the invention, the expression system of the genetically modified phage comprises the nucleic acid sequence of the T7 RNA polymerase under the control of a lac promoter, preferably the lacUV5 promoter. According to this embodiment, the process for producing the biomolecule of interest comprises the transformation of the host cell with a plasmid comprising the nucleic acid sequence of the biomolecule of interest under the control of a the T7 promoter. The expression from the T7 promoter is under the control of T7 RNA polymerase, which is stringently specific for the T7 promoter, *i.e.,* the T7 promoter can only be utilized by the RNA polymerase of bacteriophage T7. When IPTG is added to the culture medium, T7 RNA polymerase is expressed by transcription from the lac promoter, and allows the expression of the biomolecule of interest.

In the meaning of the present invention, the term "T7 promoter" includes promoters that are present in the genome of bacteriophage T7, as well as consensus sequences and variants of such promoters with the ability to mediate transcription by the T7 RNA polymerase. The bacteriophage T7 contains seventeen different promoter sequences, all of which comprise a highly conserved nucleotide sequence.

According to a preferred embodiment, the plasmid comprising the nucleic acid sequence of the biomolecule of interest also comprises the nucleic acid sequence of *ccdA,* and the host cell comprises the sequence of *ccdB* integrated in its genome. Therefore, only recombinant clones containing the plasmid are propagated.

According to one embodiment, the biomolecule of interest is secreted by the host cell in the fermentation broth. According to this embodiment, the biomolecule of interest may be easily recovered from the fermentation broth.

According to another embodiment, the biomolecule of interest is not secreted by the host cell in the fermentation broth. Methods for recovering an intracellular biomolecule of interest are well-known in the art. Examples of such method include, but are not limited to, the use of trichloroacetic acid (TCA) or cracking buffer containing sodium dodecyl sulfate (SDS) to recover total cytoplasmic proteins in denaturing conditions or the use of sonication, French press or equivalent to disrupt bacteria under pressure in order to recover total cytoplasmic proteins in native (not denaturing) conditions. Next, the protein of interest can be purified using specific methods including but not limited to the use of affinity or ion exchange columns.

### BRIEF DESCRIPTION OF THE DRAWINGS

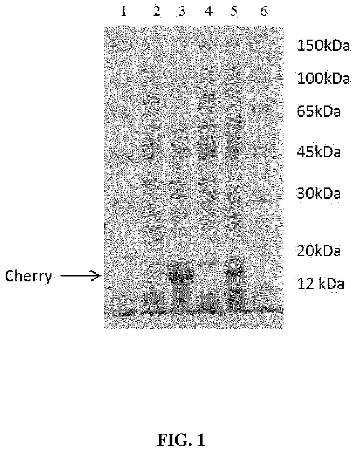

**Figure 1****: Picture of a SDS-Page gel colored with Coomassie blue staining, showing the production of the protein of interest.** 1 and 6: size; 2: pellet [pScherry1] M11DE3 before induction; 3: pellet [pScherry1] M11DE3 after induction; 4: pellet [pScherry1] HMS174DE3 before induction; 5: pellet [pScherry1] HMS174DE3 after induction. The array is for identifying the protein of interest.

### EXAMPLES

The present invention is further illustrated by the following examples.

### Example 1: Deletion of the xis, exo, bet, gam, kil, cIII, N and ral genes

### a) Deletion of xis DNA region and replacement by galK

First the galK gene was amplified by polymerase chain reaction (PCR) on pGalK plasmid using the primers XisgalKstart (5' *GGGGGTAAATCCCGGCGCTCATGACTTCGCCTTCTTCCCAGAATTCCTGTTGAC* AATTA 3', SEQ ID NO: 24) and XisgalK stop (5' *GTTCTGATTATTGGAAATCTTCTTTGCCCTCCAGTGTGAGCAGCACTGTCCTGCT* CCTTG 3', SEQ ID NO: 25). These primers contain at the 5' end a sequence of 40 bases identical to the DNA target (italicized). These sequences of 40 bases are the recombination arms. The 3' ends were designed to amplify the *galK* gene and its constitutive promoter. The DNA fragment amplified by PCR (1315bp) targeted the genes *xis, exo, bet, gam, kil* and *cIII* (DNA fragment of 5133bp): these genes were replaced during homologous recombination by the galK gene and its promoter. Electrocompetent bacteria carrying the T7(DE3) prophage and the pKD46 plasmid were prepared according to Datsenko and Wanner (PNAS 97-12, 6640-6645, 2000). Next, the amplified galK fragment was electroporated in these bacteria according to standard procedures (200ng of DNA fragment was used for each electroporation). SOC medium was added and bacteria were incubated during 1 hour at 37°C. Next, bacteria were washed (centrifuged, medium removal, addition of fresh medium and resuspension) twice with M9 minimal medium (Sambrook et al (2001, ISBN 978-087969577-4)) and plated on bacterial plates containing minimal M9 medium and 1% galactose. Plates were incubated at 37°C during 1 or 2 days.

The next step was the bacterial screening: PCR screening was performed directly on colonies using the Xis1 (5'GTCTTCAAGTGGAGCATCAG3', SEQ ID NO: 26) and Xis4 (5'ACCAGGACTATCCGTATGAC3', SEQ ID NO: 27) primers. An amplification of a 5774bp DNA fragment corresponds to a non-modified chromosome (non-recombinant colony) and, on the contrary, an amplification of a 1955bp DNA fragment corresponds to a recombinant chromosome. Bacteria allowing amplification of the 1955bp DNA fragment were selected and streaked two times on selective plates (minimal M9 medium supplemented with 1 % galactose) in order to purify it and to remove possible unmodified copies of the chromosome. The PCR screening was done one more time at the end of the purification step and 3 bacteria allowing amplification of the 1955bp DNA fragment were selected. The amplified DNA fragments corresponding to these bacteria were sequenced using the same primers in order to confirm the DNA recombination and the deletion of the Xis DNA region (*xis, exo, bet, gam, kil* and *cIII* genes).

### b) GalK removal

A DNA fragment containing large recombination arms (of 350bp and 343 bp) was constructed by PCR to remove galK and the N and ral genes. The first arm was amplified byPCR on bacterial colonies containing the T7(DE3) prophage using the Xis1 and Xis2 (5'CCAAACGGAACAGATGAAGAAGGCGAAGTCATGAG3', SEQ ID NO: 28) primers. A DNA fragment of 365 bases was amplified. The second arm was also performed by PCR on the same bacteria using the Xis3 (5' GACTTCGCCTTCTTCATCTGTTCCGTTTGGCTTCC3', SEQ ID NO: 29) and Xis6 (5' GTAATGGAAAGCTGGTAGTCG3', SEQ ID NO: 30) primers. A DNA of fragment of 358 bases was amplified. Both recombination arms were purified after agarose gel electrophoresis. Xis2 and Xis3 primers were designed to generate DNA fragments containing an identical sequence of 30 base pairs. This sequence was used to join both recombination arms in a third PCR using Xis1 and Xis6 primers. A DNA fragment of 693 bp was generated. This DNA fragment was electroporated in bacteria selected above and carrying the pKD46 plasmid (prepared as described in Datsenko and Wanner). SOC medium was added and bacteria were incubated at 37°C during 1 hour. Next, bacteria were washed twice with M9 medium and plated on selective plates containing M9 medium supplemented with 0,2% glycerol and 1% DOG. Plates were incubated during 2 days at 37°C. Several colonies were screened by PCR using the Xis5 (5' CAGCCGTAAGTCTTGATCTC3', SEQ ID NO: 31) and Xis7 (5'CAGCAGGCATGATCCAAGAG3', SEQ ID NO: 32) primers. An amplification of 3246bp corresponding to the unmodified DNA chromosome was always obtained instead of an amplification of 1122bp corresponding to the modified chromosome. The experiment was reproduced completely and independently three times without success: no bacteria comprising the desired deletion was obtained.

Consequently, we decided to remove only the GalK fragment and to leave the N and ral genes. The DNA fragment containing the recombination arms was generated as described above using Xis,1 Xis2b (5' TTTGCCCTCCAGTGTGAAGAAGGCGAAGTCATGAG3', SEQ ID NO: 33) for the first recombination arm (365bp) and Xis8 (5' CTCATGACTTCGCCTTCTTCACACTGGAGGGCAAAGAAG, SEQ ID NO: 34) and Xis4 for the second recombination arm (384bp). The joining PCR was performed using Xis1 and Xis4 primers and generated a DNA fragment of 714bp. This fragment was electroporated as described above in the bacteria selected and carrying the pKD46 plasmid. Bacteria were plated on the same selective plates containing DOG and incubated during two days at 37°C. PCR screening was performed using Xis5 and Xis6 primers. Bacteria showing an amplification of 1770bp (instead of an amplification of 3010bp corresponding to the unmodified chromosome) were selected and purified. The DNA fragment was sequenced using the Xis5 and Xis6 primers and showed the right removal of the galK gene. This recombination was performed only once since bacteria were obtained immediately.

These results show that it was not possible to remove galK associated to the N and ral genes using homologous recombination. However, using exactly the same procedure, we were able to remove galK alone.

In conclusion, we demonstrated that removal of the N and ral genes leads to the death of bacteria, which was unexpected.

### Example 2: Protein expression using MG1655

In order to test the protein production efficiency of the strain constructed according to the invention, small-scale expression test was performed using bacteria called M11(DE3). The genotype of this strain is MG1655 ΔgalK, Δrcs A, Δlon, ΔhsdR-mmr, ΔfhuA, ΔendA, ΔrecA, ΔaraB, ΔompT, λ-DES (T7pol, Δxis-ea10, ΔS-C). Since MG1655 is an *Escherichia coli* K-12, it was compared to another K-12 strain used as a standard in the field of protein production and called HSM174(DE3) (genotype: recA1, hsdR, λ-DE3, (Rif R)). Both strains were transformed with pSCherryl plasmid DNA (Delphi Genetics, Belgium). This plasmid encodes a protein called "cherry" easily detectable (by eyes, red color) under the control of the T7 promoter.

Protocol for a small-scale expression using IPTG:
1) Two Erlenmeyer flasks containing 10ml of LB medium were inoculated each with a single colony of the HMS174 (DE3) and the M11(DE3) carrying both the pSCherry1 plasmid and incubated at 30°C overnight.
2) Two new flasks containing fresh medium were inoculated with 1ml of the overnight cultures and incubated with shaking at 37°C until OD600 reached 0.6.
3) A sample (1ml) from each flask was taken and centrifuged. The medium was discarded and the pellet was kept on ice. The samples were the non-induced controls. To induce protein expression in the remaining culture, IPTG (Isopropyl β-D-1-thiogalactopyranoside, 90µl of a fresh 100mM stock solution) was added to reach a final concentration of 1mM in both flasks. Incubation of both flasks was continued for 4 hours.
4) At the end of the induction period, the Optical Density at 600nm was measured for each culture (1.09 for M1 1(DE3) and 1.13 for HMS174(DE3)). A 1ml sample of each flask was centrifuged at maximum speed (13000 g) for 10 min at 4°C. It was observed that the pellet was red according to the expression of the Cherry protein. The supernatant was discarded and 100µl of water was added to resuspend the bacteria. 100µl of "cracking" buffer (100mM DTT, 2% SDS, 80mM Tris-HCl, pH 6.8, 0.006% bromophenol blue, 15% glycerol) was also added to lyse the bacteria. The non-induced samples were treated with the same protocol except that only 60µl of water and 60µl of cracking buffer were used (according to the optical density of the samples).
5) The samples were heated at 70°C-100°C (10min.) to resuspend all proteins and to denature the proteins.
6) 10 µl of each sample was loaded on 12% SDS-PAGE gel and migrated during 2 hours at 100 Volt.
7) After migration, the proteins were colored with Coomassie-blue staining.

As shown on figure 1, both strains were able to produce the protein of interest (Cherry protein, indicated by the array) but the production is about 5 to 10 times higher using M1 1(DE3) than using HMS174(DE3). The experiment was performed twice with exactly the same results.

### Example 3: Protein expression using BL21(DE3)

The BL21(DE3) strains deleted of Axis-ea10 and/or ΔS-C were constructed according to example 1. The deletion ΔS-C was inserted in the chromosome of the bacteria (in lambda DE3 deleted of the *int* gene and encoding the T7 RNA polymerase) alone or in combination with the Axis-ea10 deletion. Two BL21(DE3) derivatives were thus constructed: BL21(DE3) ΔS-C and BL21(DE3) Δxis-ea10, ΔS-C. The deletions were confirmed by PCR amplification of the modified region and this region was sequenced. This sequencing step confirmed the presence of the corresponding deletions according to the theoretical sequences. Next, the constructed bacteria were tested for protein expression using two different plasmids encoding the cherry protein (pSCherry1 and pSSC-Cherry1, Delphi Genetics) according to example 2. The results showed clearly over-expression of the cherry gene encoding the cherry protein. This protein was easily detected by eyes and on SDS-PAGE analysis.

We thus conclude that these bacteria are able to over-produce proteins without risk to unwanted bacterial lysis due to the lambda DE3 phage because this phage is at least deleted of the *int, S, R* and *Rz* genes encoding functions required for phage excision and bacterial lysis.

Since bacterial lysis during protein depends on several parameters including growth conditions, protein overexpressed... we designed a model strain to show that the constructed strains are resistant to lysis due to the specific gene deletions. In lambda phage, it is required to have a constitutive expression of the CI repressor in order to repress lytic development and to maintain lysogenic state of the prophage. A mutant (CI⁸⁵⁷) of this repressor is well known due to its ability to be thermosensitive: at 30°C, the mutated repressor is fully active and it maintains the lysogenic state. However, at higher temperature (37°C to 42°C), the CI⁸⁵⁷ is not efficient and it induces the lytic state of lambda phage. Strains carrying this CI⁸⁵⁷ mutation and the deletions according to the invention were constructed: BL21(DE3) ΔS-C corresponding to a BL21 strain comprising P41; BL21(DE3) Axis-ea10 and ΔS-C corresponding to a BL21 strain comprising P11; BL21(DE3) Δxis-ea10, ΔS-C and ΔQ corresponding to a BL21 strain comprising P13; BL21 (DE3) ΔQ corresponding to a BL21 strain comprising P15 and BL21(DE3) Δxis-ea10 and ΔQ corresponding to a BL21 strain comprising P53.

By shifting the temperature to 42°C, lytic state is induced. No lysis was observed for the strains of the invention. In addition, we observed that yield of production of the protein of interest (cherry protein) was improved for the strains of the invention compared to control.

## Claims

1. A bacterial host cell comprising a genetically modified phage wherein
- an expression system is inserted, said expression system comprising a nucleic acid sequence encoding a protein, said protein further inducing the expression of a biomolecule of interest, and
- the *int, S, R, Rz, Xis* genes are deleted, and
wherein said genetically modified phage is integrated in the genome of said bacterial host cell, and
wherein the phage is the lambda phage, the 434phage, the phi80 phage, the phi81 phage, the HK97 phage, or the P21 phage.

2. The bacterial host cell according to claim **1,** wherein the *Q* gene is further inactivated.

3. The bacterial host cell according to claim **1** or **2,** wherein the promoter comprised in the expression system is inducible.

4. The bacterial host cell according to any one of claims **1** to **3,** wherein the expression system is the T7 expression system.

5. The bacterial host cell according to any one of claims **1** to **4,** wherein said phage is one of the phages P11 or P13, wherein
- P11 comprises the sequence NC_001416 wherein the coding sequences of genes *S, R, Rz, Xis* and *Int* are deleted, and
- P13 comprises the sequence NC_001416 wherein the coding sequences of genes *S, R, Rz, Q, Xis* and *Int* are deleted.

6. The bacterial host cell according to any one of claims **1** to **5,** wherein the bacterial host cell is an enterobacteria, preferably *E. coli,* more preferably BL21.

7. The bacterial host cell according to any one of claims **1** to **6,** further comprising the inactivation of at least one of the genes *tonA, galK, araB, araA, lon, ompT, rcsA, hsdR, mrr, endA* and *recA.*

8. The bacterial host cell according to any one of claims **1** to **7,** comprising the insertion of the *ccdb* gene.

9. A kit comprising the bacterial host cell according to claim **8** and a plasmid comprising the *ccdA* gene.

10. A process for preparing the bacterial host cell according to any one of claims **1** to **8,** comprising infecting a bacterial host cell with a genetically modified phage wherein
- an expression system is inserted, said expression system comprising a nucleic acid sequence encoding a protein, said protein further inducing the expression of a biomolecule of interest,
- the *int, S, R, Rz, Xis* genes are deleted, and
- the phage is the lambda phage, the 434 phage, the phi80 phage, the phi81 phage, the HK97 phage, or the P21 phage,
and using a helper phage for complementation.

11. A process for producing a biomolecule of interest, comprising
- cultivating a bacterial host cell according to any one of claims **1** to **8,** wherein said host cell comprises the nucleic acid sequence of the biomolecule of interest,
- expressing said biomolecule of interest.

## Patentansprüche

1. Bakterielle Wirtszelle, die einen genetisch modifizierten Phagen umfasst, wobei
- ein Expressionssystem inseriert wird, wobei das Expressionssystem eine Nukleinsäuresequenz umfasst, die für ein Protein kodiert ist, wobei das Protein weiterhin die Expression eines Biomoleküls von Bedeutung induziert, und
- *int-, S-, R-, Rz-, Xis-Gene* entfernt werden, und
wobei der genetisch modifizierte Phage in das Genom der bakteriellen Wirtszelle integriert wird, und
wobei der Phage der Lambda-Phage, der 434-Phage, der phi80-Phage, der phi81-Phage, der HK97-Phage oder der P21-Phage ist.

2. Bakterielle Wirtszelle nach Anspruch **1,** wobei das Q-Gen weiterhin inaktiviert ist.

3. Bakterielle Wirtszelle nach Anspruch **1** oder **2,** wobei der im Expressionssystem enthaltene Promotor induzierbar ist.

4. Bakterielle Wirtszelle nach einem der Ansprüche **1** bis **3,** wobei das Expressionssystem das T7-Expressionssystem ist.

5. Bakterielle Wirtszelle nach einem der Ansprüche **1** bis **4,** wobei der Phage einer der Phagen P11 oder P13 ist, wobei
- P11 die Sequenz NC_001416 umfasst, wobei die kodierenden Sequenzen der *S*-, *R*-, *Rz-, Xis-* und *Int*-Gene entfernt werden, und
- P13 die Sequenz NC_001416 umfasst, wobei die kodierenden Sequenzen der *S*-, *R*-, *Rz-, Q*-, *Xis-* und *Int*-Gene entfernt werden.

6. Bakterielle Wirtszelle nach einem der Ansprüche **1** bis **5,** wobei die bakterielle Wirtszelle ein Enterobakterium, vorzugsweise *E. coli,* besonders bevorzugt BL21, ist.

7. Bakterielle Wirtszelle nach einem der Ansprüche **1** bis **6,** weiterhin das Inaktivieren von mindestens einem der Gene *tonA, galK, araB, araA, lon, ompT, rcsA, hsdR, mrr, endA* und *recA* umfassend.

8. Bakterielle Wirtszelle nach einem der Ansprüche **1** bis **7,** die die Insertion des *ccdb-*Gens umfasst.

9. Kit, das die bakterielle Wirtszelle nach Anspruch **8** umfasst, und ein Plasmid, das das ccdA-Gen umfasst.

10. Verfahren zur Vorbereitung der bakteriellen Wirtszelle nach einem der Ansprüche **1** bis **8,** das die Infizierung einer bakteriellen Wirtszelle mit einem genetisch modifizierten Phagen umfasst, wobei
- ein Expressionssystem inseriert wird, wobei das Expressionssystem eine Nukleinsäuresequenz umfasst, die ein Protein kodiert, wobei das Protein weiterhin die Expression eines Biomoleküls von Bedeutung induziert,
- die *int-, S-, R-, Rz-, Xis*-Gene entfernt werden und
- der Phage der Lambda-Phage, der 434-Phage, der phi80-Phage, der phi81-Phage, der HK97-Phage oder der P21-Phage ist,
und eine Helfer-Phage zur Komplementierung verwendet.

11. Verfahren zur Herstellung eines Biomoleküls von Bedeutung, umfassend
- Kultivieren einer bakteriellen Wirtszelle nach einem der Ansprüche **1** bis **8,** wobei die Wirtszelle die Nukleinsäuresequenz des Biomoleküls von Bedeutung umfasst,
- Exprimieren des Biomoleküls von Bedeutung.

## Revendications

1. Une cellule hôte bactérienne, comprenant un phage génétiquement modifié dans lequel
- un système d'expression est inséré, ledit système d'expression comprenant une séquence d'acide nucléique codant une protéine, ladite protéine induisant en outre l'expression d'une biomolécule d'intérêt, et
- les gènes *int, S, R, Rz, Xis* sont délétés, et
dans laquelle ledit phage génétiquement modifié est intégré dans le génome de ladite cellule hôte bactérienne, et
dans laquelle le phage est le phage lambda, le phage 434, le phage phi80, le phage phi81, le phage HK97, ou le phage P21.

2. La cellule hôte bactérienne selon la revendication **1,** dans laquelle le gène *Q* est en outre inactivé.

3. La cellule hôte bactérienne selon la revendication **1** ou **2,** dans laquelle le promoteur compris dans le système d'expression est inductible.

4. La cellule hôte bactérienne selon l'une quelconque des revendications **1** à **3,** dans laquelle le système d'expression est le système d'expression T7.

5. La cellule hôte bactérienne selon l'une quelconque des revendications **1** à **4,** dans laquelle ledit phage est l'un des phages P11, ou P13 dans lequel
- P11 comprend la séquence NC_001416 dans laquelle les séquences codantes des gènes *S, R, Rz, Xis* et *Int* sont délétés, et
- P13 comprend la séquence NC_001416 dans laquelle les séquences codantes des gènes *S, R, Rz, Q, Xis* et *Int* sont délétés.

6. La cellule hôte bactérienne selon l'une quelconque des revendications **1** à **5,** dans laquelle la cellule hôte bactérienne est une entérobactérie, de préférence *E. coli,* plus préférablement BL21.

7. La cellule hôte bactérienne selon l'une quelconque des revendications **1** à **6,** comprenant en outre l'inactivation d'au moins un des gènes *tonA, galK, araB, araA, lon, ompT, rcsA, hsdR, mrr, endA* and *recA.*

8. La cellule hôte bactérienne selon l'une quelconque des revendications **1** à **7,** comprenant l'insertion du gène *ccdb.*

9. Un kit comprenant la cellule hôte bactérienne selon la revendication **8** et un plasmide comprenant le gène *ccdA.*

10. Un procédé pour préparer la cellule hôte bactérienne selon l'une quelconque des revendications **1** à **8,** comprenant l'infection d'une cellule hôte bactérienne par un phage génétiquement modifié dans lequel
- un système d'expression est inséré, ledit système d'expression comprenant une séquence d'acide nucléique codant une protéine, ladite protéine induisant en outre l'expression d'une biomolécule d'intérêt,
- les gènes *int, S, R, Rz, Xis* sont délétés, et
- le phage est le phage lambda, le phage 434, le phage phi80, le phage phi81, le phage HK97, ou le phage P21,
et utilisant un phage auxiliaire pour la complémentation.

11. Un procédé de production d'une biomolécule d'intérêt, comprenant
- la culture d'une cellule hôte bactérienne selon l'une quelconque des revendications **1** à **8,** dans laquelle ladite cellule hôte comprend la séquence d'acide nucléique de la biomolécule d'intérêt,
- l'expression de ladite biomolécule d'intérêt.


### SEQUENCE LISTING

<110> DELPHI GENETICS
   Szpirer, CÃ©dric
<120> GENETICALLY MODIFIED PHAGE AND USE THEREOF
<130> CV-153/PCT
<160> 36
<170> BiSSAP 1.0
<210> 1
   <211> 324
   <212> DNA
   <213> Enterobacteria phage lambda
<220>
   <221> source
   <222> 1..324
   <223> /mol_type="DNA" /organism="Enterobacteria phage lambda"
<400> 1
   atgaagatgc cagaaaaaca tgacctgttg gccgccattc tcgcggcaaa ggaacaaggc      60
   atcggggcaa tccttgcgtt tgcaatggcg taccttcgcg gcagatataa tggcggtgcg     120
   tttacaaaaa cagtaatcga cgcaacgatg tgcgccatta tcgcctggtt cattcgtgac     180
   cttctcgact tcgccggact aagtagcaat ctcgcttata taacgagcgt gtttatcggc     240
   tacatcggta ctgactcgat tggttcgctt atcaaacgct tcgctgctaa aaaagccgga     300
   gtagaagatg gtagaaatca ataa                                            324
<210> 2
   <211> 477
   <212> DNA
   <213> Enterobacteria phage lambda
<220>
   <221> source
   <222> 1..477
   <223> /mol_type="DNA" /organism="Enterobacteria phage lambda"
<400> 2
   atggtagaaa tcaataatca acgtaaggcg ttcctcgata tgctggcgtg gtcggaggga      60
   actgataacg gacgtcagaa aaccagaaat catggttatg acgtcattgt aggcggagag     120
   ctattcactg attactccga tcaccctcgc aaacttgtca cgctaaaccc aaaactcaaa     180
   tcaacaggcg ccggacgcta ccagcttctt tcccgttggt gggatgccta ccgcaagcag     240
   cttggcctga aagacttctc tccgaaaagt caggacgctg tggcattgca gcagattaag     300
   gagcgtggcg ctttacctat gattgatcgt ggtgatatcc gtcaggcaat cgaccgttgc     360
   agcaatatct gggcttcact gccgggcgct ggttatggtc agttcgagca taaggctgac     420
   agcctgattg caaaattcaa agaagcgggc ggaacggtca gagagattga tgtatga        477
<210> 3
   <211> 462
   <212> DNA
   <213> Enterobacteria phage lambda
<220>
   <221> source
   <222> 1..462
   <223> /mol_type="DNA" /organism="Enterobacteria phage lambda"
<400> 3
   atgagcagag tcaccgcgat tatctccgct ctggttatct gcatcatcgt ctgcctgtca 60
   tgggctgtta atcattaccg tgataacgcc attacctaca aagcccagcg cgacaaaaat 120
   gccagagaac tgaagctggc gaacgcggca attactgaca tgcagatgcg tcagcgtgat 180
   gttgctgcgc tcgatgcaaa atacacgaag gagttagctg atgctaaagc tgaaaatgat 240
   gctctgcgtg atgatgttgc cgctggtcgt cgtcggttgc acatcaaagc agtctgtcag 300
   tcagtgcgtg aagccaccac cgcctccggc gtggataatg cagcctcccc ccgactggca 360
   gacaccgctg aacgggatta tttcaccctc agagagaggc tgatcactat gcaaaaacaa 420
   ctggaaggaa cccagaagta tattaatgag cagtgcagat ag 462
<210> 4
   <211> 201
   <212> DNA
   <213> Enterobacteria phage lambda
<220>
   <221> source
   <222> 1..201
   <223> /mol_type="DNA" /organism="Enterobacteria phage lambda"
<400> 4
   atgactacga ctattgataa aaatcaatgg tgtggacaat tcaagcgatg caatggatgc 60
   aagctgcaat cggaatgcat ggttaagcct gaagaaatgt ttcctgtaat ggaagatggg 120
   aaatatgtcg ataaatgggc aatacgaacg acggcaatga ttgccagaga acttggtaaa 180
   cagaacaaca aagctgcctg a 201
<210> 5
   <211> 300
   <212> DNA
   <213> Enterobacteria phage lambda
<220>
   <221> source
   <222> 1..300
   <223> /mol_type="DNA" /organism="Enterobacteria phage lambda"
<400> 5
   atggtaacca ttgtctggaa agaatccaaa ggtacggcaa aaagccgcta caaagctcgc 60
   agagcagaac ttattgccga gcgacgcagt aatgaagcac tggcgcgaaa aattgcgcta 120
   aagctctctg gttgcgtcag agcagataaa gcagcatcac tcggaagcct tcgctgcaag 180
   aaggcagaag aagtcgagcg taaacagaac cgtatttact acagcaagcc acgcagtgaa 240
   atgggtgtga cttgtgttgg tcgccagaaa attaaattag gcagcaaacc acttatttga 300
<210> 6
   <211> 651
   <212> DNA
   <213> Enterobacteria phage lambda
<220>
   <221> source
   <222> 1..651
   <223> /mol_type="DNA" /organism="Enterobacteria phage lambda"
<400> 6
   atgaatacac aactgatggg tgagcgtatt cgcgctcgca gaaaagaact caagattagg      60
   caggctgccc ttggcaagat ggttggcgtg tctaatgttg ctatttccca atgggagcga     120
   tctgaaactg agcccaatgg cgaaaaccta ttggccttag ccaaggcttt gcagtgctcc     180
   cctgattacc tgttgaaagg agaggatagt ctttcaaaca ttgcctatca cagcaggcat     240
   gatccaagag gttcgtatcc tctaattagt tgggtaagcg caggatgttg gatggaagct     300
   gtagagccat atcataggcg tgcaatagat aactggtacg acacaacggt agattgttct     360
   gaagactctt tttggctcga cgttaaaggc gattcaatga ctgccccggc aggactgagt     420
   attcctgagg ggatgattat tctcgtcgac ccagaagtcg aaccacgtaa tggaaagctg     480
   gtagtcgcca aacttgaagg agaaaacgag gcgacattca aaaagttagt tattgatgcc     540
   ggtagaaaat tcctgaaacc actcaatcca caatacccaa tgattgaaat caatgggaac     600
   tgtaaaatca ttggcgttgt cgttgatgcc aagctagcaa accttcctta a              651
<210> 7
   <211> 1071
   <212> DNA
   <213> Enterobacteria phage lambda
<220>
   <221> source
   <222> 1..1071
   <223> /mol_type="DNA" /organism="Enterobacteria phage lambda"
<400> 7
   atgggaagaa ggcgaagtca tgagcgccgg gatttacccc ctaaccttta tataagaaac      60
   aatggatatt actgctacag ggacccaagg acgggtaaag agtttggatt aggcagagac     120
   aggcgaatcg caatcactga agctatacag gccaacattg agttattttc aggacacaaa     180
   cacaagcctc tgacagcgag aatcaacagt gataattccg ttacgttaca ttcatggctt     240
   gatcgctacg aaaaaatcct ggccagcaga ggaatcaagc agaagacact cataaattac 300
   atgagcaaaa ttaaagcaat aaggaggggt ctgcctgatg ctccacttga agacatcacc 360
   acaaaagaaa ttgcggcaat gctcaatgga tacatagacg agggcaaggc ggcgtcagcc 420
   aagttaatca gatcaacact gagcgatgca ttccgagagg caatagctga aggccatata 480
   acaacaaacc atgtcgctgc cactcgcgca gcaaaatcag aggtaaggag atcaagactt 540
   acggctgacg aatacctgaa aatttatcaa gcagcagaat catcaccatg ttggctcaga 600
   cttgcaatgg aactggctgt tgttaccggg caacgagttg gtgatttatg cgaaatgaag 660
   tggtctgata tcgtagatgg atatctttat gtcgagcaaa gcaaaacagg cgtaaaaatt 720
   gccatcccaa cagcattgca tattgatgct ctcggaatat caatgaagga aacacttgat 780
   aaatgcaaag agattcttgg cggagaaacc ataattgcat ctactcgtcg cgaaccgctt 840
   tcatccggca cagtatcaag gtattttatg cgcgcacgaa aagcatcagg tctttccttc 900
   gaaggggatc cgcctacctt tcacgagttg cgcagtttgt ctgcaagact ctatgagaag 960
   cagataagcg ataagtttgc tcaacatctt ctcgggcata agtcggacac catggcatca 1020
   cagtatcgtg atgacagagg cagggagtgg gacaaaattg aaatcaaata a 1071
<210> 8
   <211> 219
   <212> DNA
   <213> Enterobacteria phage lambda
<220>
   <221> source
   <222> 1..219
   <223> /mol_type="DNA" /organism="Enterobacteria phage lambda"
<400> 8
   atgtacttga cacttcagga gtggaacgca cgccagcgac gtccaagaag ccttgaaaca 60
   gttcgtcgat gggttcggga atgcaggata ttcccacctc cggttaagga tggaagagag 120
   tatctgttcc acgaatcagc ggtaaaggtt gacttaaatc gaccagtaac aggtggcctt 180
   ttgaagagga tcagaaatgg gaagaaggcg aagtcatga 219
<210> 9
   <211> 232
   <212> DNA
   <213> Enterobacteria phage lambda
<220>
   <221> source
   <222> 1..232
   <223> /mol_type="DNA" /organism="Enterobacteria phage lambda"
<400> 9
   tacaggtcac taataccatc taagtagttg attcatagtg actgcatatg ttgtgtttta     60
   cagtattatg tagtctgttt tttatgcaaa atctaattta atatattgat atttatatca    120
   ttttacgttt ctcgttcagc ttttttatac taagttggca ttataaaaaa gcattgctta    180
   tcaatttgtt gcaacgaaca ggtcactatc agtcaaaata aaatcattat tt            232
<210> 10
   <211> 29865
   <212> DNA
   <213> artificial sequences
<220>
   <221> source
   <222> 1..29865
   <223> /mol_type="DNA" /note="Genetically modified phage" /organism="artificial sequences"
<400> 10
   gcttttttat actaagttgg cattataaaa aagcattgct tatcaatttg ttgcaacgaa      60
   caggtcacta tcagtcaaaa taaaatcatt atttgatttc aattttgtcc cactccctgc     120
   ctctgtcatc acgatactgt gatgccatgg tgtccgactt atgcccgaga agatgttgag     180
   caaacttatc gcttatctgc ttctcataga gtcttgcaga caaactgcgc aactcgtgaa     240
   aggtaggcgg atccagatcc cggacaccat cgaatggcgc aaaacctttc gcggtatggc     300
   atgatagcgc ccggaagaga gtcaattcag ggtggtgaat gtgaaaccag taacgttata     360
   cgatgtcgca gagtatgccg gtgtctctta tcagaccgtt tcccgcgtgg tgaaccaggc     420
   cagccacgtt tctgcgaaaa cgcgggaaaa agtggaagcg gcgatggcgg agctgaatta     480
   cattcccaac cgcgtggcac aacaactggc gggcaaacag tcgttgctga ttggcgttgc     540
   cacctccagt ctggccctgc acgcgccgtc gcaaattgtc gcggcgatta aatctcgcgc     600
   cgatcaactg ggtgccagcg tggtggtgtc gatggtagaa cgaagcggcg tcgaagcctg     660
   taaagcggcg gtgcacaatc ttctcgcgca acgcgtcagt gggctgatca ttaactatcc     720
   gctggatgac caggatgcca ttgctgtgga agctgcctgc actaatgttc cggcgttatt     780
   tcttgatgtc tctgaccaga cacccatcaa cagtattatt ttctcccatg aagacggtac     840
   gcgactgggc gtggagcatc tggtcgcatt gggtcaccag caaatcgcgc tgttagcggg     900
   cccattaagt tctgtctcgg cgcgtctgcg tctggctggc tggcataaat atctcactcg     960
   caatcaaatt cagccgatag cggaacggga aggcgactgg agtgccatgt ccggttttca    1020
   acaaaccatg caaatgctga atgagggcat cgttcccact gcgatgctgg ttgccaacga    1080
   tcagatggcg ctgggcgcaa tgcgcgccat taccgagtcc gggctgcgcg ttggtgcgga    1140
   tatctcggta gtgggatacg acgataccga agacagctca tgttatatcc cgccgttaac    1200
   caccatcaaa caggattttc gcctgctggg gcaaaccagc gtggaccgct tgctgcaact    1260
   ctctcagggc caggcggtga agggcaatca gctgttgccc gtctcactgg tgaaaagaaa    1320
   aaccaccctg gcgcccaata cgcaaaccgc ctctccccgc gcgttggccg attcattaat    1380
   gcagctggca cgacaggttt cccgactgga aagcgggcag tgagcgcaac gcaattaatg     1440
   taagttagct cactcattag gcaccccagg ctttacactt tatgcttccg gctcgtataa     1500
   tgtgtggaat tgtgagcgga taacaatttc acacaggaaa cagctatgac catgattacg     1560
   gattcactgg ccgtcgtttt acaacgtcgt gactgggaaa accctggcgt tacccaactt     1620
   aatcgccttg cagcacatcc ccctttcgcc agctggcgta atagcgaaga ggcccgcacc     1680
   gatcgccctt cccaacagtt gcgcagcctg aatggcgaat ggcgctttgc ctggtttccg     1740
   gcaccagaag cggtgccgga aagctggctg gagtgcgatc ttcctgaggc cgatactgtc     1800
   gtcgtcccct caaactggca gatgcacggt tacgatgcgc ccatctacac caacgtgacc     1860
   tatcccatta cggtcaatcc gccgtttgtt cccacggaga atccgacggg ttgttactcg     1920
   ctcacattta atgttgatga aagctggcta caggaaggcc agacgcgaat tatttttgat     1980
   ggcgtcggga tctgatccgg atttactaac tggaagaggc actaaatgaa cacgattaac     2040
   atcgctaaga acgacttctc tgacatcgaa ctggctgcta tcccgttcaa cactctggct     2100
   gaccattacg gtgagcgttt agctcgcgaa cagttggccc ttgagcatga gtcttacgag     2160
   atgggtgaag cacgcttccg caagatgttt gagcgtcaac ttaaagctgg tgaggttgcg     2220
   gataacgctg ccgccaagcc tctcatcact accctactcc ctaagatgat tgcacgcatc     2280
   aacgactggt ttgaggaagt gaaagctaag cgcggcaagc gcccgacagc cttccagttc     2340
   ctgcaagaaa tcaagccgga agccgtagcg tacatcacca ttaagaccac tctggcttgc     2400
   ctaaccagtg ctgacaatac aaccgttcag gctgtagcaa gcgcaatcgg tcgggccatt     2460
   gaggacgagg ctcgcttcgg tcgtatccgt gaccttgaag ctaagcactt caagaaaaac     2520
   gttgaggaac aactcaacaa gcgcgtaggg cacgtctaca agaaagcatt tatgcaagtt     2580
   gtcgaggctg acatgctctc taagggtcta ctcggtggcg aggcgtggtc ttcgtggcat     2640
   aaggaagact ctattcatgt aggagtacgc tgcatcgaga tgctcattga gtcaaccgga     2700
   atggttagct tacaccgcca aaatgctggc gtagtaggtc aagactctga gactatcgaa     2760
   ctcgcacctg aatacgctga ggctatcgca acccgtgcag gtgcgctggc tggcatctct     2820
   ccgatgttcc aaccttgcgt agttcctcct aagccgtgga ctggcattac tggtggtggc     2880
   tattgggcta acggtcgtcg tcctctggcg ctggtgcgta ctcacagtaa gaaagcactg     2940
   atgcgctacg aagacgttta catgcctgag gtgtacaaag cgattaacat tgcgcaaaac     3000
   accgcatgga aaatcaacaa gaaagtccta gcggtcgcca acgtaatcac caagtggaag     3060
   cattgtccgg tcgaggacat ccctgcgatt gagcgtgaag aactcccgat gaaaccggaa     3120
   gacatcgaca tgaatcctga ggctctcacc gcgtggaaac gtgctgccgc tgctgtgtac     3180
   cgcaaggaca aggctcgcaa gtctcgccgt atcagccttg agttcatgct tgagcaagcc     3240
   aataagtttg ctaaccataa ggccatctgg ttcccttaca acatggactg gcgcggtcgt     3300
   gtttacgctg tgtcaatgtt caacccgcaa ggtaacgata tgaccaaagg actgcttacg     3360
   ctggcgaaag gtaaaccaat cggtaaggaa ggttactact ggctgaaaat ccacggtgca 3420
   aactgtgcgg gtgtcgataa ggttccgttc cctgagcgca tcaagttcat tgaggaaaac 3480
   cacgagaaca tcatggcttg cgctaagtct ccactggaga acacttggtg ggctgagcaa 3540
   gattctccgt tctgcttcct tgcgttctgc tttgagtacg ctggggtaca gcaccacggc 3600
   ctgagctata actgctccct tccgctggcg tttgacgggt cttgctctgg catccagcac 3660
   ttctccgcga tgctccgaga tgaggtaggt ggtcgcgcgg ttaacttgct tcctagtgaa 3720
   accgttcagg acatctacgg gattgttgct aagaaagtca acgagattct acaagcagac 3780
   gcaatcaatg ggaccgataa cgaagtagtt accgtgaccg atgagaacac tggtgaaatc 3840
   tctgagaaag tcaagctggg cactaaggca ctggctggtc aatggctggc ttacggtgtt 3900
   actcgcagtg tgactaagcg ttcagtcatg acgctggctt acgggtccaa agagttcggc 3960
   ttccgtcaac aagtgctgga agataccatt cagccagcta ttgattccgg caagggtctg 4020
   atgttcactc agccgaatca ggctgctgga tacatggcta agctgatttg ggaatctgtg 4080
   agcgtgacgg tggtagctgc ggttgaagca atgaactggc ttaagtctgc tgctaagctg 4140
   ctggctgctg aggtcaaaga taagaagact ggagagattc ttcgcaagcg ttgcgctgtg 4200
   cattgggtaa ctcctgatgg tttccctgtg tggcaggaat acaagaagcc tattcagacg 4260
   cgcttgaacc tgatgttcct cggtcagttc cgcttacagc ctaccattaa caccaacaaa 4320
   gatagcgaga ttgatgcaca caaacaggag tctggtatcg ctcctaactt tgtacacagc 4380
   caagacggta gccaccttcg taagactgta gtgtgggcac acgagaagta cggaatcgaa 4440
   tcttttgcac tgattcacga ctccttcggt accattccgg ctgacgctgc gaacctgttc 4500
   aaagcagtgc gcgaaactat ggttgacaca tatgagtctt gtgatgtact ggctgatttc 4560
   tacgaccagt tcgctgacca gttgcacgag tctcaattgg acaaaatgcc agcacttccg 4620
   gctaaaggta acttgaacct ccgtgacatc ttagagtcgg acttcgcgtt cgcgtaacgc 4680
   caaatcaata cgactccgga tccccttcga aggaaagacc tgatgctttt cgtgcgcgca 4740
   taaaatacct tgatactgtg ccggatgaaa gcggttcgcg acgagtagat gcaattatgg 4800
   tttctccgcc aagaatctct ttgcatttat caagtgtttc cttcattgat attccgagag 4860
   catcaatatg caatgctgtt gggatggcaa tttttacgcc tgttttgctt tgctcgacat 4920
   aaagatatcc atctacgata tcagaccact tcatttcgca taaatcacca actcgttgcc 4980
   cggtaacaac agccagttcc attgcaagtc tgagccaaca tggtgatgat tctgctgctt 5040
   gataaatttt caggtattcg tcagccgtaa gtcttgatct ccttacctct gattttgctg 5100
   cgcgagtggc agcgacatgg tttgttgtta tatggccttc agctattgcc tctcggaatg 5160
   catcgctcag tgttgatctg attaacttgg ctgacgccgc cttgccctcg tctatgtatc 5220
   cattgagcat tgccgcaatt tcttttgtgg tgatgtcttc aagtggagca tcaggcagac 5280
   ccctccttat tgctttaatt ttgctcatgt aatttatgag tgtcttctgc ttgattcctc 5340
   tgctggccag gattttttcg tagcgatcaa gccatgaatg taacgtaacg gaattatcac 5400
   tgttgattct cgctgtcaga ggcttgtgtt tgtgtcctga aaataactca atgttggcct 5460
   gtatagcttc agtgattgcg attcgcctgt ctctgcctaa tccaaactct ttacccgtcc 5520
   ttgggtccct gtagcagtaa tatccattgt ttcttatata aaggttaggg ggtaaatccc 5580
   ggcgctcatg acttcgcctt cttcacactg gagggcaaag aagatttcca ataatcagaa 5640
   caagtcggct cctgtttagt tacgagcgac attgctccgt gtattcactc gttggaatga 5700
   atacacagtg cagtgtttat tctgttattt atgccaaaaa taaaggccac tatcaggcag 5760
   ctttgttgtt ctgtttacca agttctctgg caatcattgc cgtcgttcgt attgcccatt 5820
   tatcgacata tttcccatct tccattacag gaaacatttc ttcaggctta accatgcatt 5880
   ccgattgcag cttgcatcca ttgcatcgct tgaattgtcc acaccattga tttttatcaa 5940
   tagtcgtagt catacggata gtcctggtat tgttccatca catcctgagg atgctcttcg 6000
   aactcttcaa attcttcttc catatatcac ctcaaataag tggtttgctg cctaatttaa 6060
   ttttctggcg accaacacaa gtcacaccca tttcactgcg tggcttgctg tagtaaatac 6120
   ggttctgttt acgctcgact tcttctgcct tcttgcagcg aaggcttccg agtgatgctg 6180
   ctttatctgc tctgacgcaa ccagagagct ttagcgcaat ttttcgcgcc agtgcttcat 6240
   tactgcgtcg ctcggcaata agttctgctc tgcgagcttt gtagcggctt tttgccgtac 6300
   ctttggattc tttccagaca atggttacca tgatggtctc ctttaagtgg ctttggcgca 6360
   tgacgcgtcg aggtgcttat cttctcgatc gctgtcttgt agctgcaatt cgcgccatcc 6420
   ccaaaaccac tcaagttctg gtctcaacgg ttaggttgag agtccgtcga tgttaaagag 6480
   cctgccaatc tgttccgttt ggcttccagc gtcctgctga tggcttaaat ttaagacttc 6540
   ttaatttatt ggtcaagtgc atttttgaag aaaacttaat tttatgggcg tgaatttagt 6600
   ttgtctttga tttttaacgg gaaataaaaa aggggcgaaa gccccttaag gaaggtttgc 6660
   tagcttggca tcaacgacaa cgccaatgat tttacagttc ccattgattt caatcattgg 6720
   gtattgtgga ttgagtggtt tcaggaattt tctaccggca tcaataacta actttttgaa 6780
   tgtcgcctcg ttttctcctt caagtttggc gactaccagc tttccattac gtggttcgac 6840
   ttctgggtcg acgagaataa tcatcccctc aggaatactc agtcctgccg gggcagtcat 6900
   tgaatcgcct ttaacgtcga gccaaaaaga gtcttcagaa caatctaccg ttgtgtcgta 6960
   ccagttatct attgcacgcc tatgatatgg ctctacagct tccatccaac atcctgcgct 7020
   tacccaacta attagaggat acgaacctct tggatcatgc ctgctgtgat aggcaatgtt 7080
   tgaaagacta tcctctcctt tcaacaggta atcaggggag cactgcaaag ccttggctaa 7140
   ggccaatagg ttttcgccat tgggctcagt ttcagatcgc tcccattggg aaatagcaac 7200
   attagacacg ccaaccatct tgccaagggc agcctgccta atcttgagtt cttttctgcg 7260
   agcgcgaata cgctcaccca tcagttgtgt attcatagtt aagacatctt aaataaactt 7320
   gacttaagat tcctttggtg gataatttaa gtgttcttta atttcggagc gagtctatgt 7380
   acaaaaaaga tgttattgac cacttcggaa cccagcgtgc tgttgctaaa gcactaggca 7440
   ttagcgatgc agcagtctct cagtggaaag aagttatccc agagaaagac gcctatcgat 7500
   tggaaatcgt tacagctggc gccctgaagt atcaagaaag tgcttaccgc caagcggcat 7560
   aagcaaattg ctctttaaca gttctggcct ttcacctcta accgggtgag caaacatcag 7620
   cggcaaatcc attgggtgtg ccgctataac tcaatatcaa tataggtaaa ttaacaaatg 7680
   gcacaagcaa gctacagcaa gccaacacag cgagaaattg atcgcgctga aactgattta 7740
   ctcatcaacc tgtcaacgct tacccagcgc ggtctggcaa agatgattgg ctgtcatgaa 7800
   tcgaagataa gcagaacgga ctggagattt attgcttcgg tcttgtgtgc tttcggaatg 7860
   gcatcagaca tcagtccgat tagcagggct tttaagtatg cgcttgatgg actcacaaag 7920
   aaaaaacgcc cggtgtgcaa gaccgagcgt tctgaacaaa tccagatgga gttctgaggt 7980
   cattactgga tctatcaaca ggagtcatta tgacaaatac agcaaaaata ctcaacttcg 8040
   gcagaggtaa ctttgccgga caggagcgta atgtggcaga tctcgatgat ggttacgcca 8100
   gactatcaaa tatgctgctt gaggcttatt cgggcgcaga tctgaccaag cgacagttta 8160
   aagtgctgct tgccattctg cgtaaaacct atgggtggaa taaaccaatg gacagaatca 8220
   ccgattctca acttagcgag attacaaagt tacctgtcaa acggtgcaat gaagccaagt 8280
   tagaactcgt cagaatgaat attatcaagc agcaaggcgg catgtttgga ccaaataaaa 8340
   acatctcaga atggtgtatc cctcaaaacg agggaaaatc ccctaaaacg agggataaaa 8400
   catccctcaa attgggggat tgctatccct caaaacaggg ggacacaaaa gacactatta 8460
   caaaagaaaa aagaaaagat tattcgtcag agaattctgg cgaatcctct gaccagccag 8520
   aaaacgacct ttctgtggtg aaaccggatg ctgcaattca gagcggcagc aagtggggga 8580
   cagcagaaga cctgaccgcc gcagagtgga tgtttgacat ggtgaagact atcgcaccat 8640
   cagccagaaa accgaatttt gctgggtggg ctaacgatat ccgcctgatg cgtgaacgtg 8700
   acggacgtaa ccaccgcgac atgtgtgtgc tgttccgctg ggcatgccag gacaacttct 8760
   ggtccggtaa cgtgctgagc ccggccaaac tccgcgataa gtggacccaa ctcgaaatca 8820
   accgtaacaa gcaacaggca ggcgtgacag ccagcaaacc aaaactcgac ctgacaaaca 8880
   cagactggat ttacggggtg gatctatgaa aaacatcgcc gcacagatgg ttaactttga 8940
   ccgtgagcag atgcgtcgga tcgccaacaa catgccggaa cagtacgacg aaaagccgca 9000
   ggtacagcag gtagcgcaga tcatcaacgg tgtgttcagc cagttactgg caactttccc 9060
   ggcgagcctg gctaaccgtg accagaacga agtgaacgaa atccgtcgcc agtgggttct 9120
   ggcttttcgg gaaaacggga tcaccacgat ggaacaggtt aacgcaggaa tgcgcgtagc 9180
   ccgtcggcag aatcgaccat ttctgccatc acccgggcag tttgttgcat ggtgccggga 9240
   agaagcatcc gttaccgccg gactgccaaa cgtcagcgag ctggttgata tggtttacga 9300
   gtattgccgg aagcgaggcc tgtatccgga tgcggagtct tatccgtgga aatcaaacgc 9360
   gcactactgg ctggttacca acctgtatca gaacatgcgg gccaatgcgc ttactgatgc 9420
   ggaattacgc cgtaaggccg cagatgagct tgtccatatg actgcgagaa ttaaccgtgg 9480
   tgaggcgatc cctgaaccag taaaacaact tcctgtcatg ggcggtagac ctctaaatcg 9540
   tgcacaggct ctggcgaaga tcgcagaaat caaagctaag ttcggactga aaggagcaag 9600
   tgtatgacgg gcaaagaggc aattattcat tacctgggga cgcataatag cttctgtgcg 9660
   ccggacgttg ccgcgctaac aggcgcaaca gtaaccagca taaatcaggc cgcggctaaa 9720
   atggcacggg caggtcttct ggttatcgaa ggtaaggtct ggcgaacggt gtattaccgg 9780
   tttgctacca gggaagaacg ggaaggaaag atgagcacga acctgatgaa caaactggat 9840
   acgattggat tcgacaacaa aaaagacctg cttatctcgg tgggcgattt ggttgatcgt 9900
   ggtgcagaga acgttgaatg cctggaatta atcacattcc cctggttcag agctgtacgt 9960
   ggaaaccatg agcaaatgat gattgatggc ttatcagagc gtggaaacgt taatcactgg 10020
   ctgcttaatg gcggtggctg gttctttaat ctcgattacg acaaagaaat tctggctaaa 10080
   gctcttgccc ataaagcaga tgaacttccg ttaatcatcg aactggtgag caaagataaa 10140
   aaatatgtta tctgccacgc cgattatccc tttgacgaat acgagtttgg aaagccagtt 10200
   gatcatcagc aggtaatctg gaaccgcgaa cgaatcagca actcacaaaa cgggatcgtg 10260
   aaagaaatca aaggcgcgga cacgttcatc tttggtcata cgccagcagt gaaaccactc 10320
   aagtttgcca accaaatgta tatcgatacc ggcgcagtgt tctgcggaaa cctaacattg 10380
   attcaggtac agggagaagg cgcatgagac tcgaaagcgt agctaaattt cattcgccaa 10440
   aaagcccgat gatgagcgac tcaccacggg ccacggcttc tgactctctt tccggtactg 10500
   atgtgatggc tgctatgggg atggcgcaat cacaagccgg attcggtatg gctgcattct 10560
   gcggtaagca cgaactcagc cagaacgaca aacaaaaggc tatcaactat ctgatgcaat 10620
   ttgcacacaa ggtatcgggg aaataccgtg gtgtggcata tcttgaagga aatactaagg 10680
   caaaggtact gcaagtgctc gcaacattcg cttatgcgga ttattgccgt agtgccgcga 10740
   cgccgggggc aagatgcaga gattgccatg gtacaggccg tgcggttgat attgccaaaa 10800
   cagagctgtg ggggagagtt gtcgagaaag agtgcggaag atgcaaaggc gtcggctatt 10860
   caaggatgcc agcaagcgca gcatatcgcg ctgtgacgat gctaatccca aaccttaccc 10920
   aacccacctg gtcacgcact gttaagccgc tgtatgacgc tctggtggtg caatgccaca 10980
   aagaagagtc aatcgcagac aacattttga atgcggtcac acgttagcag catgattgcc 11040
   acggatggca acatattaac ggcatgatat tgacttattg aataaaattg ggtaaatttg 11100
   actcaacgat gggttaattc gctcgttgtg gtagtgagat gaaaagaggc ggcgcttact 11160
   accgattccg cctagttggt cacttcgacg tatcgtctgg aactccaacc atcgcaggca 11220
   gagaggtctg caaaatgcaa tcccgaaaca gttcgcaggt aatagttaga gcctgcataa 11280
   cggtttcggg attttttata tctgcacaac aggtaagagc attgagtcga taatcgtgaa 11340
   gagtcggcga gcctggttag ccagtgctct ttccgttgtg ctgaattaag cgaataccgg 11400
   aagcagaacc ggatcaccaa atgcgtacag gcgtcatcgc cgcccagcaa cagcacaacc 11460
   caaactgagc cgtagccact gtctgtcctg aattccatgc ttgaacccgc ctatgcgcgg 11520
   gttttctttt gtgcgcttgc aggccagctt gggatcagca gcctgacgga tgcggtgtcc 11580
   ggcgacagcc tgactgccca ggaggcactc gcgacgctgg cattatccgg tgatgatgac 11640
   ggaccacgac aggcccgcag ttatcaggtc atgaacggca tcgccgtgct gccggtgtcc 11700
   ggcacgctgg tcagccggac gcgggcgctg cagccgtact cggggatgac cggttacaac 11760
   ggcattatcg cccgtctgca acaggctgcc agcgatccga tggtggacgg cattctgctc 11820
   gatatggaca cgcccggcgg gatggtggcg ggggcatttg actgcgctga catcatcgcc 11880
   cgtgtgcgtg acataaaacc ggtatgggcg cttgccaacg acatgaactg cagtgcaggt 11940
   cagttgcttg ccagtgccgc ctcccggcgt ctggtcacgc agaccgcccg gacaggctcc 12000
   atcggcgtca tgatggctca cagtaattac ggtgctgcgc tggagaaaca gggtgtggaa 12060
   atcacgctga tttacagcgg cagccataag gtggatggca acccctacag ccatcttccg 12120
   gatgacgtcc gggagacact gcagtcccgg atggacgcaa cccgccagat gtttgcgcag 12180
   aaggtgtcgg catataccgg cctgtccgtg caggttgtgc tggataccga ggctgcagtg 12240
   tacagcggtc aggaggccat tgatgccgga ctggctgatg aacttgttaa cagcaccgat 12300
   gcgatcaccg tcatgcgtga tgcactggat gcacgtaaat cccgtctctc aggagggcga 12360
   atgaccaaag agactcaatc aacaactgtt tcagccactg cttcgcaggc tgacgttact 12420
   gacgtggtgc cagcgacgga gggcgagaac gccagcgcgg cgcagccgga cgtgaacgcg 12480
   cagatcaccg cagcggttgc ggcagaaaac agccgcatta tggggatact caactgtgag 12540
   gaggctcacg gacgcgaaga acaggcacgc gtgctggcag aaacccccgg tatgaccgtg 12600
   aaaacggccc gccgcattct ggccgcagca ccacagagtg cacaggcgcg cagtgacact 12660
   gcgctggatc gtctgatgca gggggcaccg gcaccgctgg ctgcaggtaa cccggcatct 12720
   gatgccgtta acgatttgct gaacacacca gtgtaaggga tgtttatgac gagcaaagaa 12780
   acctttaccc attaccagcc gcagggcaac agtgacccgg ctcataccgc aaccgcgccc 12840
   ggcggattga gtgcgaaagc gcctgcaatg accccgctga tgctggacac ctccagccgt 12900
   aagctggttg cgtgggatgg caccaccgac ggtgctgccg ttggcattct tgcggttgct 12960
   gctgaccaga ccagcaccac gctgacgttc tacaagtccg gcacgttccg ttatgaggat 13020
   gtgctctggc cggaggctgc cagcgacgag acgaaaaaac ggaccgcgtt tgccggaacg 13080
   gcaatcagca tcgtttaact ttacccttca tcactaaagg ccgcctgtgc ggcttttttt 13140
   acgggatttt tttatgtcga tgtacacaac cgcccaactg ctggcggcaa atgagcagaa 13200
   atttaagttt gatccgctgt ttctgcgtct ctttttccgt gagagctatc ccttcaccac 13260
   ggagaaagtc tatctctcac aaattccggg actggtaaac atggcgctgt acgtttcgcc 13320
   gattgtttcc ggtgaggtta tccgttcccg tggcggctcc acctctgaat ttacgccggg 13380
   atatgtcaag ccgaagcatg aagtgaatcc gcagatgacc ctgcgtcgcc tgccggatga 13440
   agatccgcag aatctggcgg acccggctta ccgccgccgt cgcatcatca tgcagaacat 13500
   gcgtgacgaa gagctggcca ttgctcaggt cgaagagatg caggcagttt ctgccgtgct 13560
   taagggcaaa tacaccatga ccggtgaagc cttcgatccg gttgaggtgg atatgggccg 13620
   cagtgaggag aataacatca cgcagtccgg cggcacggag tggagcaagc gtgacaagtc 13680
   cacgtatgac ccgaccgacg atatcgaagc ctacgcgctg aacgccagcg gtgtggtgaa 13740
   tatcatcgtg ttcgatccga aaggctgggc gctgttccgt tccttcaaag ccgtcaagga 13800
   gaagctggat acccgtcgtg gctctaattc cgagctggag acagcggtga aagacctggg 13860
   caaagcggtg tcctataagg ggatgtatgg cgatgtggcc atcgtcgtgt attccggaca 13920
   gtacgtggaa aacggcgtca aaaagaactt cctgccggac aacacgatgg tgctggggaa 13980
   cactcaggca cgcggtctgc gcacctatgg ctgcattcag gatgcggacg cacagcgcga 14040
   aggcattaac gcctctgccc gttacccgaa aaactgggtg accaccggcg atccggcgcg 14100
   tgagttcacc atgattcagt cagcaccgct gatgctgctg gctgaccctg atgagttcgt 14160
   gtccgtacaa ctggcgtaat catggccctt cggggccatt gtttctctgt ggaggagtcc 14220
   atgacgaaag atgaactgat tgcccgtctc cgctcgctgg gtgaacaact gaaccgtgat 14280
   gtcagcctga cggggacgaa agaagaactg gcgctccgtg tggcagagct gaaagaggag 14340
   cttgatgaca cggatgaaac tgccggtcag gacacccctc tcagccggga aaatgtgctg 14400
   accggacatg aaaatgaggt gggatcagcg cagccggata ccgtgattct ggatacgtct 14460
   gaactggtca cggtcgtggc actggtgaag ctgcatactg atgcacttca cgccacgcgg 14520
   gatgaacctg tggcatttgt gctgccggga acggcgtttc gtgtctctgc cggtgtggca 14580
   gccgaaatga cagagcgcgg cctggccaga atgcaataac gggaggcgct gtggctgatt 14640
   tcgataacct gttcgatgct gccattgccc gcgccgatga aacgatacgc gggtacatgg 14700
   gaacgtcagc caccattaca tccggtgagc agtcaggtgc ggtgatacgt ggtgtttttg 14760
   atgaccctga aaatatcagc tatgccggac agggcgtgcg cgttgaaggc tccagcccgt 14820
   ccctgtttgt ccggactgat gaggtgcggc agctgcggcg tggagacacg ctgaccatcg 14880
   gtgaggaaaa tttctgggta gatcgggttt cgccggatga tggcggaagt tgtcatctct 14940
   ggcttggacg gggcgtaccg cctgccgtta accgtcgccg ctgaaagggg gatgtatggc 15000
   cataaaaggt cttgagcagg ccgttgaaaa cctcagccgt atcagcaaaa cggcggtgcc 15060
   tggtgccgcc gcaatggcca ttaaccgcgt tgcttcatcc gcgatatcgc agtcggcgtc 15120
   acaggttgcc cgtgagacaa aggtacgccg gaaactggta aaggaaaggg ccaggctgaa 15180
   aagggccacg gtcaaaaatc cgcaggccag aatcaaagtt aaccgggggg atttgcccgt 15240
   aatcaagctg ggtaatgcgc gggttgtcct ttcgcgccgc aggcgtcgta aaaaggggca 15300
   gcgttcatcc ctgaaaggtg gcggcagcgt gcttgtggtg ggtaaccgtc gtattcccgg 15360
   cgcgtttatt cagcaactga aaaatggccg gtggcatgtc atgcagcgtg tggctgggaa 15420
   aaaccgttac cccattgatg tggtgaaaat cccgatggcg gtgccgctga ccacggcgtt 15480
   taaacaaaat attgagcgga tacggcgtga acgtcttccg aaagagctgg gctatgcgct 15540
   gcagcatcaa ctgaggatgg taataaagcg atgaaacata ctgaactccg tgcagccgta 15600
   ctggatgcac tggagaagca tgacaccggg gcgacgtttt ttgatggtcg ccccgctgtt 15660
   tttgatgagg cggattttcc ggcagttgcc gtttatctca ccggcgctga atacacgggc 15720
   gaagagctgg acagcgatac ctggcaggcg gagctgcata tcgaagtttt cctgcctgct 15780
   caggtgccgg attcagagct ggatgcgtgg atggagtccc ggatttatcc ggtgatgagc 15840
   gatatcccgg cactgtcaga tttgatcacc agtatggtgg ccagcggcta tgactaccgg 15900
   cgcgacgatg atgcgggctt gtggagttca gccgatctga cttatgtcat tacctatgaa 15960
   atgtgaggac gctatgcctg taccaaatcc tacaatgccg gtgaaaggtg ccgggaccac 16020
   cctgtgggtt tataagggga gcggtgaccc ttacgcgaat ccgctttcag acgttgactg 16080
   gtcgcgtctg gcaaaagtta aagacctgac gcccggcgaa ctgaccgctg agtcctatga 16140
   cgacagctat ctcgatgatg aagatgcaga ctggactgcg accgggcagg ggcagaaatc 16200
   tgccggagat accagcttca cgctggcgtg gatgcccgga gagcaggggc agcaggcgct 16260
   gctggcgtgg tttaatgaag gcgatacccg tgcctataaa atccgcttcc cgaacggcac 16320
   ggtcgatgtg ttccgtggct gggtcagcag tatcggtaag gcggtgacgg cgaaggaagt 16380
   gatcacccgc acggtgaaag tcaccaatgt gggacgtccg tcgatggcag aagatcgcag 16440
   cacggtaaca gcggcaaccg gcatgaccgt gacgcctgcc agcacctcgg tggtgaaagg 16500
   gcagagcacc acgctgaccg tggccttcca gccggagggc gtaaccgaca agagctttcg 16560
   tgcggtgtct gcggataaaa caaaagccac cgtgtcggtc agtggtatga ccatcaccgt 16620
   gaacggcgtt gctgcaggca aggtcaacat tccggttgta tccggtaatg gtgagtttgc 16680
   tgcggttgca gaaattaccg tcaccgccag ttaatccgga gagtcagcga tgttcctgaa 16740
   aaccgaatca tttgaacata acggtgtgac cgtcacgctt tctgaactgt cagccctgca 16800
   gcgcattgag catctcgccc tgatgaaacg gcaggcagaa caggcggagt cagacagcaa 16860
   ccggaagttt actgtggaag acgccatcag aaccggcgcg tttctggtgg cgatgtccct 16920
   gtggcataac catccgcaga agacgcagat gccgtccatg aatgaagccg ttaaacagat 16980
   tgagcaggaa gtgcttacca cctggcccac ggaggcaatt tctcatgctg aaaacgtggt 17040
   gtaccggctg tctggtatgt atgagtttgt ggtgaataat gcccctgaac agacagagga 17100
   cgccgggccc gcagagcctg tttctgcggg aaagtgttcg acggtgagct gagttttgcc 17160
   ctgaaactgg cgcgtgagat ggggcgaccc gactggcgtg ccatgcttgc cgggatgtca 17220
   tccacggagt atgccgactg gcaccgcttt tacagtaccc attattttca tgatgttctg 17280
   ctggatatgc acttttccgg gctgacgtac accgtgctca gcctgttttt cagcgatccg 17340
   gatatgcatc cgctggattt cagtctgctg aaccggcgcg aggctgacga agagcctgaa 17400
   gatgatgtgc tgatgcagaa agcggcaggg cttgccggag gtgtccgctt tggcccggac 17460
   gggaatgaag ttatccccgc ttccccggat gtggcggaca tgacggagga tgacgtaatg 17520
   ctgatgacag tatcagaagg gatcgcagga ggagtccggt atggctgaac cggtaggcga 17580
   tctggtcgtt gatttgagtc tggatgcggc cagatttgac gagcagatgg ccagagtcag 17640
   gcgtcatttt tctggtacgg aaagtgatgc gaaaaaaaca gcggcagtcg ttgaacagtc 17700
   gctgagccga caggcgctgg ctgcacagaa agcggggatt tccgtcgggc agtataaagc 17760
   cgccatgcgt atgctgcctg cacagttcac cgacgtggcc acgcagcttg caggcgggca 17820
   aagtccgtgg ctgatcctgc tgcaacaggg ggggcaggtg aaggactcct tcggcgggat 17880
   gatccccatg ttcagggggc ttgccggtgc gatcaccctg ccgatggtgg gggccacctc 17940
   gctggcggtg gcgaccggtg cgctggcgta tgcctggtat cagggcaact caaccctgtc 18000
   cgatttcaac aaaacgctgg tcctttccgg caatcaggcg ggactgacgg cagatcgtat 18060
   gctggtcctg tccagagccg ggcaggcggc agggctgacg tttaaccaga ccagcgagtc 18120
   actcagcgca ctggttaagg cgggggtaag cggtgaggct cagattgcgt ccatcagcca 18180
   gagtgtggcg cgtttctcct ctgcatccgg cgtggaggtg gacaaggtcg ctgaagcctt 18240
   cgggaagctg accacagacc cgacgtcggg gctgacggcg atggctcgcc agttccataa 18300
   cgtgtcggcg gagcagattg cgtatgttgc tcagttgcag cgttccggcg atgaagccgg 18360
   ggcattgcag gcggcgaacg aggccgcaac gaaagggttt gatgaccaga cccgccgcct 18420
   gaaagagaac atgggcacgc tggagacctg ggcagacagg actgcgcggg cattcaaatc 18480
   catgtgggat gcggtgctgg atattggtcg tcctgatacc gcgcaggaga tgctgattaa 18540
   ggcagaggct gcgtataaga aagcagacga catctggaat ctgcgcaagg atgattattt 18600
   tgttaacgat gaagcgcggg cgcgttactg ggatgatcgt gaaaaggccc gtcttgcgct 18660
   tgaagccgcc cgaaagaagg ctgagcagca gactcaacag gacaaaaatg cgcagcagca 18720
   gagcgatacc gaagcgtcac ggctgaaata taccgaagag gcgcagaagg cttacgaacg 18780
   gctgcagacg ccgctggaga aatataccgc ccgtcaggaa gaactgaaca aggcactgaa 18840
   agacgggaaa atcctgcagg cggattacaa cacgctgatg gcggcggcga aaaaggatta 18900
   tgaagcgacg ctgaaaaagc cgaaacagtc cagcgtgaag gtgtctgcgg gcgatcgtca 18960
   ggaagacagt gctcatgctg ccctgctgac gcttcaggca gaactccgga cgctggagaa 19020
   gcatgccgga gcaaatgaga aaatcagcca gcagcgccgg gatttgtgga aggcggagag 19080
   tcagttcgcg gtactggagg aggcggcgca acgtcgccag ctgtctgcac aggagaaatc 19140
   cctgctggcg cataaagatg agacgctgga gtacaaacgc cagctggctg cacttggcga 19200
   caaggttacg tatcaggagc gcctgaacgc gctggcgcag caggcggata aattcgcaca 19260
   gcagcaacgg gcaaaacggg ccgccattga tgcgaaaagc cgggggctga ctgaccggca 19320
   ggcagaacgg gaagccacgg aacagcgcct gaaggaacag tatggcgata atccgctggc 19380
   gctgaataac gtcatgtcag agcagaaaaa gacctgggcg gctgaagacc agcttcgcgg 19440
   gaactggatg gcaggcctga agtccggctg gagtgagtgg gaagagagcg ccacggacag 19500
   tatgtcgcag gtaaaaagtg cagccacgca gacctttgat ggtattgcac agaatatggc 19560
   ggcgatgctg accggcagtg agcagaactg gcgcagcttc acccgttccg tgctgtccat 19620
   gatgacagaa attctgctta agcaggcaat ggtggggatt gtcgggagta tcggcagcgc 19680
   cattggcggg gctgttggtg gcggcgcatc cgcgtcaggc ggtacagcca ttcaggccgc 19740
   tgcggcgaaa ttccattttg caaccggagg atttacggga accggcggca aatatgagcc 19800
   agcggggatt gttcaccgtg gtgagtttgt cttcacgaag gaggcaacca gccggattgg 19860
   cgtggggaat ctttaccggc tgatgcgcgg ctatgccacc ggcggttatg tcggtacacc 19920
   gggcagcatg gcagacagcc ggtcgcaggc gtccgggacg tttgagcaga ataaccatgt 19980
   ggtgattaac aacgacggca cgaacgggca gataggtccg gctgctctga aggcggtgta 20040
   tgacatggcc cgcaagggtg cccgtgatga aattcagaca cagatgcgtg atggtggcct 20100
   gttctccgga ggtggacgat gaagaccttc cgctggaaag tgaaacccgg tatggatgtg 20160
   gcttcggtcc cttctgtaag aaaggtgcgc tttggtgatg gctattctca gcgagcgcct 20220
   gccgggctga atgccaacct gaaaacgtac agcgtgacgc tttctgtccc ccgtgaggag 20280
   gccacggtac tggagtcgtt tctggaagag cacgggggct ggaaatcctt tctgtggacg 20340
   ccgccttatg agtggcggca gataaaggtg acctgcgcaa aatggtcgtc gcgggtcagt 20400
   atgctgcgtg ttgagttcag cgcagagttt gaacaggtgg tgaactgatg caggatatcc 20460
   ggcaggaaac actgaatgaa tgcacccgtg cggagcagtc ggccagcgtg gtgctctggg 20520
   aaatcgacct gacagaggtc ggtggagaac gttatttttt ctgtaatgag cagaacgaaa 20580
   aaggtgagcc ggtcacctgg caggggcgac agtatcagcc gtatcccatt caggggagcg 20640
   gttttgaact gaatggcaaa ggcaccagta cgcgccccac gctgacggtt tctaacctgt 20700
   acggtatggt caccgggatg gcggaagata tgcagagtct ggtcggcgga acggtggtcc 20760
   ggcgtaaggt ttacgcccgt tttctggatg cggtgaactt cgtcaacgga aacagttacg 20820
   ccgatccgga gcaggaggtg atcagccgct ggcgcattga gcagtgcagc gaactgagcg 20880
   cggtgagtgc ctcctttgta ctgtccacgc cgacggaaac ggatggcgct gtttttccgg 20940
   gacgtatcat gctggccaac acctgcacct ggacctatcg cggtgacgag tgcggttata 21000
   gcggtccggc tgtcgcggat gaatatgacc agccaacgtc cgatatcacg aaggataaat 21060
   gcagcaaatg cctgagcggt tgtaagttcc gcaataacgt cggcaacttt ggcggcttcc 21120
   tttccattaa caaactttcg cagtaaatcc catgacacag acagaatcag cgattctggc 21180
   gcacgcccgg cgatgtgcgc cagcggagtc gtgcggcttc gtggtaagca cgccggaggg 21240
   ggaaagatat ttcccctgcg tgaatatctc cggtgagccg gaggcgtatt tccgtatgtc 21300
   gccggaagac tggctgcagg cagaaatgca gggtgagatt gtggcgctgg tccacagcca 21360
   ccccggtggt ctgccctggc tgagtgaggc cgaccggcgg ctgcaggtgc agagtgattt 21420
   gccgtggtgg ctggtctgcc gggggacgat tcataagttc cgctgtgtgc cgcatctcac 21480
   cgggcggcgc tttgagcacg gtgtgacgga ctgttacaca ctgttccggg atgcttatca 21540
   tctggcgggg attgagatgc cggactttca tcgtgaggat gactggtggc gtaacggcca 21600
   gaatctctat ctggataatc tggaggcgac ggggctgtat caggtgccgt tgtcagcggc 21660
   acagccgggc gatgtgctgc tgtgctgttt tggttcatca gtgccgaatc acgccgcaat 21720
   ttactgcggc gacggcgagc tgctgcacca tattcctgaa caactgagca aacgagagag 21780
   gtacaccgac aaatggcagc gacgcacaca ctccctctgg cgtcaccggg catggcgcgc 21840
   atctgccttt acggggattt acaacgattt ggtcgccgca tcgaccttcg tgtgaaaacg 21900
   ggggctgaag ccatccgggc actggccaca cagctcccgg cgtttcgtca gaaactgagc 21960
   gacggctggt atcaggtacg gattgccggg cgggacgtca gcacgtccgg gttaacggcg 22020
   cagttacatg agactctgcc tgatggcgct gtaattcata ttgttcccag agtcgccggg 22080
   gccaagtcag gtggcgtatt ccagattgtc ctgggggctg ccgccattgc cggatcattc 22140
   tttaccgccg gagccaccct tgcagcatgg ggggcagcca ttggggccgg tggtatgacc 22200
   ggcatcctgt tttctctcgg tgccagtatg gtgctcggtg gtgtggcgca gatgctggca 22260
   ccgaaagcca gaactccccg tatacagaca acggataacg gtaagcagaa cacctatttc 22320
   tcctcactgg ataacatggt tgcccagggc aatgttctgc ctgttctgta cggggaaatg 22380
   cgcgtggggt cacgcgtggt ttctcaggag atcagcacgg cagacgaagg ggacggtggt 22440
   caggttgtgg tgattggtcg ctgatgcaaa atgttttatg tgaaaccgcc tgcgggcggt 22500
   tttgtcattt atggagcgtg aggaatgggt aaaggaagca gtaaggggca taccccgcgc 22560
   gaagcgaagg acaacctgaa gtccacgcag ttgctgagtg tgatcgatgc catcagcgaa 22620
   gggccgattg aaggtccggt ggatggctta aaaagcgtgc tgctgaacag tacgccggtg 22680
   ctggacactg aggggaatac caacatatcc ggtgtcacgg tggtgttccg ggctggtgag 22740
   caggagcaga ctccgccgga gggatttgaa tcctccggct ccgagacggt gctgggtacg 22800
   gaagtgaaat atgacacgcc gatcacccgc accattacgt ctgcaaacat cgaccgtctg 22860
   cgctttacct tcggtgtaca ggcactggtg gaaaccacct caaagggtga caggaatccg 22920
   tcggaagtcc gcctgctggt tcagatacaa cgtaacggtg gctgggtgac ggaaaaagac 22980
   atcaccatta agggcaaaac cacctcgcag tatctggcct cggtggtgat gggtaacctg 23040
   ccgccgcgcc cgtttaatat ccggatgcgc aggatgacgc cggacagcac cacagaccag 23100
   ctgcagaaca aaacgctctg gtcgtcatac actgaaatca tcgatgtgaa acagtgctac 23160
   ccgaacacgg cactggtcgg cgtgcaggtg gactcggagc agttcggcag ccagcaggtg 23220
   agccgtaatt atcatctgcg cgggcgtatt ctgcaggtgc cgtcgaacta taacccgcag 23280
   acgcggcaat acagcggtat ctgggacgga acgtttaaac cggcatacag caacaacatg 23340
   gcctggtgtc tgtgggatat gctgacccat ccgcgctacg gcatggggaa acgtcttggt 23400
   gcggcggatg tggataaatg ggcgctgtat gtcatcggcc agtactgcga ccagtcagtg 23460
   ccggacggct ttggcggcac ggagccgcgc atcacctgta atgcgtacct gaccacacag 23520
   cgtaaggcgt gggatgtgct cagcgatttc tgctcggcga tgcgctgtat gccggtatgg 23580
   aacgggcaga cgctgacgtt cgtgcaggac cgaccgtcgg ataagacgtg gacctataac 23640
   cgcagtaatg tggtgatgcc ggatgatggc gcgccgttcc gctacagctt cagcgccctg 23700
   aaggaccgcc ataatgccgt tgaggtgaac tggattgacc cgaacaacgg ctgggagacg 23760
   gcgacagagc ttgttgaaga tacgcaggcc attgcccgtt acggtcgtaa tgttacgaag 23820
   atggatgcct ttggctgtac cagccggggg caggcacacc gcgccgggct gtggctgatt 23880
   aaaacagaac tgctggaaac gcagaccgtg gatttcagcg tcggcgcaga agggcttcgc 23940
   catgtaccgg gcgatgttat tgaaatctgc gatgatgact atgccggtat cagcaccggt 24000
   ggtcgtgtgc tggcggtgaa cagccagacc cggacgctga cgctcgaccg tgaaatcacg 24060
   ctgccatcct ccggtaccgc gctgataagc ctggttgacg gaagtggcaa tccggtcagc 24120
   gtggaggttc agtccgtcac cgacggcgtg aaggtaaaag tgagccgtgt tcctgacggt 24180
   gttgctgaat acagcgtatg ggagctgaag ctgccgacgc tgcgccagcg actgttccgc 24240
   tgcgtgagta tccgtgagaa cgacgacggc acgtatgcca tcaccgccgt gcagcatgtg 24300
   ccggaaaaag aggccatcgt ggataacggg gcgcactttg acggcgaaca gagtggcacg 24360
   gtgaatggtg tcacgccgcc agcggtgcag cacctgaccg cagaagtcac tgcagacagc 24420
   ggggaatatc aggtgctggc gcgatgggac acaccgaagg tggtgaaggg cgtgagtttc 24480
   ctgctccgtc tgaccgtaac agcggacgac ggcagtgagc ggctggtcag cacggcccgg 24540
   acgacggaaa ccacataccg cttcacgcaa ctggcgctgg ggaactacag gctgacagtc 24600
   cgggcggtaa atgcgtgggg gcagcagggc gatccggcgt cggtatcgtt ccggattgcc 24660
   gcaccggcag caccgtcgag gattgagctg acgccgggct attttcagat aaccgccacg 24720
   ccgcatcttg ccgtttatga cccgacggta cagtttgagt tctggttctc ggaaaagcag 24780
   attgcggata tcagacaggt tgaaaccagc acgcgttatc ttggtacggc gctgtactgg 24840
   atagccgcca gtatcaatat caaaccgggc catgattatt acttttatat ccgcagtgtg 24900
   aacaccgttg gcaaatcggc attcgtggag gccgtcggtc gggcgagcga tgatgcggaa 24960
   ggttacctgg attttttcaa aggcaagata accgaatccc atctcggcaa ggagctgctg 25020
   gaaaaagtcg agctgacgga ggataacgcc agcagactgg aggagttttc gaaagagtgg 25080
   aaggatgcca gtgataagtg gaatgccatg tgggctgtca aaattgagca gaccaaagac 25140
   ggcaaacatt atgtcgcggg tattggcctc agcatggagg acacggagga aggcaaactg 25200
   agccagtttc tggttgccgc caatcgtatc gcatttattg acccggcaaa cgggaatgaa 25260
   acgccgatgt ttgtggcgca gggcaaccag atattcatga acgacgtgtt cctgaagcgc 25320
   ctgacggccc ccaccattac cagcggcggc aatcctccgg ccttttccct gacaccggac 25380
   ggaaagctga ccgctaaaaa tgcggatatc agtggcagtg tgaatgcgaa ctccgggacg 25440
   ctcagtaatg tgacgatagc tgaaaactgt acgataaacg gtacgctgag ggcggaaaaa 25500
   atcgtcgggg acattgtaaa ggcggcgagc gcggcttttc cgcgccagcg tgaaagcagt 25560
   gtggactggc cgtcaggtac ccgtactgtc accgtgaccg atgaccatcc ttttgatcgc 25620
   cagatagtgg tgcttccgct gacgtttcgc ggaagtaagc gtactgtcag cggcaggaca 25680
   acgtattcga tgtgttatct gaaagtactg atgaacggtg cggtgattta tgatggcgcg 25740
   gcgaacgagg cggtacaggt gttctcccgt attgttgaca tgccagcggg tcggggaaac 25800
   gtgatcctga cgttcacgct tacgtccaca cggcattcgg cagatattcc gccgtatacg 25860
   tttgccagcg atgtgcaggt tatggtgatt aagaaacagg cgctgggcat cagcgtggtc 25920
   tgagtgtgtt acagaggttc gtccgggaac gggcgtttta ttataaaaca gtgagaggtg 25980
   aacgatgcgt aatgtgtgta ttgccgttgc tgtctttgcc gcacttgcgg tgacagtcac 26040
   tccggcccgt gcggaaggtg gacatggtac gtttacggtg ggctattttc aagtgaaacc 26100
   gggtacattg ccgtcgttgt cgggcgggga taccggtgtg agtcatctga aagggattaa 26160
   cgtgaagtac cgttatgagc tgacggacag tgtgggggtg atggcttccc tggggttcgc 26220
   cgcgtcgaaa aagagcagca cagtgatgac cggggaggat acgtttcact atgagagcct 26280
   gcgtggacgt tatgtgagcg tgatggccgg accggtttta caaatcagta agcaggtcag 26340
   tgcgtacgcc atggccggag tggctcacag tcggtggtcc ggcagtacaa tggattaccg 26400
   taagacggaa atcactcccg ggtatatgaa agagacgacc actgccaggg acgaaagtgc 26460
   aatgcggcat acctcagtgg cgtggagtgc aggtatacag attaatccgg cagcgtccgt 26520
   cgttgttgat attgcttatg aaggctccgg cagtggcgac tggcgtactg acggattcat 26580
   cgttggggtc ggttataaat tctgattagc caggtaacac agtgttatga cagcccgccg 26640
   gaaccggtgg gcttttttgt ggggtgaata tggcagtaaa gatttcagga gtcctgaaag 26700
   acggcacagg aaaaccggta cagaactgca ccattcagct gaaagccaga cgtaacagca 26760
   ccacggtggt ggtgaacacg gtgggctcag agaatccgga tgaagccggg cgttacagca 26820
   tggatgtgga gtacggtcag tacagtgtca tcctgcaggt tgacggtttt ccaccatcgc 26880
   acgccgggac catcaccgtg tatgaagatt cacaaccggg gacgctgaat gattttctct 26940
   gtgccatgac ggaggatgat gcccggccgg aggtgctgcg tcgtcttgaa ctgatggtgg 27000
   aagaggtggc gcgtaacgcg tccgtggtgg cacagagtac ggcagacgcg aagaaatcag 27060
   ccggcgatgc cagtgcatca gctgctcagg tcgcggccct tgtgactgat gcaactgact 27120
   cagcacgcgc cgccagcacg tccgccggac aggctgcatc gtcagctcag gaagcgtcct 27180
   ccggcgcaga agcggcatca gcaaaggcca ctgaagcgga aaaaagtgcc gcagccgcag 27240
   agtcctcaaa aaacgcggcg gccaccagtg ccggtgcggc gaaaacgtca gaaacgaatg 27300
   ctgcagcgtc acaacaatca gccgccacgt ctgcctccac cgcggccacg aaagcgtcag 27360
   aggccgccac ttcagcacga gatgcggtgg cctcaaaaga ggcagcaaaa tcatcagaaa 27420
   cgaacgcatc atcaagtgcc ggtcgtgcag cttcctcggc aacggcggca gaaaattctg 27480
   ccagggcggc aaaaacgtcc gagacgaatg ccaggtcatc tgaaacagca gcggaacgga 27540
   gcgcctctgc cgcggcagac gcaaaaacag cggcggcggg gagtgcgtca acggcatcca 27600
   cgaaggcgac agaggctgcg ggaagtgcgg tatcagcatc gcagagcaaa agtgcggcag 27660
   aagcggcggc aatacgtgca gaaaattcgg caaaacgtgc agaagatata gcttcagctg 27720
   tcgcgcttga ggatgcggac acaacgagaa aggggatagt gcagctcagc agtgcaacca 27780
   acagcacgtc tgaaacgctt gctgcaacgc caaaggcggt taaggtggta atggatgaaa 27840
   cgaacagaaa agcccactgg acagtccggc actgaccgga acgccaacag caccaaccgc 27900
   gctcagggga acaaacaata cccagattgc gaacaccgct tttgtactgg ccgcgattgc 27960
   agatgttatc gacgcgtcac ctgacgcact gaatacgctg aatgaactgg ccgcagcgct 28020
   cgggaatgat ccagattttg ctaccaccat gactaacgcg cttgcgggta aacaaccgaa 28080
   gaatgcgaca ctgacggcgc tggcagggct ttccacggcg aaaaataaat taccgtattt 28140
   tgcggaaaat gatgccgcca gcctgactga actgactcag gttggcaggg atattctggc 28200
   aaaaaattcc gttgcagatg ttcttgaata ccttggggcc ggtgagaatt ctaagcggag 28260
   atcgcctagt gattttaaac tattgctggc agcattcttg agtccaatat aaaagtattg 28320
   tgtacctttt gctgggtcag gttgttcttt aggaggagta aaaggatcaa atgcactaaa 28380
   cgaaactgaa acaagcgatc gaaaatatcc ctttgggatt cttgactcga taagtctatt 28440
   attttcagag aaaaaatatt cattgttttc tgggttggtg attgcaccaa tcattccatt 28500
   caaaattgtt gttttaccac acccattccg cccgataaaa gcatgaatgt tcgtgctggg 28560
   catagaatta accgtcacct caaaaggtat agttaaatca ctgaatccgg gagcactttt 28620
   tctattaaat gaaaagtgga aatctgacaa ttctggcaaa ccatttaaca cacgtgcgaa 28680
   ctgtccatga atttctgaaa gagttacccc tctaagtaat gaggtgttaa ggacgctttc 28740
   attttcaatg tcggctaatc gatttggcca tactactaaa tcctgaatag ctttaagaag 28800
   gttatgttta aaaccatcgc ttaatttgct gagattaaca tagtagtcaa tgctttcacc 28860
   taaggaaaaa aacatttcag ggagttgact gaatttttta tctattaatg aataagtgct 28920
   tacttcttct ttttgaccta caaaaccaat tttaacattt ccgatatcgc atttttcacc 28980
   atgctcatca aagacagtaa gataaaacat tgtaacaaag gaatagtcat tccaaccatc 29040
   tgctcgtagg aatgccttat ttttttctac tgcaggaata tacccgcctc tttcaataac 29100
   actaaactcc aacatatagt aacccttaat tttattaaaa taaccgcaat ttatttggcg 29160
   gcaacacagg atctctcttt taagttactc tctattacat acgttttcca tctaaaaatt 29220
   agtagtattg aacttaacgg ggcatcgtat tgtagttttc catatttagc tttctgcttc 29280
   cttttggata acccactgtt attcatgttg catggtgcac tgtttatacc aacgatatag 29340
   tctattaatg catatatagt atcgccgaac gattagctct tcaggcttct gaagaagcgt 29400
   ttcaagtact aataagccga tagatagcca cggacttcgt agccattttt cataagtgtt 29460
   aacttccgct cctcgctcat aacagacatt cactacagtt atggcggaaa ggtatgcatg 29520
   ctgggtgtgg ggaagtcgtg aaagaaaaga agtcagctgc gtcgtttgac atcactgcta 29580
   tcttcttact ggttatgcag gtcgtagtgg gtggcacaca aagctttgca ctggattgcg 29640
   aggctttgtg cttctctgga gtgcgacagg tttgatgaca aaaaattagc gcaagaagac 29700
   aaaaatcacc ttgcgctaat gctctgttac aggtcactaa taccatctaa gtagttgatt 29760
   catagtgact gcatatgttg tgttttacag tattatgtag tctgtttttt atgcaaaatc 29820
   taatttaata tattgatatt tatatcattt tacgtttctc gttca 29865
<210> 11
   <211> 2244
   <212> DNA
   <213> Escherichia coli
<220>
   <221> source
   <222> 1..2244
   <223> /mol_type="DNA" /organism="Escherichia coli"
<400> 11
   atggcgcgtt ccaaaactgc tcagccaaaa cactcactgc gtaaaatcgc agttgtagta      60
   gccacagcgg ttagcggcat gtctgtttat gcacaggcag cggttgaacc gaaagaagac     120
   actatcaccg ttaccgctgc acctgcgccg caagaaagcg catgggggcc tgctgcaact     180
   attgcggcgc gacagtctgc taccggcact aaaaccgata cgccgattca aaaagtgcca     240
   cagtctattt ctgttgtgac cgccgaagag atggcgctgc atcagccgaa gtcggtaaaa     300
   gaagcgctta gctacacgcc gggtgtctct gttggtacgc gtggcgcatc caacacctat     360
   gaccacctga tcattcgcgg ctttgcggca gaaggccaaa gccagaataa ctatctgaat 420
   ggcctgaagt tgcagggcaa cttctataac gatgcggtca ttgacccgta tatgctggaa 480
   cgcgctgaaa ttatgcgtgg cccggtttcc gtgctttacg gtaaaagcag tcctggcggc 540
   ctgttgaata tggtcagcaa gcgtccgacc accgaaccgc tgaaagaagt tcagtttaaa 600
   gccggtactg acagcctgtt ccagactggt tttgacttta gcgattcgtt ggatgatgac 660
   ggtgtttact cttatcgcct gaccggtctt gcgcgttctg ccaatgccca gcagaaaggg 720
   tcagaagagc agcgttatgc tattgcaccg gcgttcacct ggcgtccgga tgataaaacc 780
   aattttacct tcctttctta cttccagaac gagccggaaa ccggttatta cggctggttg 840
   ccgaaagagg gaaccgttga gccgctgccg aacggtaagc gtctgccgac agactttaat 900
   gaaggggcga agaacaacac ctattctcgt aatgagaaga tggtcggcta cagcttcgat 960
   cacgaattta acgacacctt tactgtgcgt cagaacctgc gctttgctga aaacaaaacc 1020
   tcgcaaaaca gcgtttatgg ttacggcgtc tgctccgatc cggcgaatgc ttacagcaaa 1080
   cagtgtgcgg cattagcgcc agcggataaa ggccattatc tggcacgtaa atacgtcgtt 1140
   gatgatgaga agctgcaaaa cttctccgtt gatacccagt tgcagagcaa gtttgccact 1200
   ggcgatatcg accacaccct gctgaccggt gtcgacttta tgcgtatgcg taatgacatc 1260
   aacgcctggt ttggttacga cgactctgtg ccactgctca atctgtacaa tccggtgaat 1320
   accgatttcg acttcaatgc caaagatccg gcaaactccg gcccttaccg cattctgaat 1380
   aaacagaaac aaacgggcgt ttatgttcag gatcaggcgc agtgggataa agtgctggtc 1440
   accctaggcg gtcgttatga ctgggcagat caagaatctc ttaaccgcgt tgccgggacg 1500
   accgataaac gtgatgacaa acagtttacc tggcgtggtg gtgttaacta cctgtttgat 1560
   aatggtgtaa caccttactt cagctatagc gaatcgtttg aaccttcttc gcaagttggg 1620
   aaggatggta atattttcgc accgtctaaa ggtaagcagt atgaagtcgg cgtgaaatat 1680
   gtaccggaag atcgtccgat tgtagttact ggtgccgtgt ataatctcac taaaaccaac 1740
   aacctgatgg cggaccctga gggttccttc ttctcggttg aaggtggcga gatccgcgca 1800
   cgtggcgtag aaatcgaagc gaaagcggcg ctgtcggcga gtgttaacgt agtcggttct 1860
   tatacttaca ccgatgcgga atacaccacc gatactacct ataaaggcaa tacgcctgca 1920
   caggtgccaa aacacatggc ttcgttgtgg gctgactaca ccttctttga cggtccgctt 1980
   tcaggtctga cgctgggcac cggtggtcgt tatactggct ccagttatgg tgatccggct 2040
   aactccttta aagtgggaag ttatacggtc gtggatgcgt tagtacgtta tgatctggcg 2100
   cgagtcggca tggctggctc caacgtggcg ctgcatgtta acaacctgtt cgatcgtgaa 2160
   tacgtcgcca gctgctttaa cacttatggc tgcttctggg gcgcagaacg tcaggtcgtt 2220
   gcaaccgcaa ccttccgttt ctaa 2244
<210> 12
   <211> 1149
   <212> DNA
   <213> Escherichia coli
<220>
   <221> source
   <222> 1..1149
   <223> /mol_type="DNA" /organism="Escherichia coli"
<400> 12
   atgagtctga aagaaaaaac acaatctctg tttgccaacg catttggcta ccctgccact      60
   cacaccattc aggcgcctgg ccgcgtgaat ttgattggtg aacacaccga ctacaacgac     120
   ggtttcgttc tgccctgcgc gattgattat caaaccgtga tcagttgtgc accacgcgat     180
   gaccgtaaag ttcgcgtgat ggcagccgat tatgaaaatc agctcgacga gttttccctc     240
   gatgcgccca ttgtcgcaca tgaaaactat caatgggcta actacgttcg tggcgtggtg     300
   aaacatctgc aactgcgtaa caacagcttc ggcggcgtgg acatggtgat cagcggcaat     360
   gtgccgcagg gtgccgggtt aagttcttcc gcttcactgg aagtcgcggt cggaaccgta     420
   ttgcagcagc tttatcatct gccgctggac ggcgcacaaa tcgcgcttaa cggtcaggaa     480
   gcagaaaacc agtttgtagg ctgtaactgc gggatcatgg atcagctaat ttccgcgctc     540
   ggcaagaaag atcatgcctt gctgatcgat tgccgctcac tggggaccaa agcagtttcc     600
   atgcccaaag gtgtggctgt cgtcatcatc aacagtaact tcaaacgtac cctggttggc     660
   agcgaataca acacccgtcg tgaacagtgc gaaaccggtg cgcgtttctt ccagcagcca     720
   gccctgcgtg atgtcaccat tgaagagttc aacgctgttg cgcatgaact ggacccgatc     780
   gtggcaaaac gcgtgcgtca tatactgact gaaaacgccc gcaccgttga agctgccagc     840
   gcgctggagc aaggcgacct gaaacgtatg ggcgagttga tggcggagtc tcatgcctct     900
   atgcgcgatg atttcgaaat caccgtgccg caaattgaca ctctggtaga aatcgtcaaa     960
   gctgtgattg gcgacaaagg tggcgtacgc atgaccggcg gcggatttgg cggctgtatc    1020
   gtcgcgctga tcccggaaga gctggtgcct gccgtacagc aagctgtcgc tgaacaatat    1080
   gaagcaaaaa caggtattaa agagactttt tacgtttgta aaccatcaca aggagcagga    1140
   cagtgctga                                                            1149
<210> 13
   <211> 1701
   <212> DNA
   <213> Escherichia coli
<220>
   <221> source
   <222> 1..1701
   <223> /mol_type="DNA" /organism="Escherichia coli"
<400> 13
   atggcgattg caattggcct cgattttggc agtgattctg tgcgagcttt ggcggtggac      60
   tgcgctaccg gtgaagagat cgccaccagc gtagagtggt atccccgttg gcagaaaggg     120
   caattttgtg atgccccgaa taaccagttc cgtcatcatc cgcgtgacta cattgagtca     180
   atggaagcgg cactgaaaac cgtgcttgca gagcttagcg tcgaacagcg cgcagctgtg     240
   gtcgggattg gcgttgacag taccggctcg acgcccgcac cgattgatgc cgacggaaac     300
   gtgctggcgc tgcgcccgga gtttgccgaa aacccgaacg cgatgttcgt attgtggaaa     360
   gaccacactg cggttgaaga agcggaagag attacccgtt tgtgccacgc gccgggcaac     420
   gttgactact cccgctacat tggtggtatt tattccagcg aatggttctg ggcaaaaatc     480
   ctgcatgtga ctcgccagga cagcgccgtg gcgcaatctg ccgcatcgtg gattgagctg     540
   tgcgactggg tgccagctct gctttccggt accacccgcc cgcaggatat tcgtcgcgga     600
   cgttgcagcg ccgggcataa atctctgtgg cacgaaagct ggggcggcct gccgccagcc     660
   agtttctttg atgagctgga cccgatcctc aatcgccatt tgccttcccc gctgttcact     720
   gacacttgga ctgccgatat tccggtgggc accttatgcc cggaatgggc gcagcgtctc     780
   ggcctgcctg aaagcgtggt gatttccggc ggcgcgtttg actgccatat gggcgcagtt     840
   ggcgcaggcg cacagcctaa cgcactggta aaagttatcg gtacttccac ctgcgacatt     900
   ctgattgccg acaaacagag cgttggcgag cgggcagtta aaggtatttg cggtcaggtt     960
   gatggcagcg tggtgcctgg atttatcggt ctggaagcag gccaatcggc gtttggtgat    1020
   atctacgcct ggtttggtcg cgtactcggc tggccgctgg aacagcttgc cgcccagcat    1080
   ccggaactga aaacgcaaat caacgccagc cagaaacaac tgcttccggc gctgaccgaa    1140
   gcatgggcca aaaatccgtc tctggatcac ctgccggtgg tgctcgactg gtttaacggc    1200
   cgccgcacac cgaacgctaa ccaacgcctg aaaggggtga ttaccgatct taacctcgct    1260
   accgacgctc cgctgctgtt cggcggtttg attgctgcca ccgcctttgg cgcacgcgca    1320
   atcatggagt gctttaccga tcaggggatc gccgttaata acgtgatggc actgggcggc    1380
   atcgcgcgga aaaaccaggt cattatgcag gcctgctgcg acgtgctgaa tcgcccgctg    1440
   caaattgttg cctctgacca gtgctgtgcg ctcggtgcgg cgatttttgc tgccgtcgcc    1500
   gcgaaagtgc acgcagacat cccatcagct cagcaaaaaa tggccagtgc ggtagagaaa    1560
   accctgcaac cgtgcagcga gcaggcacaa cgctttgaac agctttatcg ccgctatcag    1620
   caatgggcga tgagcgccga acaacactat cttccaactt ccgccccggc acaggctgcc    1680
   caggccgttg cgactctata a                                               1701
<210> 14
   <211> 1503
   <212> DNA
   <213> Escherichia coli
<220>
   <221> source
   <222> 1..1503
   <223> /mol_type="DNA" /organism="Escherichia coli"
<400> 14
   atgacgattt ttgataatta tgaagtgtgg tttgtcattg gcagccagca tctgtatggc      60
   ccggaaaccc tgcgtcaggt cacccaacat gccgagcacg tcgttaatgc gctgaatacg     120
   gaagcgaaac tgccctgcaa actggtgttg aaaccgctgg gcaccacgcc ggatgaaatc     180
   accgctattt gccgcgacgc gaattacgac gatcgttgcg ctggtctggt ggtgtggctg     240
   cacaccttct ccccggccaa aatgtggatc aacggcctga ccatgctcaa caaaccgttg     300
   ctgcaattcc acacccagtt caacgcggcg ctgccgtggg acagtatcga tatggacttt     360
   atgaacctga accagactgc acatggcggt cgcgagttcg gcttcattgg cgcgcgtatg     420
   cgtcagcaac atgccgtggt taccggtcac tggcaggata aacaagccca tgagcgtatc     480
   ggctcctgga tgcgtcaggc ggtctctaaa caggataccc gtcatctgaa agtctgccga     540
   tttggcgata acatgcgtga agtggcggtc accgatggcg ataaagttgc cgcacagatc     600
   aagttcggtt tctccgtcaa tacctgggcg gttggcgatc tggtgcaggt ggtgaactcc     660
   atcagcgacg gcgatgttaa cgcgctggtc gatgagtacg aaagctgcta caccatgacg     720
   cctgccacac aaatccacgg caaaaaacga cagaacgtgc tggaagcggc gcgtattgag     780
   ctggggatga agcgtttcct ggaacaaggt ggcttccacg cgttcaccac cacctttgaa     840
   gatttgcacg gtctgaaaca gcttcctggt ctggccgtac agcgtctgat gcagcagggt     900
   tacggctttg cgggcgaagg cgactggaaa actgccgccc tgcttcgcat catgaaggtg     960
   atgtcaaccg gtctgcaggg cggcacctcc tttatggagg actacaccta tcacttcgag    1020
   aaaggtaatg acctggtgct cggctcccat atgctggaag tctgcccgtc gatcgccgca    1080
   gaagagaaac cgatcctcga cgttcagcat ctcggtattg gtggtaagga cgatcctgcc    1140
   cgcctgatct tcaataccca aaccggccca gcgattgtcg ccagcttgat tgatctcggc    1200
   gatcgttacc gtctactggt taactgcatc gacacggtga aaacaccgca ctccctgccg    1260
   aaactgccgg tggcgaatgc gctgtggaaa gcgcaaccgg atctgccaac tgcttccgaa    1320
   gcgtggatcc tcgctggtgg cgcgcaccat accgtcttca gccatgcact gaacctcaac    1380
   gatatgcgcc aattcgccga gatgcacgac attgaaatca cggtgattga taacgacaca    1440
   cgcctgccag cgtttaaaga cgcgctgcgc tggaacgaag tgtattacgg gtttcgtcgc    1500
   taa                                                                  1503
<210> 15
   <211> 2355
   <212> DNA
   <213> Escherichia coli
<220>
   <221> source
   <222> 1..2355
   <223> /mol_type="DNA" /organism="Escherichia coli"
<400> 15
   atgaatcctg agcgttctga acgcattgaa atccccgtat tgccgctgcg cgatgtggtg 60
   gtttatccgc acatggtcat ccccttattt gtcgggcggg aaaaatctat ccgttgtctg 120
   gaagcggcga tggaccatga taaaaaaatt atgctggtcg cgcagaaaga agcttcaacg 180
   gatgagccgg gtgtaaacga tcttttcacc gtcgggaccg tggcctctat attgcagatg 240
   ctgaaactgc ctgacggcac cgtcaaagtg ctggtcgagg ggttacagcg cgcgcgtatt 300
   tctgcgctct ctgacaatgg cgaacacttt tctgcgaagg cggagtatct ggagtcgccg 360
   accattgatg agcgggaaca ggaagtgctg gtgcgtactg caatcagcca gttcgaaggc 420
   tacatcaagc tgaacaaaaa aatcccacca gaagtgctga cgtcgctgaa tagcatcgac 480
   gatccggcgc gtctggcgga taccattgct gcacatatgc cgctgaaact ggctgacaaa 540
   cagtctgttc tggagatgtc cgacgttaac gaacgtctgg aatatctgat ggcaatgatg 600
   gaatcggaaa tcgatctgct gcaggttgag aaacgcattc gcaaccgcgt taaaaagcag 660
   atggagaaat cccagcgtga gtactatctg aacgagcaaa tgaaagctat tcagaaagaa 720
   ctcggtgaaa tggacgacgc gccggacgaa aacgaagccc tgaagcgcaa aatcgacgcg 780
   gcgaagatgc cgaaagaggc aaaagagaaa gcggaagcag agttgcagaa gctgaaaatg 840
   atgtctccga tgtcggcaga agcgaccgta gtgcgtggtt atatcgactg gatggtacag 900
   gtgccgtgga atgcgcgtag caaggtcaaa aaagacctgc gtcaggcgca ggaaatcctt 960
   gataccgacc attatggtct ggagcgcgtg aaagatcgaa tccttgagta tcttgcggtt 1020
   caaagccgtg tcaacaaaat caagggaccg atcctctgcc tggtagggcc gccgggggta 1080
   ggtaaaacct ctcttggtca gtccattgcc aaagccaccg ggcgtaaata tgtccgtatg 1140
   gcgctgggcg gcgtgcgtga tgaagcggaa atccgtggtc accgccgtac ttacatcggt 1200
   tctatgccgg gtaaactgat ccagaaaatg gcgaaagtgg gcgtgaaaaa cccgctgttc 1260
   ctgctcgatg agatcgacaa aatgtcttct gacatgcgtg gcgatccggc ctctgcactg 1320
   cttgaagtgc tggatccaga gcagaacgta gcgttcagcg accactacct ggaagtggat 1380
   tacgatctca gcgacgtgat gtttgtcgcg acgtcgaact ccatgaacat tccggcaccg 1440
   ctgctggatc gtatggaagt gattcgcctc tccggttata ccgaagatga aaaactgaac 1500
   atcgccaaac gtcacctgct gccgaagcag attgaacgta atgcactgaa aaaaggtgag 1560
   ctgaccgtcg acgatagcgc cattatcggc attattcgtt actacacccg tgaggcgggc 1620
   gtgcgtggtc tggagcgtga aatctccaaa ctgtgtcgca aagcggttaa gcagttactg 1680
   ctcgataagt cattaaaaca tatcgaaatt aacggcgata acctgcatga ctatctcggt 1740
   gttcagcgtt tcgactatgg tcgcgcggat aacgaaaacc gtgtcggtca ggtaaccggt 1800
   ctggcgtgga cggaagtggg cggtgacttg ctgaccattg aaaccgcatg tgttccgggt    1860
   aaaggcaaac tgacctatac cggttcgctc ggcgaagtga tgcaggagtc cattcaggcg    1920
   gcgttaacgg tggttcgtgc gcgtgcggaa aaactgggga tcaaccctga tttttacgaa    1980
   aaacgtgaca tccacgtcca cgtaccggaa ggtgcgacgc cgaaagatgg tccgagtgcc    2040
   ggtattgcta tgtgcaccgc gctggtttct tgcctgaccg gtaacccggt tcgtgccgat    2100
   gtggcaatga ccggtgagat cactctgcgt ggtcaggtac tgccgatcgg tggtttgaaa    2160
   gaaaaactcc tggcagcgca tcgcggcggg attaaaacag tgctaattcc gttcgaaaat    2220
   aaacgcgatc tggaagagat tcctgacaac gtaattgccg atctggacat tcatcctgtg    2280
   aagcgcattg aggaagttct gactctggcg ctgcaaaatg aaccgtctgg tatgcaggtt    2340
   gtgactgcaa aatag                                                     2355
<210> 16
   <211> 954
   <212> DNA
   <213> Escherichia coli
<220>
   <221> source
   <222> 1..954
   <223> /mol_type="DNA" /organism="Escherichia coli"
<400> 16
   atgcgggcga aacttctggg aatagtcctg acaaccccta ttgcgatcag ctcttttgct      60
   tctaccgaga ctttatcgtt tactcctgac aacataaatg cggacattag tcttggaact     120
   ctgagcggaa aaacaaaaga gcgtgtttat ctagccgaag aaggaggccg aaaagtcagt     180
   caactcgact ggaaattcaa taacgctgca attattaaag gtgcaattaa ttgggatttg     240
   atgccccaga tatctatcgg ggctgctggc tggacaactc tcggcagccg aggtggcaat     300
   atggtcgatc aggactggat ggattccagt aaccccggaa cctggacgga tgaaagtaga     360
   caccctgata cacaactcaa ttatgccaac gaatttgatc tgaatatcaa aggctggctc     420
   ctcaacgaac ccaattaccg cctgggactc atggccggat atcaggaaag ccgttatagc     480
   tttacagcca gaggtggttc ctatatctac agttctgagg agggattcag agatgatatc     540
   ggctccttcc cgaatggaga aagagcaatc ggctacaaac aacgttttaa aatgccctac     600
   attggcttga ctggaagtta tcgttatgaa gattttgaac tcggtggcac atttaaatac     660
   agcggctggg tggaatcatc tgataacgat gaacactatg acccgggaaa aagaatcact     720
   tatcgcagta aggtcaaaga ccaaaattac tattctgttg cagtcaatgc aggttattac     780
   gtcacaccta acgcaaaagt ttatgttgaa ggcgcatgga atcgggttac gaataaaaaa     840
   ggtaatactt cactttatga tcacaataat aacacttcag actacagcaa aaatggagca     900
   ggtatagaaa actataactt catcactact gctggtctta agtacacatt ttaa           954
<210> 17
   <211> 624
   <212> DNA
   <213> Escherichia coli
<220>
   <221> source
   <222> 1..624
   <223> /mol_type="DNA" /organism="Escherichia coli"
<400> 17
   atgtcaacga ttattatgga tttatgtagt tacacccgac taggtttaac cgggtatctg 60
   ttgagtagag gggttaaaaa aagagaaatc aacgacattg aaaccgttga tgaccttgcc 120
   atagcttgtg attcacagcg cccttcagtg gtgtttatta atgaggactg tttcatccac 180
   gatgcttcta acagtcagcg tatcaagctc atcattaatc aacatcccaa tacgttattt 240
   atcgttttta tggcaattgc caatgttcat tttgatgaat atctattggt cagaaaaaat 300
   ttattgatca gttctaaatc gattaaaccg gaatctctcg acgatatcct tggcgatatt 360
   ctgaaaaaag agacaacgat aacctcgttt ttaaatatgc cgacgttatc attgagccga 420
   accgaatcga gtatgttgcg aatgtggatg gcaggtcagg gaaccattca aatctctgac 480
   caaatgaata tcaaagccaa gaccgtttca tcgcataaag gtaatattaa acgtaagatc 540
   aaaacgcata ataaacaggt tatctaccat gtcgtccgac tgacggataa tgtgactaat 600
   ggtatttttg tcaacatgcg ctaa 624
<210> 18
   <211> 3567
   <212> DNA
   <213> Escherichia coli
<220>
   <221> source
   <222> 1..3567
   <223> /mol_type="DNA" /organism="Escherichia coli"
<400> 18
   atgttatggg ccttaaatat ttggacaggc ccgcacagca atggattaat aacaatgatg 60
   aataaatcca attttgaatt cctgaagggc gtcaacgact tcacttatgc catcgcctgt 120
   gcggcggaaa ataactaccc ggatgatccc aacacgacgc tgattaaaat gcgtatgttt 180
   ggcgaagcca cagcgaaaca tcttggtctg ttactcaaca tccccccttg tgagaatcaa 240
   cacgatctcc tgcgtgaact cggcaaaatc gcctttgttg atgacaacat cctctctgta 300
   tttcacaaat tacgccgcat tggtaaccag gcggtgcacg aatatcataa cgatctcaac 360
   gatgcccaga tgtgcctgcg actcgggttc cgcctggctg tctggtacta ccgtctggtc 420
   actaaagatt atgacttccc ggtgccggtg tttgtgttgc cggaacgtgg tgaaaacctc 480
   tatcaccagg aagtgctgac gctaaaacaa cagcttgaac agcaggtgcg agaaaaagcg 540
   cagactcagg cagaagtcga agcgcaacag cagaagctgg ttgccctgaa cggctatatc 600
   gccattctgg aaggcaaaca gcaggaaacc gaagcgcaaa cccaggctcg ccttgcggca 660
   ctggaagcac agctcgccga gaagaacgcg gaactggcaa aacagaccga acaggaacgt 720
   aaggcttacc acaaagaaat taccgatcag gccatcaagc gcacactcaa ccttagcgaa 780
   gaagagagtc gcttcctgat tgatgcgcaa ctgcgtaaag caggctggca ggccgacagc 840
   aaaaccctgc gcttctccaa aggcgcacgt ccggaacccg gcgtcaataa agccattgcc 900
   gaatggccga ccggaaaaga tgaaacgggt aatcagggct ttgcggatta tgtgctgttt 960
   gtcggcctca aacccatcgc ggtggtagag gcgaaacgta acaatatcga cgttcccgcc 1020
   aggctcaatg agtcgtatcg ctacagtaaa tgtttcgata atggcttcct gcgggaaacc 1080
   ttgcttgagc actactcacc ggatgaagtg catgaagcag tgccagagta tgaaaccagc 1140
   tggcaggaca ccagcggcaa acaacggttt aaaatcccct tctgctactc gaccaacggg 1200
   cgcgaatacc gcgcaacaat gaagaccaaa agcggcatct ggtatcgcga cgtgcgtgat 1260
   acccgcaata tgtcgaaagc cttacccgag tggcaccgcc cggaagagct gctggaaatg 1320
   ctcggcagcg aaccgcaaaa acagaatcag tggtttgccg ataaccctgg catgagcgag 1380
   ctgggcctgc gttattatca ggaagatgcc gtccgcgcgg ttgaaaaggc aatcgtcaag 1440
   gggcaacaag agatcctgct ggcgatggcg accggtaccg gtaaaacccg tacggcaatc 1500
   gccatgatgt tccgcctgat ccagtcccag cgttttaaac gcattctctt ccttgtcgac 1560
   cgccgttctc ttggcgaaca ggcgctgggc gcgtttgaag atacgcgtat taacggcgac 1620
   accttcaaca gcattttcga cattaaaggg ctgacggata aattcccgga agacagcacc 1680
   aaaattcacg ttgccaccgt acagtcgctg gtgaaacgca ccctgcaatc agatgaaccg 1740
   atgccggtgg cccgttacga ctgtatcgtc gttgacgaag cgcatcgcgg ctatattctc 1800
   gataaagagc agaccgaagg cgaactgcag ttccgcagcc agctggatta cgtctctgcc 1860
   taccgtcgca ttctcgatca cttcgatgcg gtaaaaatcg ctctcaccgc caccccggcg 1920
   ctacatactg tgcagatttt cggcgagccg gtttaccgtt atacctaccg taccgcggtt 1980
   atcgacggtt ttctgatcga ccaggatccg cctattcaga tcatcacccg caacgcgcag 2040
   gagggggttt atctctccaa aggcgagcag gtagagcgca tcagcccgca gggagaagtg 2100
   atcaatgaca ccctggaaga cgatcaggat tttgaagtcg ccgactttaa ccgtggcctg 2160
   gtgatcccgg cgtttaaccg cgccgtctgt aacgaactca ccaattatct tgacccgacc 2220
   ggatcgcaaa aaacgctggt cttctgcgtc accaatgccc atgccgatat ggtggtggaa 2280
   gagctgcgtg ccgcgttcaa gaaaaagtat ccgcaactgg agcacgacgc gatcatcaag 2340
   atcaccggtg atgccgataa agacgcgcgc aaagtgcaga ccatgatcac ccgcttcaat 2400
   aaagagcggc tgcccaatat cgtggtaacc gtcgacctgc tgacgaccgg cgtcgatatt 2460
   ccgtcgatct gtaatatcgt gttcctgcgt aaagtacgca gccgcattct gtacgaacag 2520
   atgaaaggcc gcgccacgcg cttatgcccg gaggtgaata aaaccagctt taagattttt 2580
   gactgtgtcg atatctacag cacgctggag agcgtcgaca ccatgcgtcc ggtggtggtg 2640
   cgcccgaagg tggaactgca aacgctggtc aatgaaatta ccgattcaga aacctataaa 2700
   atcaccgaag cggatggccg cagttttgcc gagcacagcc atgaacaact ggtggcgaag 2760
   ctccagcgta tcatcggtct ggccacgttt aaccgtgacc gcagcgaaac gatagataaa 2820
   caggtgcgtc gtctggatga gctatgccag gacgcggcgg gcgtgaactt taacggcttc 2880
   gcctcgcgcc tgcgggaaaa agggccgcac tggagcgccg aagtctttaa caaactgcct 2940
   ggctttatcg cccgtctgga aaagctgaaa acggacatca acaacctgaa tgatgcgccg 3000
   atcttcctcg atatcgacga tgaagtggtg agtgtaaaat cgctgtacgg tgattacgac 3060
   acgccgcagg atttcctcga agcctttgac tcgctggtgc aacgttcccc gaacgcgcaa 3120
   ccggcattgc aggcagttat taatcgcccg cgcgatctca cccgtaaagg gctggtcgag 3180
   ctacaggagt ggtttgaccg ccagcacttt gaggaatctt ccctgcgcaa agcatggaaa 3240
   gagacgcgca atgaagatat cgccgcccgg ctgattggtc atattcgccg cgctgcggtg 3300
   ggcgatgcgc tgaaaccgtt tgaggaacgt gtcgatcacg cgctgacgcg cattaagggc 3360
   gaaaacgact ggagcagcga gcaattaagc tggctcgatc gtttagcgca ggcgctgaaa 3420
   gagaaagtgg tgctcgacga cgatgtcttc aaaaccggca acttccaccg tcgcggcggg 3480
   aaggcgatgc tgcaaagaac ctttgacgat aatctcgata ccctgctggg caaattcagc 3540
   gattatatct gggacgagct ggcctga 3567
<210> 19
   <211> 915
   <212> DNA
   <213> Escherichia coli
<220>
   <221> source
   <222> 1..915
   <223> /mol_type="DNA" /organism="Escherichia coli"
<400> 19
   atgacggttc ctacctatga caaatttatt gaacctgttc tgcgttatct ggcaacaaaa      60
   ccggaaggtg cagccgcgcg tgatgttcat gaggctgccg cggatgcatt aggactggat     120
   gacagccagc gagcgaaagt cattaccagc ggacaacttg tttataaaaa tcgtgcaggc     180
   tgggcgcatg accgtttaaa acgtgccggg ttgtcgcaaa gtttgtcgcg tggcaaatgg     240
   tgcctgactc ctgcgggttt tgactgggtt gcgtctcatc cccagccaat gacggagcag     300
   gagacgaacc atctggcctt cgcttttgtg aatgtcaaac ttaagtcacg gccggatgcc     360
   gtcgatttag atccgaaagc cgactctccc gatcatgaag aacttgcaaa gagcagcccg     420
   gacgatcggt tagatcaggc gctaaaagag cttcgtgatg cggtggctga tgaggttctg     480
   gaaaacttat tgcaggtttc tccttcgcgc tttgaagtca ttgttctgga tgttttgcat     540
   cgcctggggt atggcggcca ccgtgatgat ttgcagcgtg ttggcggtac tggagatggt     600
   ggcatcgatg gtgtgatatc gcttgataaa cttggcctgg agaaagttta tgttcaggca     660
   aaacgttggc agaatactgt aggcaggcca gaattacagg cattttacgg cgcactggct     720
   gggcaaaaag cgaaacgtgg ggtgtttatt accacttctg gatttacttc tcaggcgcgt     780
   gactttgccc aatccgtcga gggtatggtg ttggttgatg gggaacgcct ggtgcactta     840
   atgatcgaaa acgaagtagg ggtttcttca cgtttgttga aggtgccgaa actggatatg     900
   gactattttg agtga                                                      915
<210> 20
   <211> 708
   <212> DNA
   <213> Escherichia coli
<220>
   <221> source
   <222> 1..708
   <223> /mol_type="DNA" /organism="Escherichia coli"
<400> 20
   atgtaccgtt atttgtctat tgctgcggtg gtactgagcg cagcattttc cggcccggcg      60
   ttggccgaag gtatcaatag tttttctcag gcgaaagccg cggcggtaaa agtccacgct     120
   gacgcgcccg gtacgtttta ttgcggatgt aaaattaact ggcagggcaa aaaaggcgtt     180
   gttgatctgc aatcgtgcgg ctatcaggtg cgcaaaaatg aaaaccgcgc cagccgcgta     240
   gagtgggaac atgtcgttcc cgcctggcag ttcggtcacc agcgccagtg ctggcaggac     300
   ggtggacgta aaaactgcgc taaagatccg gtctatcgca agatggaaag cgatatgcat     360
   aacctgcagc cgtcagtcgg tgaggtgaat ggcgatcgcg gcaactttat gtacagccag     420
   tggaatggcg gtgaaggcca gtacggtcaa tgcgccatga aggtcgattt caaagaaaaa     480
   gctgccgaac caccagcgcg tgcacgcggt gccattgcgc gcacctactt ctatatgcgc     540
   gaccaataca acctgacact ctctcgccag caaacgcagc tgttcaacgc atggaacaag     600
   atgtatccgg ttaccgactg ggagtgcgag cgcgatgaac gcatcgcgaa ggtgcagggc     660
   aatcataacc cgtatgtgca acgcgcttgc caggcgcgaa agagctaa                  708
<210> 21
   <211> 1062
   <212> DNA
   <213> Escherichia coli
<220>
   <221> source
   <222> 1..1062
   <223> /mol_type="DNA" /organism="Escherichia coli"
<400> 21
   atggctatcg acgaaaacaa acagaaagcg ttggcggcag cactgggcca gattgagaaa      60
   caatttggta aaggctccat catgcgcctg ggtgaagacc gttccatgga tgtggaaacc     120
   atctctaccg gttcgctttc actggatatc gcgcttgggg caggtggtct gccgatgggc     180
   cgtatcgtcg aaatctacgg accggaatct tccggtaaaa ccacgctgac gctgcaggtg     240
   atcgccgcag cgcagcgtga aggtaaaacc tgtgcgttta tcgatgctga acacgcgctg     300
   gacccaatct acgcacgtaa actgggcgtc gatatcgaca acctgctgtg ctcccagccg     360
   gacaccggcg agcaggcact ggaaatctgt gacgccctgg cgcgttctgg cgcagtagac     420
   gttatcgtcg ttgactccgt ggcggcactg acgccgaaag cggaaatcga aggcgaaatc     480
   ggcgactctc acatgggcct tgcggcacgt atgatgagcc aggcgatgcg taagctggcg     540
   ggtaacctga agcagtccaa cacgctgctg atcttcatca accagatccg tatgaaaatt     600
   ggtgtgatgt tcggtaaccc ggaaaccact accggtggta acgcgctgaa attctacgcc     660
   tctgttcgtc tcgacatccg tcgtatcggc gcggtgaaag agggcgaaaa cgtggtgggt     720
   agcgaaaccc gcgtgaaagt ggtgaagaac aaaatcgctg cgccgtttaa acaggctgaa     780
   ttccagatcc tctacggcga aggtatcaac ttctacggcg aactggttga cctgggcgta     840
   aaagagaagc tgatcgagaa agcaggcgcg tggtacagct acaaaggtga gaagatcggt     900
   cagggtaaag cgaatgcgac tgcctggctg aaagataacc cggaaaccgc gaaagagatc     960
   gagaagaaag tacgtgagtt gctgctgagc aacccgaact caacgccgga tttctctgta    1020
   gatgatagcg aaggcgtagc agaaactaac gaagattttt aa                       1062
<210> 22
   <211> 306
   <212> DNA
   <213> Escherichia coli
<220>
   <221> source
   <222> 1..306
   <223> /mol_type="DNA" /organism="Escherichia coli"
<400> 22
   atgcagttta aggtttacac ctataaaaga gagagccgtt atcgtctgtt tgtggatgta      60
   cagagtgata ttattgacac gcccgggcga cggatggtga tccccctggc cagtgcacgt     120
   ctgctgtcag ataaagtctc ccgtgaactt tacccggtgg tgcatatcgg ggatgaaagc     180
   tggcgcatga tgaccaccga tatggccagt gtgccggtct ccgttatcgg ggaagaagtg     240
   gctgatctca gccaccgcga aaatgacatc aaaaacgcca ttaacctgat gttctgggga     300
   atataa                                                                306
<210> 23
   <211> 219
   <212> DNA
   <213> Escherichia coli
<220>
   <221> source
   <222> 1..219
   <223> /mol_type="DNA" /organism="Escherichia coli"
<400> 23
   atgaagcagc gtattacagt gacagttgac agcgacagct atcagttgct caaggcatat 60
   gatgtcaata tctccggtct ggtaagcaca accatgcaga atgaagcccg tcgtctgcgt 120
   gccgaacgct ggaaagcgga aaatcaggaa gggatggctg aggtcgcccg gtttattgaa 180
   atgaacggct cttttgctga cgagaacagg gactggtga 219
<210> 24
   <211> 59
   <212> DNA
   <213> artificial sequences
<220>
   <221> source
   <222> 1..59
   <223> /mol_type="DNA" /note="primers XisgalKstart" /organism="artificial sequences"
<400> 24
   gggggtaaat cccggcgctc atgacttcgc cttcttccca gaattcctgt tgacaatta 59
<210> 25
   <211> 60
   <212> DNA
   <213> artificial sequences
<220>
   <221> source
   <222> 1..60
   <223> /mol_type="DNA" /note="XisgalK stop" /organism="artificial sequences"
<400> 25
   gttctgatta ttggaaatct tctttgccct ccagtgtgag cagcactgtc ctgctccttg 60
<210> 26
   <211> 20
   <212> DNA
   <213> artificial sequences
<220>
   <221> source
   <222> 1..20
   <223> /mol_type="DNA" /note="Xis1 primer" /organism="artificial sequences"
<400> 26
   gtcttcaagt ggagcatcag 20
<210> 27
   <211> 20
   <212> DNA
   <213> artificial sequences
<220>
   <221> source
   <222> 1..20
   <223> /mol_type="DNA" /note="Xis4 primer" /organism="artificial sequences"
<400> 27
   accaggacta tccgtatgac 20
<210> 28
   <211> 35
   <212> DNA
   <213> artificial sequences
<220>
   <221> source
   <222> 1..35
   <223> /mol_type="DNA" /note="Xis2 primer" /organism="artificial sequences"
<400> 28
   ccaaacggaa cagatgaaga aggcgaagtc atgag 35
<210> 29
   <211> 35
   <212> DNA
   <213> artificial sequences
<220>
   <221> source
   <222> 1..35
   <223> /mol_type="DNA" /note="Xis3 primer" /organism="artificial sequences"
<400> 29
   gacttcgcct tcttcatctg ttccgtttgg cttcc 35
<210> 30
   <211> 21
   <212> DNA
   <213> artificial sequences
<220>
   <221> source
   <222> 1..21
   <223> /mol_type="DNA" /note="Xis6 primer" /organism="artificial sequences"
<400> 30
   gtaatggaaa gctggtagtc g 21
<210> 31
   <211> 20
   <212> DNA
   <213> artificial sequences
<220>
   <221> source
   <222> 1..20
   <223> /mol_type="DNA" /note="Xis5 primer" /organism="artificial sequences"
<400> 31
   cagccgtaag tcttgatctc 20
<210> 32
   <211> 20
   <212> DNA
   <213> artificial sequences
<220>
   <221> source
   <222> 1..20
   <223> /mol_type="DNA" /note="Xis7 primer" /organism="artificial sequences"
<400> 32
   cagcaggcat gatccaagag 20
<210> 33
   <211> 35
   <212> DNA
   <213> artificial sequences
<220>
   <221> source
   <222> 1..35
   <223> /mol_type="DNA" /note="Xis2b primer" /organism="artificial sequences"
<400> 33
   tttgccctcc agtgtgaaga aggcgaagtc atgag 35
<210> 34
   <211> 39
   <212> DNA
   <213> artificial sequences
<220>
   <221> source
   <222> 1..39
   <223> /mol_type="DNA" /note="Xis8 primer" /organism="artificial sequences"
<400> 34
   ctcatgactt cgccttcttc acactggagg gcaaagaag 39
<210> 35
   <211> 624
   <212> DNA
   <213> Enterobacteria phage lambda
<220>
   <221> source
   <222> 1..624
   <223> /mol_type="DNA" /organism="Enterobacteria phage lambda"
<400> 35
   atgagactcg aaagcgtagc taaatttcat tcgccaaaaa gcccgatgat gagcgactca      60
   ccacgggcca cggcttctga ctctctttcc ggtactgatg tgatggctgc tatggggatg     120
   gcgcaatcac aagccggatt cggtatggct gcattctgcg gtaagcacga actcagccag     180
   aacgacaaac aaaaggctat caactatctg atgcaatttg cacacaaggt atcggggaaa     240
   taccgtggtg tggcaaagct tgaaggaaat actaaggcaa aggtactgca agtgctcgca     300
   acattcgctt atgcggatta ttgccgtagt gccgcgacgc cgggggcaag atgcagagat     360
   tgccatggta caggccgtgc ggttgatatt gccaaaacag agctgtgggg gagagttgtc     420
   gagaaagagt gcggaagatg caaaggcgtc ggctattcaa ggatgccagc aagcgcagca     480
   tatcgcgctg tgacgatgct aatcccaaac cttacccaac ccacctggtc acgcactgtt     540
   aagccgctgt atgacgctct ggtggtgcaa tgccacaaag aagagtcaat cgcagacaac     600
   attttgaatg cggtcacacg ttag                                            624
<210> 36
   <211> 144
   <212> DNA
   <213> Enterobacteria phage lambda
<220>
   <221> source
   <222> 1..144
   <223> /mol_type="DNA" /organism="Enterobacteria phage lambda"
<400> 36
   atggatcaaa cacttatggc tatccagact aaattcacta tcgccacttt tattggcgat      60
   gaaaagatgt ttcgtgaagc cgtcgacgct tataaaaaat ggatattaat actgaaactg     120
   agatcaagca aaagcattca ctaa                                            144